# EUROPEAN PATENT APPLICATION

(11) **EP 3 705 575 A1**
(43) Date of publication of application: **09.09.2020**
(21) Application number: 18873830.6
(22) Date of filing: 02.11.2018
(51) Int. Cl.: C12N 15/63, A01K 67/027, C12N 5/10, C12P 21/02

(54) **HIGH-YIELD PRODUCTION METHOD FOR PROTEIN BY USING MAMMALIAN ARTIFICIAL CHROMOSOME VECTOR**

(30) Priority: 02.11.2017 JP 2017213237
(71) Applicant: National University Corporation Tottori University, Tottori-shi, Tottori 680-8550 (JP); Trans Chromosomics, Inc., Yonago-shi, Tottori 683-8503 (JP)
(72) Inventor: KAZUKI, Yasuhiro, Yonago-shi Tottori 683-8503 (JP); OHIRA, Takahito, Yonago-shi Tottori 683-8503 (JP); UNO, Narumi, Yonago-shi Tottori 683-8503 (JP); KUGOH, Hiroyuki, Yonago-shi Tottori 683-8503 (JP); OSHIMURA, Mitsuo, Yonago-shi Tottori 683-8503 (JP)
(74) Representative: D Young & Co LLP
(86) International application number: PCT/JP2018/040852
(87) International publication number: WO 2019/088257

(57) **Abstract**

This application provides: a nucleic acid construct characterized by comprising, in a mammalian artificial chromosome vector, a DNA sequence of interest, a matrix attachment region, and a replication origin, and by using for high expression of the DNA; a mammalian cell comprising the nucleic acid construct; and a method for high production of a protein encoded by the DNA of interest in the mammalian cell.

SEQUENCE LISTING

<110>  National University Corporation Tottori University
       Trans Chromosomics, Inc.

<120>  Method for High Production of Proteins Utilizing
       Mammalian Artificial Chromosome Vector

<130>  PH-7520-PCT

<150>  JP 2017-213237
<151>  2017-11-02

<160>  23

<170>  PatentIn version 3.5

<210>  1
<211>  35
<212>  DNA
<213>  Artificial

<220>
<223>  primer

<400>  1
gaaggcctgg agttccgcgt tacataactt acggt                                35


<210>  2
<211>  36
<212>  DNA
<213>  Artificial

<220>
<223>  primer

<400>  2
gaaggcctgg tgacctaagc ttgcatgcaa cttcgt                               36


<210>  3
<211>  38
<212>  DNA
<213>  Artificial

<220>
<223>  primer

<400>  3
aaaagtactc caattaacca attctgatta gaaaaact                             38


<210>  4
<211>  38
<212>  DNA
<213>  Artificial

<220>
<223>  primer

<400>  4
ggactagtaa agccacgttg tgtctcaaaa tctctgat                             38

<210>  5
<211>  20
<212>  DNA
<213>  Artificial

<220>
<223>  junction region

<400>  5
cgtaacaact ccgccccatt                                                    20


<210>  6
<211>  20
<212>  DNA
<213>  Artificial

<220>
<223>  junction region

<400>  6
gcagccgatt gtctgttgtg                                                    20


<210>  7
<211>  20
<212>  DNA
<213>  Artificial

<220>
<223>  junction region

<400>  7
gggcgtactt ggcatatgat                                                    20


<210>  8
<211>  21
<212>  DNA
<213>  Artificial

<220>
<223>  junction region

<400>  8
atccgctcac aattccacac a                                                  21


<210>  9
<211>  20
<212>  DNA
<213>  Artificial

<220>
<223>  primer

<400>  9
cttcttcaag tccgccatgc                                                    20


<210>  10

<211>  20
<212>  DNA
<213>  Artificial

<220>
<223>  primer

<400>  10
ggtcttgtag ttgccgtcgt                                                    20


<210>  11
<211>  20
<212>  DNA
<213>  Artificial

<220>
<223>  primer

<400>  11
acaggtttct gcttctggca                                                    20


<210>  12
<211>  20
<212>  DNA
<213>  Artificial

<220>
<223>  primer

<400>  12
catcagctga ctggttcaca                                                    20


<210>  13
<211>  41
<212>  DNA
<213>  Artificial

<220>
<223>  primer

<400>  13
attaattaat cttagtatgg taaacctttt gaagtagatt c                            41


<210>  14
<211>  45
<212>  DNA
<213>  Artificial

<220>
<223>  primer

<400>  14
gtaacgcgtc aagtttttat tttgttctca caattaagtc tatac                        45


<210>  15
<211>  7311
<212>  DNA
<213>  Artificial

<220>
<223>  anti-VEGF heavy chain gene vector

<400>  15
   atcagatcgg aagagcacac gtctgaactc cagtcttcaa tggtatcgcc cagatagctt      60
   ggcgtaatca tggtcatggt catagctgtt tcctgtgtga aattgttatc cgctcacaat     120
   tccacacaac atacgagccg gaagcataaa gtgtaaagcc tggggtgcct aatgagtgag     180
   ctaactcaca ttaattgcgt tgcgctcact gcccgctttc cagtcgggaa acctgtcgtg     240
   ccagctgcat taatgaatcg gccaacgcgc ggggagaggc ggtttgcgta ttgggcgctc     300
   ttccgcttcc tcgctcactg actcgctgcg ctcggtcgtt cggctgcggc gagcggtatc     360
   agctcactca aaggcggtaa tacggttatc cacagaatca ggggataacg caggaaagaa     420
   catgtgagca aaaggccagc aaaaggccag gaaccgtaaa aaggccgcgt tgctggcgtt     480
   tttccatagg ctccgccccc ctgacgagca tcacaaaaat cgacgctcaa gtcagaggtg     540
   gcgaaacccg acaggactat aaagatacca ggcgtttccc cctggaagct ccctcgtgcg     600
   ctctcctgtt ccgaccctgc cgcttaccgg atacctgtcc gcctttctcc cttcgggaag     660
   cgtggcgctt tctcatagct cacgctgtag gtatctcagt tcggtgtagg tcgttcgctc     720
   caagctgggc tgtgtgcacg aaccccccgt tcagcccgac cgctgcgcct tatccggtaa     780
   ctatcgtctt gagtccaacc cggtaagaca cgacttatcg ccactggcag cagccactgg     840
   taacaggatt agcagagcga ggtatgtagg cggtgctaca gagttcttga agtggtggcc     900
   taactacggc tacactagaa gaacagtatt tggtatctgc gctctgctga agccagttac     960
   cttcggaaaa agagttggta gctcttgatc cggcaaacaa accaccgctg gtagcggtgg    1020
   tttttttgtt tgcaagcagc agattacgcg cagaaaaaaa ggatctcaag aagatccttt    1080
   gatcttttct acggggtctg acgctcagtg gaacgaaaac tcacgttaag ggattttggt    1140
   catgagatta tcaaaaagga tcttcaccta gatcctttta aattaaaaat gaagttttaa    1200
   atcaatctaa agtatatatg agtaaacttg gtctgacagt taccaatgct taatcagtga    1260
   ggcacctatc tcagcgatct gtctatttcg ttcatccata gttgcctgac tccccgtcgt    1320
   gtagataact acgatacggg agggcttacc atctggcccc agtgctgcaa tgataccgcg    1380
   agacccacgc tcaccggctc cagatttatc agcaataaac cagccagccg gaagggccga    1440
   gcgcagaagt ggtcctgcaa ctttatccgc ctccatccag tctattaatt gttgccggga    1500
   agctagagta agtagttcgc cagttaatag tttgcgcaac gttgttgcca ttgctacagg    1560
   catcgtggtg tcacgctcgt cgtttggtat ggcttcattc agctccggtt cccaacgatc    1620
   aaggcgagtt acatgatccc ccatgttgtg caaaaaagcg gttagctcct tcggtcctcc    1680
   gatcgttgtc agaagtaagt tggccgcagt gttatcactc atggttatgg cagcactgca    1740
   taattctctt actgtcatgc catccgtaag atgcttttct gtgactggtg agtactcaac 1800
   caagtcattc tgagaatagt gtatgcggcg accgagttgc tcttgcccgg cgtcaatacg 1860
   ggataatacc gcgccacata gcagaacttt aaaagtgctc atcattggaa aacgttcttc 1920
   ggggcgaaaa ctctcaagga tcttaccgct gttgagatcc agttcgatgt aacccactcg 1980
   tgcacccaac tgatcttcag catcttttac tttcaccagc gtttctgggt gagcaaaaac 2040
   aggaaggcaa aatgccgcaa aaaagggaat aagggcgaca cggaaatgtt gaatactcat 2100
   actcttcctt tttcaatatt attgaagcat ttatcagggt tattgtctca tgagcggata 2160
   catatttgaa tgtatttaga aaaataaaca aataggggtt ccgcgcacat ttccccgaaa 2220
   agtgccacct gacgtctaag aaaccattat tatcatgaca ttaacctata aaaataggcg 2280
   tatcacgagg ccctttcgtc tcgcgcgttt cggtgatgac ggtgaaaacc tctgacacat 2340
   gcagctcccg gagacggtca cagcttgtct gtaagcggat gccgggagca gacaagcccg 2400
   tcagggcgcg tcagcgggtg ttggcgggtg tcggggctgg cttaattaaa tgccccaaaa 2460
   catcaggtta atggcgttct tgatgtcatt ctcgcgatgc gacagatccg caacctcttc 2520
   gccaattacc gacactggta ctgacgccat atccgtggtc atcatacgcc agctttcatc 2580
   cccaatgtgg acaaccggat agagttcacg ggaaactttg tcgctcagta accgtgcaga 2640
   agccaacgga atcaccgtgc gacgaccagg agtatcgatg atgtcgcttt gcacatccac 2700
   aaacaggcga tagcgacttt cgcgtttgta ggtgtagact ttgaactgcg gtacgttaca 2760
   gagagcgcaa ctgctgtgac ggacgatctg tacgcgcttc ttcgctattc cgccaactcg 2820
   caaacggagg atgtgctgca aggcgattaa gttgggtaac gcctgggttt tcccagtcac 2880
   gacgttgtaa aacgacggcc agtgaattgt aatagctctc tgagtacgga acactctttc 2940
   ccttaacgac gctcttccga tctgatgaac gcgttcctta aggacggatc gggagatctc 3000
   ccgatcccct atgtctcagt acaatctgct ctgatgccgc atagttaagc cagtatctgc 3060
   tccctgcttg tgtgttggag gtcgctgagt agtgcgcgag caaaatttaa gctacaacaa 3120
   ggcaaggctt gaccgacaat tgcatgaaga atctgcttag ggttaggcgt tttgcgctgc 3180
   ttcgcgatgt acgggccaga tattgacatt gattattgac taggcttttg caaaaagctt 3240
   gtttaaacct tgtatattaa cagcacatta acagctaaaa agaaaaactc acataatcat 3300
   attagttcat agaaacaatg catttgacat actccaacat ccattcatga ttttatttta 3360
   ttttttattt atatattttt tttgagatgg agtctcgctg tcacccaggc tggagtgcaa 3420
   tggctcgatc tcggctcact gcaggctccg cccccggcgg ttcacgccat tctcctgcct 3480
   cagcctcccg agtagctggg actacaggcg cccgccacct cgcccagcta attttttgta 3540
   tttttagtag agacggggtt tcaccgtgtt agccaggatg gtctcgatct cctgacctcg 3600
   tgatccaccc gcctcggcct cccaaagtgc tgggattaca ggcgtgagcc accgtgcccg 3660
   gcccatgatt ttaaaaaaac ctctcagaaa tagaaacaga gggaacttcc tgaatttgat 3720
   taaaaatacc tgcaaaatcc tagagctaat attatactta atggtgaaag actgaatggt 3780
   tttcccctaa gatggagaac aaggcacgga tgtccttgct caccactcct attcaacaaa 3840
   ggactggaaa ccagaaataa ctttaatttt ttttgttctc aagtgatagg ttcacagaga 3900
   aaaagcttta ctggatgaac ttttagatta ctacttttat agagcagcag agataaaagc 3960
   caggtcgaaa agtgcatgtg gagtaaggaa atggacctag ttcgacaaaa gggctcagaa 4020
   cgactgccca gatgagattg tagacgcagc tgtagtttac tttctatctg gaagaaactt 4080
   caagttatcc ttaattttcc aaggagacag tcacttacct tttaaaaaac attattagag 4140
   aagcaccggg cgggagcata tctagcatta aaaatgtggg atgaatacca tctctgcttg 4200
   gtaaaggtgg ttgggaatcc tgagagaggg cacctagtgt ggcttctgca tttttcacag 4260
   tgcctggacc acggctgaaa gtaactcttg catgacattt gacagaagag gaaaccgaag 4320
   ctcagattaa attccctgtc cacagcggga tcattcggca cgagttcctc cctgtctgga 4380
   atgctcttcc ccagcaatag atcctgcggc tcttccgtgt ctcagtctaa tgtcattccg 4440
   ttccaggatt cccgacttct taaagcataa ataatccctc cccaccctct cattgtactg 4500
   ttatgtaagt tattacaata tgtcattata tatttagtca tactgcttta ggtaatgtct 4560
   tctccactga actgtaagct ccatgagggc aagagttcag tcggttttac ttaataatta 4620
   gcacctagta cagtactagc atagaatgaa ggcctcgcaa ttttttttaa atttattttt 4680
   agacagggtc ttgcgctgtc gcccaggctg gagtgcagtg gtgcaacctc ggctcacggc 4740
   agcctcgacc tttcggctcc agcgatcctc ccgcgtcggc ctccggggta gctgggactg 4800
   caggcgcgca ccaccatgac tggctaattt tttttttttt ttttttgtag acatggggtc 4860
   tcgccatgtt gcccaggctg gttcctgagc tcaagtgatc ctcctgcctc ggcctcccaa 4920
   agtgctggga ttacaggcgt gagcctcagc gcccagccaa gttagccttt tttaaacgtc 4980
   ctgtctccgg aggttgccga agttggtttt cttcggcctc cttctctctc ccaggcccag 5040
   ggctgggacg aggccggttc ccgcctgcaa cctgcactga agacgggaac cttgggagcc 5100
   ggtaccggaa cgctcggaaa cggcaccaaa gtacgaatcc tagggcggaa aagcgttacc 5160
   aagacactcg tccccagagc cgcttcctgg gactctctag cctcctaccg cttctcagtg 5220
   atgttccggt ttccgccctc ctcctcgcgc tgtttccgcc tcttgccttc ggacgccgga 5280
   ttttgacgtg ctctcgcgag atttgggtct cttcctaagc cggcgctcgg caaggtaggt 5340
   tggcggcctg ctctccgaca gaacttttct tcttgggttg aggaaaacgc cttttggagt 5400
   caggccctgg aggggcgagc cttgctcaca gggtggggat acagccgatt acccgccctg 5460
   tgctttccga tggcttctgc ggggcgagcg gggcctggcc ggggggtgcg ggcgggaggg 5520
   cgagccagcg gcgcctgcag cccgggccgc gtaacgctga ccgctgtgcc ttcagttctc 5580
   ccaggagaaa gccgctagcc gccaccatgg agttcgggtt gtcatggctg ttcctggtcg 5640
   ccattctgaa aggagtgcag tgcgaggttc aattggtgga gtctggcgga ggtctcgtgc 5700
   agcccggagg gagcctccgc ctgagctgcg ccgcttcagg gtacacattc acaaactacg 5760
   gcatgaactg ggttcgacag gcacccggca agggacttga atgggtgggt tggatcaaca 5820
   catacactgg agagcccact tatgctgccg attttaaaag acggttcact tttagtctcg 5880
   acacatccaa gtccactgcc tatcttcaga tgaatagctt gcgcgcagag gacacagccg 5940
   tatactattg cgccaagtat ccacattatt acgggagttc tcattggtat ttcgacgtct 6000
   ggggtcaggg tacactggta actgtgagtt ccgcctcaac caagggacct tccgtatttc 6060
   ccctcgctcc aagctctaag tccacttctg gagggactgc tgctctcgga tgtctggtaa 6120
   aggactattt tcctgagcca gtcaccgtct cttggaactc tggcgccttg acttctggcg 6180
   ttcatacatt tcccgccgtg ctgcagagtt ccggattgta ttcattgtct tcagtcgtaa 6240
   cagtgccatc ctcatcattg ggaacacaga catatatctg taatgtcaac cataagcctt 6300
   ctaacaccaa ggtcgataag aaggttgaac ctaaatcatg cgacaagaca cacacttgtc 6360
   caccatgtcc tgcacctgaa cttttgggcg gacctagcgt atttcttttc ccacctaagc 6420
   ccaaagatac ccttatgatc tcccgtacac ccgaagttac ctgtgtggtt gtggatgtca 6480
   gccatgaaga tcctgaagtt aagttcaact ggtatgtgga tggtgttgaa gtacacaacg 6540
   ccaaaaccaa gcccagggag gagcagtaca attcaactta cagggttgta tctgtcctca 6600
   ccgtactgca tcaggattgg ctcaacggca aggagtacaa gtgtaaagtg agcaacaaag 6660
   ctcttcctgc tcccattgaa aagactatta gtaaagctaa gggtcagcct agagaacccc 6720
   aagtgtacac tttgcctccc agtcgggaag agatgactaa gaaccaagta tccctgacat 6780
   gccttgtgaa gggattttat ccatcagata tcgcagtgga atgggagagc aatggccagc 6840
   ccgagaacaa ctataaaact actcctcctg tcctggattc tgatgggtcc ttcttcctgt 6900
   attccaagtt gacagtcgat aaaagccgtt ggcagcaggg aaacgtgttc agttgcagtg 6960
   tgatgcacga ggccttgcac aatcactaca cacagaaaag cctgtccctt tcaccaggga 7020
   agtaggcggc cgctgcaggg agatttcgat tccaccgccg ccttctatga aaggttgggc 7080
   ttcggaatcg ttttccggga cgccggctgg atgatcctcc agcgcgggga tctcatgctg 7140
   gagttcttcg cccaccccaa cttgtttatt gcagcttata atggttacaa ataaagcaat 7200
   agcatcacaa atttcacaaa taaagcattt ttttcactgc attctagttg tggtttgtcc 7260
   aaactcatca atgtatctta gtcggcgcgc cgacggactc gagtcgtcga c 7311

<210> 16
<211> 6660

<212>  DNA
<213>  Artificial

<220>
<223>  anti-VEGF light chain gene vector

<400>  16
atcagatcgg aagagcacac gtctgaactc cagtcttcaa tggtatcgcc cagatagctt      60

ggcgtaatca tggtcatggt catagctgtt tcctgtgtga aattgttatc cgctcacaat     120

tccacacaac atacgagccg gaagcataaa gtgtaaagcc tggggtgcct aatgagtgag     180

ctaactcaca ttaattgcgt tgcgctcact gcccgctttc cagtcgggaa acctgtcgtg     240

ccagctgcat taatgaatcg gccaacgcgc ggggagaggc ggtttgcgta ttgggcgctc     300

ttccgcttcc tcgctcactg actcgctgcg ctcggtcgtt cggctgcggc gagcggtatc     360

agctcactca aaggcggtaa tacggttatc cacagaatca ggggataacg caggaaagaa     420

catgtgagca aaaggccagc aaaaggccag gaaccgtaaa aaggccgcgt tgctggcgtt     480

tttccatagg ctccgccccc ctgacgagca tcacaaaaat cgacgctcaa gtcagaggtg     540

gcgaaacccg acaggactat aaagatacca ggcgtttccc cctggaagct ccctcgtgcg     600

ctctcctgtt ccgaccctgc cgcttaccgg atacctgtcc gcctttctcc cttcgggaag     660

cgtggcgctt tctcatagct cacgctgtag gtatctcagt tcggtgtagg tcgttcgctc     720

caagctgggc tgtgtgcacg aaccccccgt tcagcccgac cgctgcgcct tatccggtaa     780

ctatcgtctt gagtccaacc cggtaagaca cgacttatcg ccactggcag cagccactgg     840

taacaggatt agcagagcga ggtatgtagg cggtgctaca gagttcttga agtggtggcc     900

taactacggc tacactagaa gaacagtatt tggtatctgc gctctgctga agccagttac     960

cttcggaaaa agagttggta gctcttgatc cggcaaacaa accaccgctg gtagcggtgg    1020

tttttttgtt tgcaagcagc agattacgcg cagaaaaaaa ggatctcaag aagatccttt    1080

gatcttttct acggggtctg acgctcagtg gaacgaaaac tcacgttaag ggattttggt    1140

catgagatta tcaaaaagga tcttcaccta gatcctttta aattaaaaat gaagttttaa    1200

atcaatctaa agtatatatg agtaaacttg gtctgacagt taccaatgct taatcagtga    1260

ggcacctatc tcagcgatct gtctatttcg ttcatccata gttgcctgac tccccgtcgt    1320

gtagataact acgatacggg agggcttacc atctggcccc agtgctgcaa tgataccgcg    1380

agacccacgc tcaccggctc cagatttatc agcaataaac cagccagccg gaagggccga    1440

gcgcagaagt ggtcctgcaa ctttatccgc ctccatccag tctattaatt gttgccggga    1500

agctagagta agtagttcgc cagttaatag tttgcgcaac gttgttgcca ttgctacagg    1560

catcgtggtg tcacgctcgt cgtttggtat ggcttcattc agctccggtt cccaacgatc    1620

aaggcgagtt acatgatccc ccatgttgtg caaaaaagcg gttagctcct tcggtcctcc    1680

gatcgttgtc agaagtaagt tggccgcagt gttatcactc atggttatgg cagcactgca 1740

taattctctt actgtcatgc catccgtaag atgcttttct gtgactggtg agtactcaac 1800

caagtcattc tgagaatagt gtatgcggcg accgagttgc tcttgcccgg cgtcaatacg 1860

ggataatacc gcgccacata gcagaacttt aaaagtgctc atcattggaa aacgttcttc 1920

ggggcgaaaa ctctcaagga tcttaccgct gttgagatcc agttcgatgt aacccactcg 1980

tgcacccaac tgatcttcag catcttttac tttcaccagc gtttctgggt gagcaaaaac 2040

aggaaggcaa aatgccgcaa aaaagggaat aagggcgaca cggaaatgtt gaatactcat 2100

actcttcctt tttcaatatt attgaagcat ttatcagggt tattgtctca tgagcggata 2160

catatttgaa tgtatttaga aaaataaaca aataggggtt ccgcgcacat ttccccgaaa 2220

agtgccacct gacgtctaag aaaccattat tatcatgaca ttaacctata aaaataggcg 2280

tatcacgagg ccctttcgtc tcgcgcgttt cggtgatgac ggtgaaaacc tctgacacat 2340

gcagctcccg gagacggtca cagcttgtct gtaagcggat gccgggagca gacaagcccg 2400

tcagggcgcg tcagcgggtg ttggcgggtg tcggggctgg cttaattaaa tgccccaaaa 2460

catcaggtta atggcgttct tgatgtcatt ctcgcgatgc gacagatccg caacctcttc 2520

gccaattacc gacactggta ctgacgccat atccgtggtc atcatacgcc agctttcatc 2580

cccaatgtgg acaaccggat agagttcacg ggaaactttg tcgctcagta accgtgcaga 2640

agccaacgga atcaccgtgc gacgaccagg agtatcgatg atgtcgcttt gcacatccac 2700

aaacaggcga tagcgacttt cgcgtttgta ggtgtagact ttgaactgcg gtacgttaca 2760

gagagcgcaa ctgctgtgac ggacgatctg tacgcgcttc ttcgctattc cgccaactcg 2820

caaacggagg atgtgctgca aggcgattaa gttgggtaac gcctgggttt tcccagtcac 2880

gacgttgtaa aacgacggcc agtgaattgt aatagctctc tgagtacgga acactctttc 2940

ccttaacgac gctcttccga tctgatggtt aattaaccgc gatcgcgcgc ctgcaggact 3000

acgtagtcac gcgtgatcct taaggacgga tcgggagatc tcccgatccc ctatgtctca 3060

gtacaatctg ctctgatgcc gcatagttaa gccagtatct gctccctgct tgtgtgttgg 3120

aggtcgctga gtagtgcgcg agcaaaattt aagctacaac aaggcaaggc ttgaccgaca 3180

attgcatgaa gaatctgctt agggttaggc gttttgcgct gcttcgcgat gtacgggcca 3240

gatattgaca ttgattattg actaggcttt tgcaaaaagc ttgtttaaac cttgtatatt 3300

aacagcacat taacagctaa aaagaaaaac tcacataatc atattagttc atagaaacaa 3360

tgcatttgac atactccaac atccattcat gattttattt tattttttat ttatatattt 3420

tttttgagat ggagtctcgc tgtcacccag gctggagtgc aatggctcga tctcggctca 3480

ctgcaggctc cgcccccggc ggttcacgcc attctcctgc ctcagcctcc cgagtagctg 3540

ggactacagg cgcccgccac ctcgcccagc taattttttg tatttttagt agagacgggg 3600

tttcaccgtg ttagccagga tggtctcgat ctcctgacct cgtgatccac ccgcctcggc 3660

ctcccaaagt gctgggatta caggcgtgag ccaccgtgcc cggcccatga ttttaaaaaa 3720

acctctcaga aatagaaaca gagggaactt cctgaatttg attaaaaata cctgcaaaat 3780

cctagagcta atattatact taatggtgaa agactgaatg gttttcccct aagatggaga 3840

acaaggcacg gatgtccttg ctcaccactc ctattcaaca aaggactgga aaccagaaat 3900

aactttaatt ttttttgttc tcaagtgata ggttcacaga gaaaaagctt tactggatga 3960

acttttagat tactactttt atagagcagc agagataaaa gccaggtcga aaagtgcatg 4020

tggagtaagg aaatggacct agttcgacaa aagggctcag aacgactgcc cagatgagat 4080

tgtagacgca gctgtagttt actttctatc tggaagaaac ttcaagttat ccttaatttt 4140

ccaaggagac agtcacttac cttttaaaaa acattattag agaagcaccg ggcgggagca 4200

tatctagcat taaaaatgtg ggatgaatac catctctgct tggtaaaggt ggttgggaat 4260

cctgagagag ggcacctagt gtggcttctg catttttcac agtgcctgga ccacggctga 4320

aagtaactct tgcatgacat ttgacagaag aggaaaccga agctcagatt aaattccctg 4380

tccacagcgg gatcattcgg cacgagttcc tccctgtctg gaatgctctt ccccagcaat 4440

agatcctgcg gctcttccgt gtctcagtct aatgtcattc cgttccagga ttcccgactt 4500

cttaaagcat aaataatccc tccccaccct ctcattgtac tgttatgtaa gttattacaa 4560

tatgtcatta tatatttagt catactgctt taggtaatgt cttctccact gaactgtaag 4620

ctccatgagg gcaagagttc agtcggtttt acttaataat tagcacctag tacagtacta 4680

gcatagaatg aaggcctcgc aatttttttt aaatttattt ttagacaggg tcttgcgctg 4740

tcgcccaggc tggagtgcag tggtgcaacc tcggctcacg gcagcctcga cctttcggct 4800

ccagcgatcc tcccgcgtcg gcctccgggg tagctgggac tgcaggcgcg caccaccatg 4860

actggctaat tttttttttt ttttttttgt agacatgggg tctcgccatg ttgcccaggc 4920

tggttcctga gctcaagtga tcctcctgcc tcggcctccc aaagtgctgg gattacaggc 4980

gtgagcctca gcgcccagcc aagttagcct tttttaaacg tcctgtctcc ggaggttgcc 5040

gaagttggtt ttcttcggcc tccttctctc tcccaggccc agggctggga cgaggccggt 5100

tcccgcctgc aacctgcact gaagacggga accttgggag ccggtaccgg aacgctcgga 5160

aacggcacca aagtacgaat cctagggcgg aaaagcgtta ccaagacact cgtccccaga 5220

gccgcttcct gggactctct agcctcctac cgcttctcag tgatgttccg gtttccgccc 5280

tcctcctcgc gctgtttccg cctcttgcct tcggacgccg gattttgacg tgctctcgcg 5340

agatttgggt ctcttcctaa gccggcgctc ggcaaggtag gttggcggcc tgctctccga 5400

cagaactttt cttcttgggt tgaggaaaac gccttttgga gtcaggccct ggaggggcga 5460

gccttgctca cagggtgggg atacagccga ttacccgccc tgtgctttcc gatggcttct 5520

gcggggcgag cggggcctgg ccggggggtg cgggcgggag ggcgagccag cggcgcctgc 5580

agcccgggcc gcgtaacgct gaccgctgtg ccttcagttc tcccaggaga aagccgctag 5640

ccgccaccat gaagtacctc cttcctaccg ctgccgctgg cctgctgttg cttgccgctc 5700

agcctgcaat ggccgacatt cagatgaccc agtctcccag ctctctctcc gcttctgttg 5760

gtgacagagt tactattaca tgtagcgcct ctcaagacat ctctaattac ctgaattggt 5820

atcaacaaaa gcctggaaag gcacccaagg tgctgatcta tttcacaagc tcactgcatt 5880

ccggcgtgcc ctccagattt agcgggtcag gatctggaac cgactttaca ttgaccattt 5940

ccagtctgca acccgaagat ttcgctacct actactgtca gcagtacagt accgtgccct 6000

ggaccttcgg gcagggcacc aaagtcgaaa tcaagcggac agtcgcagcc ccatctgtat 6060

tcatatttcc accttcagac gagcagctca agagcggcac agcaagcgta gtttgcctgc 6120

tcaataactt ttatcctcgc gaggccaagg tccagtggaa agtggataac gctctccagt 6180

caggaaactc ccaggagagc gtcaccgagc aggactcaaa agatagcaca tactctttgt 6240

ctagtaccct tactctttct aaagccgact atgagaagca caaggtatat gcctgcgaag 6300

tgactcacca ggggctgtcc agtcccgtta ctaagtcctt taaccgaggt gaatgctagg 6360

cggccgctgc agggagattt cgattccacc gccgccttct atgaaaggtt gggcttcgga 6420

atcgttttcc gggacgccgg ctggatgatc ctccagcgcg gggatctcat gctggagttc 6480

ttcgcccacc ccaacttgtt tattgcagct tataatggtt acaaataaag caatagcatc 6540

acaaatttca caaataaagc atttttttca ctgcattcta gttgtggttt gtccaaactc 6600

atcaatgtat cttatcatgt ctgtataccg tcggcgcgcc gacggactcg agtcgtcgac 6660

<210> 17
<211> 45
<212> DNA
<213> Artificial

<220>
<223> primer

<400> 17
aactgcaggc cggccgcgga ggcctggtga cctaagcttg catgc 45

<210> 18
<211> 57
<212> DNA
<213> Artificial

<220>
<223> primer

<400> 18
attggttctg cagagcttgg cgcgccaagc ttaattaaat gagccatatt caacggg 57

<210>  19
<211>  20
<212>  DNA
<213>  Artificial

<220>
<223>  junction region

<400>  19
cagtgctgca atgataccgc                                                    20


<210>  20
<211>  20
<212>  DNA
<213>  Artificial

<220>
<223>  primer

<400>  20
ctgcacctga acttttgggc                                                    20


<210>  21
<211>  20
<212>  DNA
<213>  Artificial

<220>
<223>  primer

<400>  21
tcgggtgtac gggagatcat                                                    20


<210>  22
<211>  20
<212>  DNA
<213>  Artificial

<220>
<223>  primer

<400>  22
cagtacagta ccgtgccctg                                                    20


<210>  23
<211>  20
<212>  DNA
<213>  Artificial

<220>
<223>  primer

<400>  23
   aatacagatg gggctgcgac                                                     20

## Description

### TECHNICAL FIELD

The present invention relates to a method for stable and high expression of a DNA sequence of interest using a nucleic acid construct that comprises, in a mammalian artificial chromosome vector, the DNA sequence of interest, a matrix attachment region, and a replication origin, and to the nucleic acid construct.

### BACKGROUND ART

In recent years, the so-called biomedicines, such as proteins or nucleic acids, have been grately expected for use in treatment of diseases that is difficult to cure because of the lack of effective therapy, cancers, and the like. In particular, many antibody medicines have already been used in clinical settings or clinically tested because of their satisfactory effects and few adverse effects, and some of them are ranked high in pharmaceutical sales in the world. Unlike low molecular weight medicines, however, biomedicines have drawbacks such as a complicated production process and a high cost. In order to realize high reproducibility, that is, stable supply and low cost accordingly, it is critical to establish sophisticated production techniques.

In general, proteins such as antibodies are produced by gene recombination instead of chemical protein synthesis. Such technique comprises inserting DNA encoding a certain protein into a vectore such as plasmid in an expressible or operable manner, culturing prokaryotic or eukaryotic cells transformed by the vector, and recovering and purifying the generated proteins.

As Shimizu et al. (Hiroshima University) have conducted studies on the gene amplification mechanism of cancer cells, they discovered that plasmids comprising the matrix attachment region (MAR)/mammalian replication initiation region (IR) efficiently caused gene amplification in cancer cells or other cells and that such plasmids were incorporated into the extrachromosomal double minute (DM) (Non-Patent Document 1 and Non-Patent Document 2). Using such DM, specifically, multimeric plasmids can be prepared in the transfected cells and stably partitioned to daughter cells. Thus, plasmids can be amplified at high levels in mammalian cells (Patent Document 1 and Non-Patent Document 3).

Shimizu et al. have proposed several methods for amplifying genes of interest in mammalian animal cells.

For example, Patent Document 2 discloses that a replication fork from the replication initiation region does not allow a poly (A) addition sequence or replication fork inhibitory sequence to be present in a region where the replication and transcription from an arbitrary gene integrated into a vector collide with each other, and Patent Document 2 also discloses a vector that allows such region to include a matrix attachment region.

Patent Document 3 and Patent Document 4 each disclose that a polynucleotide of 500 bp to 10,000 bp comprising a telomere repeat sequence is introduced as a new element into a mammalian animal cell simultaneously with the vector described above.

Patent Document 5 discloses that an IR/MAR-containing sequence forms DM with a single-stranded or double-stranded hairpin structure.

In addition, Shimizu et al. discloses Non-Patent Documents 4 to 6 related to the invention described above.

Non-Patent Document 7 describes as follows. When antibodies were produced using the IR/MAR plasmid, the antibody production level increased highly in the *E. coli* cell line COLO 320DM and the Chinese hamster ovary cell line CHO DG44. In the CHO K1 cell line, while the antibody production level was significantly low even when the plasmid comprised IR/MAR. Thus, efficient gene amplification was not observed.

With respect to the IR/MAR gene amplification technique described above, the documents mentioned above do not substantially disclose the use of the mammalian artificial chromosome vector developed by the present inventors (Patent Documents 6 to 9 and Non-Patent Documents 8 and 9).

### PRIOR ART DOCUMENT

### Patent Documents

Patent Document 1: JP Patent No. 3755028
Patent Document 2: JP Patent No. 3882042
Patent Document 3: JP Patent No. 5688771
Patent Document 4: JP Patent No. 5688771
Patent Document 5: JP Patent No. 5283044
Patent Document 6: JP Patent No. 5557217
Patent Document 7: JP Patent No. 4997544
Patent Document 8: JP Patent No. 4895100
Patent Document 9: JP 2011-549044 A

### Non-Patent Documents

Non-Patent Document 1: Shimizu, N. et al., Cancer Res., 61:6987 to 6990, 2001
Non-Patent Document 2: Shimizu, N. et al., Cancer Res., 63:5281 to 5290, 2003
Non-Patent Document 3: Ohsaki, K. et al., PLos One, 12 (4): e0175585, 2017
Non-Patent Document 4: Shimizu, N. et al., Exp. Cell Res., 302 (2): 233 to 243, 2005
Non-Patent Document 5: Shimizu, N. et al., Nucleic Acids Res., 33 (19): 6296 to 6307, 2005
Non-Patent Document 6: Hashidume, T. et al., J. Cell Biochem., 101: 552 to 565, 2007
Non-Patent Document 7: Araki, Y. et al., PLos One, 7 (7): e41787, 2012
Non-Patent Document 8: Y. Yoshimura et al., Transgenic Research, 24: 717-727, 2015
Non-Patent Document 9: Y. Iida et al., ACS Synth. Biol., 21: 83-90, 2014

### SUMMARY OF THE INVENTION

### Problem to Be Solved by the Invention

As described in Patent Documents 3, 4, and the like, the presence of DM in addition to IR/MAR is necessary in order to amplify a DNA of interest (e.g., DNA encoding a protein). DM is an extrachromosomal circular DNA, and it comprises chromatin and is replicable, but it does not comprise a centromere or telomere. However, a gene amplified by IR/MAR has a problem that the expression of the gene is significantly lowered by silencing. In order to resolve the problem, it is essential to add a histone deacetylase (HDAC) inhibitor (e.g., butyrate) to a cell expressing the protein prepared by IR/MAR for removing the effect of silencing. This may adversely affect cell proliferation or damage cells. Thus, a protein production technique involving the use of IR/MAR is not suitable for use in a continuous culture system.

In order to resolve the problems described above, an object intended by the present inventors is to construct the IR/MAR system that does not comprise DM as an essential element, specifically to provide a method for high expression of a DNA sequence comprising: inserting IR/MAR and a DNA sequence of interest into a human artificial chromosome (HAC), or a mammalian artificial chromosome (MMAC) vector, suitable for stable gene expression comprising a mouse artificial chromosome (MAC) to prepare a novel IR/MAR-MMAC nucleic acid construct; and introducing the nucleic acid construct into a mammalian cell, thereby expressing the DNA sequence at a high level. Such technique has not yet been disclosed, and a possibility of gene amplification or stable protein production has not yet been known.

### Means for Solution of Problem

The present inventors conducted intensive studies in order to resolve the above-mentioned problems and, as a result, have now found that mammalian cells comprising a novel IR/MAR-MMAC nucleic acid construct would enable high expression of a DNA of interest.

Accordingly, the present invention includes the following features.
[1] A nucleic acid construct comprising a DNA sequence of interest, a matrix attachment region, and a replication origin in a mammalian artificial chromosome vector, and used for high expression of the DNA.
[2] The nucleic acid construct according to [1], wherein the mammalian artificial chromosome vector is a human artificial chromosome vector or a rodent artificial chromosome vector.
[3] The nucleic acid construct according to [2], wherein the rodent artificial chromosome vector is a mouse artificial chromosome vector.
[4] The nucleic acid construct according to [2], wherein the rodent artificial chromosome vector is a hamster-derived artificial chromosome vector.
[5] The nucleic acid construct according to [4], wherein the hamster-derived artificial chromosome vector is an artificial chromosome vector derived from Chinese hamster ovary (CHO) cell.
[6] The nucleic acid construct according to any of [1] to [5], wherein the matrix attachment region is derived from the matrix attachment region of an Igκ gene locus.
[7] The nucleic acid construct according to any of [1] to [6], wherein the replication origin is derived from the replication initiation region of a dihydrofolate reductase gene locus.
[8] The nucleic acid construct according to any of [1] to [7], wherein the DNA comprises a gene or gene locus encoding a protein, cDNA, recombinant DNA or modified DNA, or DNA comprising a gene control region and a reporter gene.
[9] The nucleic acid construct according to any of [1] to [8], wherein the protein encoded by the DNA sequence of interest is from a human.
[10] A mammalian cell for high expression of a protein encoded by a DNA sequence, which comprises the nucleic acid construct according to any of [1] to [9].
[11] The mammalian cell according to [10], which is a Chinese hamster ovary (CHO) cell.
[12] The mammalian cell according to [11], wherein the CHO cell is the CHO K1 cell line.
[13] A method for high expression of a protein encoded by a DNA sequence, comprising a step of culturing the mammalian cell according to any of [10] to [12] in a medium.
[14] A method for production of a protein encoded by a DNA sequence, comprising the following steps of: culturing the mammalian cell according to any of [10] to [12] in a medium; and recovering (or collecting) the produced protein.
[15] A non-human animal carrying the nucleic acid construct according to any of [1] to [9].
[16] The non-human animal according to [15], which is a rodent.
[17] The non-human animal according to [16], wherein the rodent is a mouse.
[18] The non-human animal according to [16], wherein the rodent is a rat.

The present invention provides a method for gene amplification using a novel IR/MAR-MMAC nucleic acid construct. With respect to the use of such method for gene amplification, for example, mammalian cells transformed with the nucleic acid construct comprising DNA encoding a useful protein of interst may be cultured to enable stable and high expression of the protein of interest, or alternatively, when such DNA is a reporter gene, the expression of the gene may be elevated in cells, so that the change in the expression level is easily detected, leading to enhancing a detection sensitivity.

This description includes the content disclosed in Japanese Patent Application No. 2017-213237 (filed November 2, 2017) from which the present application claims priority.

### BRIEF DESCRIPTION OF THE DRAWINGS

Fig. 1 shows the procedures for preparing the gene amplification system using the IR/MAR-HAC nucleic acid construct (Steps 1, 2, and 3). In the figure, the "HAC" represents a human artificial chromosome vector, the "loxP" represents a sequence used for integration of a gene into HAC, the "MAR" represents a matrix attachment region, and the "IR" represents a replication initiation region. In addition, the "EGFP" represents DNA encoding a green fluorescent protein.
Fig. 2 shows the FISH images that indicate differences in chromosomes (karyotypes) between Chinese hamster ovary (CHO) cell lines; i.e., the K1 cell line (A and C) and the DG44 cell line (B and D). In the figure, the arrows indicate large-size chromosomes. The number of such chromosomes is 3 for the CHO K cell line and 2 for the CHO DG44 cell line.
Fig. 3 shows the FISH images of all the chromosomes of the CHO K1 cell line and the CHO DG44 cell line. In the figure, the arrow indicates a large-size chromosome.
Fig. 4 shows the FISH images that indicate introduction of a normal human artificial chromosome (HAC) (indicated by arrow heads) into two CHO DG44 N10 cell clones; i.e., cl.10 (A) and cl.11 (B).
Fig. 5 shows the structure of pCX-EGFP CMV-loxP vector. In the figure, the "CAG pro" represents a hybrid promoter of cytomegalovirus (CMV) enhancer and chicken β-actin promoter, the "CMV" represents cytomegalovirus promoter, the "loxP" represents a sequence used for integration of a gene into HAC, the "EGFP" represents a highly sensitive green fluorescent protein gene, and the "b-Globulin polyA" represents a poly A signal derived from the β globulin gene.
Fig. 6 shows the structure of pCX-EGFP CMV-loxP Km vector. In the figure, the "pBR322 ori" represents the origin of plasmid pBR322, and the "Km" represents a kanamycin resistant gene.
Fig. 7 shows the structure of pCX-EGFP CMV-loxP dhfr vector. In the figure, the "p-SRalpha" represents a SRalpha promoter, the "pA" represents poly A, the "BSR" represents a blasticidin resistant gene, the "IR" represents a translation initiation region of the dihydrofolate reductase gene, the "MAR" represents a matrix attachment region derived from immunoglobulin κ gene, the "Dhfr" represents a dihydrofolate reductase gene, the "SV40prom" represents SV40 promoter, and the "pUC ori" represents the origin of plasmid pUC.
Fig. 8 shows the relative quantification (RQ) of the EGFP genome relative to NV1 (the internal standard) in the CHO DG44 N10 MAR-HAC cell clones 1, 2, 3, 10, 11, 12, 13, and 14. The control is the CHO DG44 cell carrying HAC that comprises a single (1) copy of the EGFP sequence integrated therein.
Fig. 9 shows the FISH images that indicate differences in the genome level of the EGFP gene on HAC of CHO DG44 N10 EGFP-HAC (Panel A) and CHO DG44 N10 MAR-HAC (Panel B). Panel B shows the results of Clone 1 (cl.1) and Clone 3 (cl.3). The arrow head indicates an EGFP signal on HAC.
Fig. 10 shows (A) the comparison of EGFP expression levels (bands) between CHO DG44 N10 EGFP-HAC and CHO DG44 N10 MAR-HAC as measured by Western blotting and (B) the comparison of EGFP signal levels detected by fluorescent microscopy. In Fig. 10B, Panels 1, 2, and 3 show CHO DG44 N10 MAR-HAC cl.1, cl.2, and cl.3, respectively.
Fig. 11 shows: (A), the presence of the EGFP gene on HAC (indicated by arrow head) in the nucleus of the CHO K1 N10 EGFP-HAC cl.27 cell, as detected by FISH analysis; and (B), the presence of the high-level EGFP signal detected by fluorescent microscopy.
Fig. 12 shows: (A), the presence of the EGFP gene on HAC in the CHO DG44 N10 EGFP-HAC cell clone (cl.5); and (B), the presence of the CHO DG44 N10 EGFP-HAC cell clone (cl.6), as detected by FISH analysis.
Fig. 13 shows the comparison of the EGFP gene expression levels (bands) on HAC between the CHO K1 cell line (CHO K1 N10 EGFP-HAC clone) and the CHO DG44 cell line (CHO DG44 N10 EGFP-HAC clone) as detected by Western blotting (WB). The results of the comparison indicate that the CHO K1 cell line more efficiently expresses the foreign gene (EGFP).
Fig. 14 shows the method for transferring HAC with integrated EGFP gene, which has been amplified in the CHO DG44 cell line (MAR-HAC), into CHO K1 cells by microcell-mediated chromosome transfer (MMCT). The CHO K1 cell after transfer is CHO K1R MAR-HAC. The arrow head indicates an EGFP signal on HAC.
Fig. 15 shows the microscopic images that indicate microcell induction in the CHO DG44 cell after treatment with 0.1 µg/ml colcemid for 72 hours. Panels A, B, C, and D show the images at the magnifications of x40, x100, x200, and x200, respectively. The arrow indicates a microcell.
Fig. 16 shows the FISH images that indicate EGFP signals on HAC in the CHO K1R N10 MAR-HAC cell clones cl.12, cl.13, and cl.18 (Panels A, B, and C, respectively) as detected by FISH analysis. The inserted image shows an enlarged view at the same magnification.
Fig. 17 shows the Western blot of the CHO K1R N10 MAR-HAC clones cl.3, cl.7, cl.12, cl.13, and cl.18 that indicate increased EGFP expression levels as the results of transfer of MAR-HAC from CHO DG44 N10 MAR-HAC cl.1 (the donor) to the CHO K1 cell line.
Fig. 18 shows: (A), the Western blot and FISH images that indicate the EGFP expression levels of the CHO K1R N10 MAR-HAC cell clones cl.3, cl.7, cl.12, cl.13, and cl.18; and (B), the graph indicating the relative expression levels of the EGFP protein. The control is CHO K1 N10 EGFP-HAC cl.27 (a single (1) copy of the EGFP gene).
Fig. 19 shows the EGFP expression levels detected by Western blotting at the population doubling levels (PDL), i.e., PDL3, PDL30, and PDL50, when the culture period of the CHO K1R MAR-HAC N10 cl.13 cell is represented by PDL (the upper of Panel A), the EGFP signals on HAC (the lower of Panel A), and FISH images (Panel B). Fig. 19 indicates that this clone is capable of maintaining 80% EGFP expression levels over a long culture period.

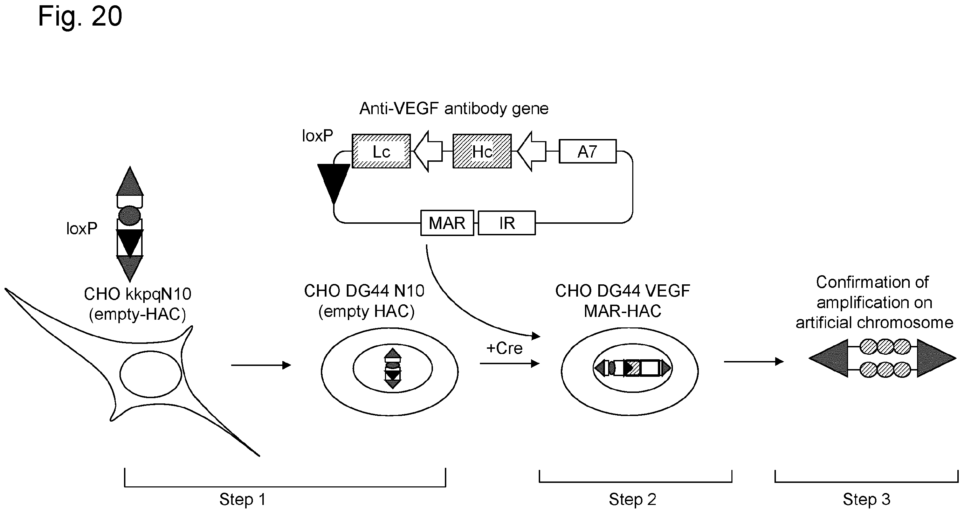

Fig. 20 shows the procedures for preparing a gene amplification system using the IR/MAR-HAC nucleic acid construct (Steps 1, 2, and 3). In the figure, the "HAC" represents a human artificial chromosome vector, the "loxP" represents a sequence used for integration of a gene into HAC, the "MAR" represents a matrix attachment region, and the "IR" represents a replication origin (i.e., a replication initiation region). In addition, the "A7" represents a gene enhancer sequence derived from human chromosome 11, the "Hc" represents a heavy chain protein of the anti-VEGF antibody gene, and "Lc" represents DNA encoding a light chain protein of the anti-VEGF antibody gene.
Fig. 21 shows the structures of pUCFa-RPS7-VEGF-Hc vector and pUCFa-RPS7-VEGF-Lc vector. In the figure, the "RPS7 promoter" represents a human RPS7 (ribosomal protein S7) gene promoter, the "Kozak" represents a DNA sequence that is recognized by the ribosome of an eukaryotic cell and serves as a signal for translation initiation, the "VEGF Hc ORF" represents a heavy chain protein gene of the anti-VEGF (vascular endothelial growth factor) antibody gene, and the "VEGF Lc ORF" represents a light chain protein gene of the anti-VEGF (vascular endothelial growth factor) antibody gene.

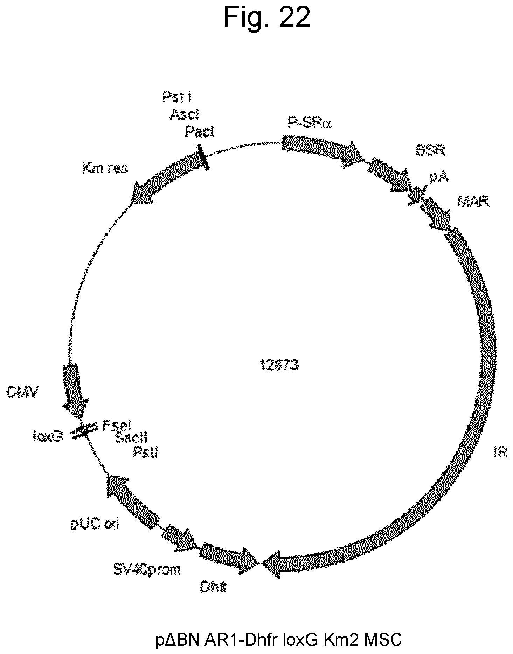

Fig. 22 shows the structure of pΔBN AR1-Dhfr loxG Km2 MSC vector. In the figure, the "p-SRalpha (p-SRα)" represents a SRalpha promoter, the "pA" represents poly A, the "BSR" represents a blasticidin resistant gene, the "IR" represents a translation initiation region of the dihydrofolate reductase gene, the "MAR" represents a matrix attachment region derived from immunoglobulin κ gene, the "Dhfr" represents a dihydrofolate reductase gene, the "SV40prom" represents SV40 promoter, the "pUC ori" represents an origin of plasmid pUC, the "PstI" represents a PstI restriction enzyme site, the "AscI" represents an AscI restriction enzyme site, the "PacI" represents a PacI restriction enzyme site, the "FseI" represents a FseI restriction enzyme site, the "SacII" represents a SacII restriction enzyme site, and the "PstII" represents a PstII restriction enzyme site.
Fig. 23 shows the structure of A7-VEGF MAR vector. In the figure, polyA and polyA2 each represent polyA.
Fig. 24 shows the structure of A7-VEGF loxG vector.
Fig. 25 shows the relative quantification (RQ) of the anti-VEGF antibody gene heavy chain (Panel A) and light chain (Panel B) relative to NV1 (the internal standard) of the CHO DG44 VEGF MAR-HAC cell clones 2, 8, 9, 10, and 13. The control is the CHO DG44 cell carrying HAC that comprises a single (1) copy of the anti-VEGF antibody gene sequence integrated therein.
Fig. 26 shows the FISH images that indicate differences in the genome levels of the anti-VEGF antibody gene on HAC in CHO DG44 VEGF-HAC (Panel A) and CHO DG44 VEGF MAR-HAC (Panel B). The arrow head indicates an anti-VEGF antibody gene signal on HAC.
Fig. 27 shows the differences in the anti-VEGF antibody gene expression levels on HAC in CHO DG44 VEGF-HAC and CHO DG44 VEGF MAR-HAC. This figure indicates that there is no significant difference in cell proliferation between the cells (Panel A), and that the antibody production levels determined by measurement of antibody concentrations are higher in case of the VEGF MAR-HAC than in case of the VEGF-HAC (Panel B). This figure also shows the results of ELISA indicating that the produced antibody specifically bind to the VEGF protein (Panels C and D).
Fig. 28 shows the procedures for preparing a gene amplification system using the IR/MAR-HAC nucleic acid construct (Steps 1, 2, and 3). In the figure, the "MAC" represents a mouse artificial chromosome vector, the loxP" represents a sequence used for integration of a gene into MAC, the "MAR" represents a matrix attachment region, and the "IR" represents a replication origin (i.e., a replication initiation region). In addition, "EGFP" represents DNA encoding a green fluorescent protein.
Fig. 29 shows the FISH images that indicate the introduction of a normal mouse artificial chromosome (MAC) (indicated by arrow head) into two CHO DG44 MAC cell clones; i.e., cl.611-1 (A) and cl.611-4 (B).
Fig. 30 shows the relative quantification (RQ) of the EGFP genome relative to NV1 (the internal standard) in the CHO DG44 MAR-MAC cell clone 3. The control is the CHO DG44 cell carrying HAC that comprises a single (1) copy of the EGFP sequence integrated therein.
Fig. 31 shows the FISH images that indicate differences in the genome levels of the EGFP gene on HAC in CHO DG44 EGFP-MAC (Panel A) and CHO DG44 MAR-MAC (Panel B). Panel B shows the results of the clone (cl.3). The arrow head indicates EGFP signals on HAC.
Fig. 32 shows: (A), the comparison of the EGFP expression levels (bands) between CHO DG44 EGFP-MAC and CHO DG44 MAR-MAC as measured by Western blotting; and (B), the comparison of the EGFP signal levels detected by fluorescent microscopy.
Fig. 33 shows the procedures for preparing a gene amplification system using the IR/MAR-HAC nucleic acid construct (Steps 1, 2, and 3). In the figure, the "tet-O HAC" represents a bottom-up type of human artificial chromosome vector, the "loxP" represents a sequence used for integration of a gene into MAC, and the "MAR" represents a matrix attachment region, and the "IR" represents a replication origin (i.e., a replication initiation region). In addition, the "EGFP" represents DNA encoding a green fluorescent protein.
Fig. 34 shows the FISH images that indicate the introduction of the normal bottom-up type of human artificial chromosome tet-O HAC (indicated by arrow heads) into two CHO DG44 tet-O HAC cell lines, i.e., cl.6 (A) and cl.9 (B).
Fig. 35 shows the relative quantification (RQ) of the EGFP genome relative to NV1 (the internal standard) in the CHO DG44 tetO-MAR HAC cell clones 6-1, 6-2, 6-3, 6-4, 6-5, 6-6, 6-7, 6-8, 9-1, 9-2, 9-3, 9-4, 9-5, 9-6, 9-7, and 9-8. The control is the CHO DG44 cell carrying HAC that comprises a single (1) copy of the EGFP sequence integrated therein.
Fig. 36 shows the FISH images that indicate differences in the genome levels of the EGFP gene on HAC in CHO DG44 EGFP-HAC (Panel A) and CHO DG44 MAR-tet-O HAC (Panel B). Panel B shows the results of clone 6-1 (cl.6-1) and clone 9-7 (cl.9-7). The arrow head indicates an EGFP signal on HAC.
Fig. 37 shows: (A), the Western blot and FISH images indicating EGFP expression levels in the CHO MAR-tetO HAC cell clones cl.6-1 and cl.9-7; and (B), the graph indicating the relative expression level of the EGFP protein. The control is CHO DG44 EGFP-HAC (a single (1) copy of the EGFP gene).

### EMBODIMENTS FOR CARRYING OUT THE INVENTION

The present invention will be described in more detail.

### 1. IR/MAR-MMAC nucleic acid construct

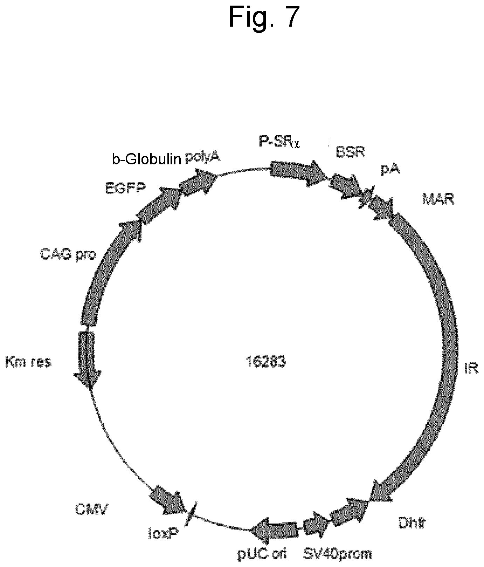

The IR/MAR-MMAC nucleic acid construct of the present invention comprises elements, such as at least one DNA sequence of interest, a matrix attachment region (MAR), and a replication origin (also referred to as "replication initiation region" (IR)), in a mammalian artificial chromosome vector (MMAC) that is maintained independently from the host cell chromosome and is capable of stably expressing the DNA of interest, and such IR/MAR-MMAC nucleic acid construct is used for high expression of the DNA of interest. The vector comprising such elements is referred to as the "IR/MAR-MMAC nucleic acid construct" (e.g., Fig. 7, in which the foreign DNA serves as the EGFP gene; however, this gene may be substituted with another DNA of interest as exemplified below) herein.

Before the particular elements are inserted, the mammalian artificial chromosome (MMAC) vector comprises a chromosome fragment comprising the centromere derived from a chromosome of a certain mammal and the telomere bound to the terminus of the chromosome fragment.

The chromosome fragment may be prepared by telomere truncation described in, for example, WO 00/10383. Specifically, an artificial chromosome vector of interest may be obtained by homologous recombination using a targeting vector that carries an artificial telomere sequence and telomere truncation in a mammalian cell that carries a mammalian chromosome. In this case, a long arm region and a short arm region (if any) located near the centromere of the chromosome are cleaved by telomere truncation and the telomere is bound to the cleavage site. Also, it is preferable that the chromosome fragment substantially comprise no endogenous gene. Alternatively, at least 99.9% of the endogenous genes are preferably deleted from the chromosome fragment, and at least 99.99% of the endogenous genes are more preferably deleted therefrom.

The term "artificial telomere sequence" used herein refers to a telomere sequence that is artificially added by telomere truncation as described in, for example, WO 00/10383. For example, both the human telomere sequence and the mouse telomere sequence comprise a repeat sequence of TTAGGG.

Examples of mammals used herein include, but are not particularly limited to, primates, such as humans, monkeys, chimpanzees, and gorillas, rodents, such as mice, rats, hamsters, and guinea pigs, ungulates, such as bovines, sheep, and goats, dogs, and cats, preferably humans, mice, and rats.

The chromosome of any of the mammals may be preferably used for preparation of an artificial chromosome vector. It is preferable to select a chromosome that enables a mammalian cell to be transformed to stably accept an artificial chromosome vector. When rodents cells (e.g., CHO cell lines, such as K1, pro-3, DG44, DUKX-X11, or S), mouse myeloma cells (e.g., NS0 or Sp2/0), or BHK cell lines (e.g., BHK21) are used as mammalian cells, for example, any of the rodent artificial chromosome vector (e.g., a mouse artificial chromosome (MAC) vector), an artificial chromosome vector derived from a hamster (e.g., an artificial chromosome vector derived from CHO), and a human artificial chromosome (HAC) vector may be introduced into rodent cells, and the mouse artificial chromosome vector may preferably be introduced thereinto. When a human cell line is used, in addition, it is preferable that the human artificial chromosome vector is introduced into a human cell line.

Concerning chromosomes, for example, mice have 40 chromosomes, rats have 42 chromosomes, and humans have 46 chromosomes. Any chromosomes may be used for preparation of a mammalian artificial chromosome (MMAC) vector. As a specific example of the use of such chromosome, the mammalian artificial chromosome (MMAC) vectors developed by the present inventors encompass the human artificial chromosome vector (JP Patent No. 4,895,100) and the mouse artificial chromosome vector (JP Patent No. 5,557,217 and JP Patent No. 4,997,544), and the chromosomes may be used to insert the elements, such as at least one DNA sequence of interest, a matrix attachment region (MAR), and a replication origin (i.e., a replication initiation region (IR)), into such vectors.

Specifically, the mammalian artificial chromosome (MMAC) vector may be prepared by the methods described in the patent documents exemplified above or the like. In addition, the vector comprises a DNA sequence insertion site into which foreign DNA is to be inserted. The DNA sequence insertion site is, for example, a site-directed recombinase recognition site. Examples of such systems include: a system of the Cre recombinase derived from bacteriophage P1 and the loxP sequence as a recognition site of the Cre recombinase (the Cre/loxP system); a system of the FLP enzyme derived from a budding yeast and the FRT sequence as a recognition site of the FLP enzyme (the Flp/FRT system); and a system of the ϕC31 integrase derived from *Streptomyces* phage and the attB/attP sequence as a recognition site of the integrase.

The mammalian artificial chromosome vector as used herein may be prepared by the top-down method that comprises performing mammalian chromosome-based chromosome modifications, or by the bottom-up method that comprises introducing a vector comprising a mammalian centromere sequence into mammalian cells (Yasuhiro Kazuki and Mitsuo Oshimura, The American Society of Gene & Cell Therapy 2011; doi:10.1038/mt.2011.136).

When preparing the IR/MAR-MMAC nucleic acid construct of the present invention, for example, a cassette comprising the DNA sequence of interest, the matrix attachment region (MAR), and the replication origin (i.e., the replication initiation region (IR)) may be inserted into a DNA sequence insertion site (e.g., the loxP sequence) of the mammalian artificial chromosome (MMAC) vector. In general, the matrix attachment region (MAR) and the replication origin (i.e., the replication initiation region (IR)) are preferably linked in that order from the 5' side, and such sequence is referred to as the "IR/MAR sequence" hereinbelow.

It is known that the IR/MAR sequence may be introduced in combination with a gene of interest into cells and may be used to amplify the copy number of the gene in the cells (e.g., Patent Documents 1 to 5). In the case of tumor cells, in addition, a plasmid comprising the IR/MAR sequence repeats multimerization to become a large cyclic molecule, and such large cyclic molecule is maintained in the form of a double minute (DM) in tumor cells. When DM is further integrated into the chromosome long arm of the nucleus in the tumor cell, it initiates the breakage-fusion-bridge (BFB) cycle to form a chromosomal homogeneously staining region (HSR), which serves as the gene amplification site in the tumor cell (Non-Patent Document 3). According to the conventional technique, however, continuous administration of an agent such as an HDAC inhibitor was necessary to keep the amplified gene to be expressed, and it was difficult to realize stable expression for a long period of time.

On the other hand, the IR/MAR-MMAC nucleic acid construct of the present invention is different from the DM or episome, although the mechanism is not elucidated, and, as a result, the construct is present on the the mammalian artificial chromosome (MMAC) vector in the state that foreign DNA is amplified in the nucleus, thus being clearly different from the DM in terms of its final form.

The IR/MAR-MMAC nucleic acid construct of the present invention comprises at least one DNA sequence of interest, the matrix attachment region (MAR), and the replication origin (i.e., the replication initiation region (IR)).

The matrix attachment region (MAR) comprises a polynucleotide derived from, for example, a matrix attachment region, such as mmunoglobulin light chain (Igκ) gene locus, Simian virus 40 (SV40) early region, or dihydrofolate reductase gene locus, although the region is not limited thereto (Tsutsui, K. et al., J. Biol. Chem.; 268: 12886-12894, 1993; Pommier, Y. et al., J. Virol.; 64:419-423, 1990; Shimizu, N. et al., Cancer Res.; 61: 6987-6990, 2001).

Examples of the replication origin (i.e., the replication initiation region (IR)) include, but are not limited to, replication initiation regions derived from the c-myc gene locus, the dihydrofolate reductase (DHFR) gene locus, and the β-globin gene locus (McWhinney, C. et al., Nucleic Acids Res.; 18: 1233-1242, 1990; Dijkwel, P.A. et al., Mol. Cell. Biol.; 8:5398-5409, 1988; Aladjem, M. et al., Science; 281:1005-1009, 1998).

Examples of DNA of interest include nucleic acids such as a gene or gene locus, cDNA, recombinant DNA, and modified DNA.

The term "protein" used herein refers to a protein, polypeptide, or peptide, unless otherwise noted. As long as it is industrially useful, the term refers to any protein, preferably a human-derived protein. Examples of such proteins include any proteins used for treatment, prevention, diagnosis, and the like, of diseases. Examples of such proteins include: antibody medicines, such as rheumatic arthritis-associated antibodies and cancer- or tumor-associated antibodies; therapeutic proteins, such as erythropoietin (EPO), G-CSF, GM-CSF, human growth hormone, blood coagulation proteins, platelet production-stimulating factor, and interferon; and interleukin, and other cytokines.

In the present invention, due to the use of the mammalian artificial chromosome (MMAC) vector, not only a gene,but also a gene locus can be inserted into the vector. When the protein to be expressed is an antibody protein, for example, the heavy chain gene locus (contained in chromosome 14 in case of human) and/or the light chain gene locus (contained in chromosome 2 (κ chain) and chromosome 22 (λ chain) in case of human), which comprise V region, D region, J region, and C region, may be used. When preparing a recombinant antibody, such as a single-stranded antibody comprising a heavy chain variable region (VH) ligated to a light chain variable region (VL) preferably via a linker, DNA encoding the antibody, may be used. In addition, in order to prepare therapeutic proteins as exemplified above, genes or cDNAs encoding the proteins and, optionally, modified DNAs may be used. The term "modified DNAs" used herein may include, for example, DNAs encoding a deletion, substitution, addition, or insertion of at least one amino acid of the proteins.

The IR/MAR-MMAC nucleic acid construct may further comprise one or two or more same or different types of elements, such as any of gene promoters, enhancers, poly A sequences, drug resistant genes, reporter genes (e.g., DNA encoding fluorescent proteins or luminescent proteins), DNA sequence insertion sites (e.g., loxP), and insulators. The elements may be inserted at an adequate site that is capable of causing the efficient amplification of a nucleic acid, such as DNA encoding a protein of interest. For example, the promoters and enhancers may be inserted at a given site in the 5'-untranslated region of the DNA. Examples of promoters include: viral promoters such as CMV, SV40, and CAG promoters; and non-viral promoters such as elongation factor (EF)-1 and mouse phosphoglycerate kinase (PGK) promoters.

### 2. Mammalian cell comprising the IR/MAR-MMAC nucleic acid construct

The present invention further provides a mammalian cell for high expression of DNA of interest that comprises the IR/MAR-MMAC nucleic acid construct described in section 1 above.

Mammalian cells include, but not particularly limited to, Chinese hamster ovary (CHO) cells, tumor cells, COS cells, NIH3T3 cells, and BHK cells, as long as the nucleic acid amplification of DNA of interest occurs. Examples of CHO cells include CHO K1 cells (e.g., ATCC CCL-61, RIKEN RCB0285, and RIKEN RCB0403), CHO-oro-3 cells, CHO DG44 cells, and CHO DUKX-X11 cells. Examples of tumor cells include human colon cancer cells (e.g., COLO 320HSR cells such as ATCC CCL-220.1), HeLa cells, and mouse myeloma cell lines (e.g., NS0 cell and Sp2/0 cell). Mammalian cells preferable for gene amplification are CHO cells, more preferably CHO DG44 cells. Mammalian cells preferable for gene expression are CHO cells, more preferably CHO K1 cells.

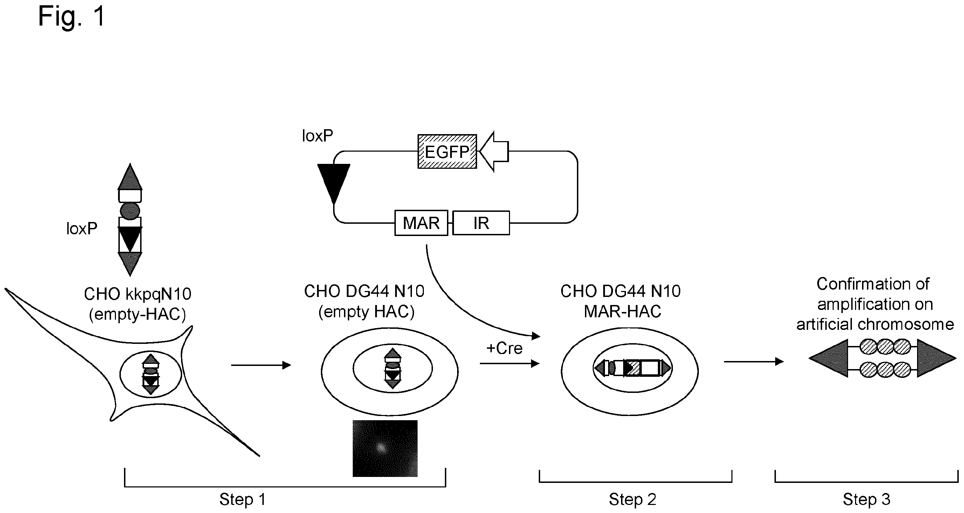

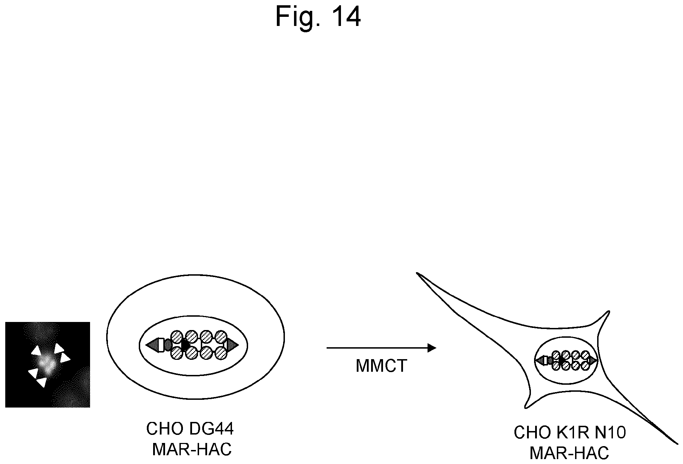

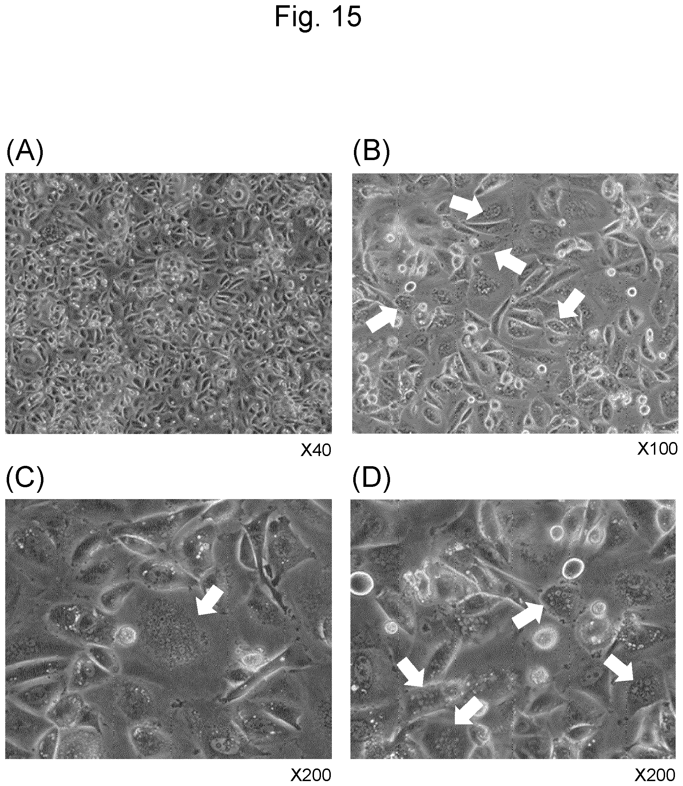

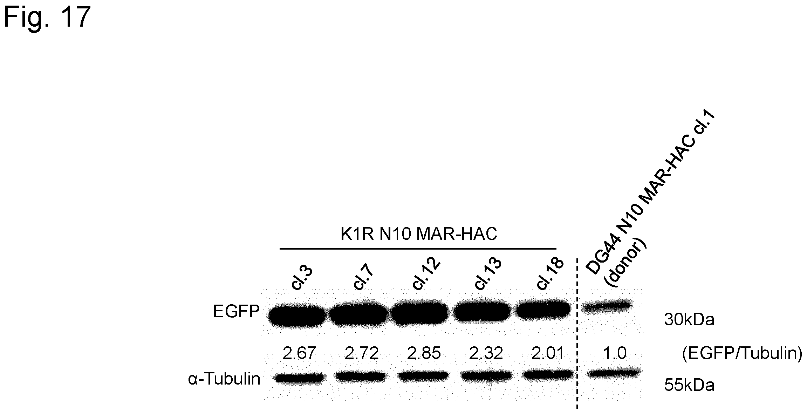

In order to introduce a cassette comprising the DNA sequence of interest, the matrix attachment region (MAR), and the replication origin (i.e., the replication initiation region (IR)) into a desired mammalian cell, for example, the Cre-loxP system may be used. Specifically, Cre recombinase may be allowed to act on a cultured mammalian cell line (e.g., CHO cell line) comprising the mammalian artificial chromosome (MMAC) vector prepared in the manner as described in section 1 above, and then a cassette comprising the DNA sequence of interest, the matrix attachment region (MAR), and the replication origin (i.e., the replication initiation region (IR)) is inserted at the loxP site of MMAC to obtain the mammalian cell line comprising the IR/MAR-MMAC nucleic acid construct (e.g., Fig. 1). Moreover, in order to transfer the IR/MAR-MMAC nucleic acid construct from the obtained mammalian cell line to another cell line, for example, the microcell-mediated chromosome transfer (MMCT) may be employed (e.g., Fig. 14). According to MMCT, for example, the mammalian cell line is treated with colcemid to form a microcell (e.g., Fig. 15), and the microcell is subjected to fusion to another cell line in the presence of polyethylene glycol to introduce the IR/MAR-MMAC nucleic acid construct into another cell line (e.g., Fig. 17). According to such method, a gene of interest may be amplified on the artificial chromosome, then gene expression or functions may be analyzed, and the amplified gene may be transferred to a more advantageous cell based on the results of analysis. As demonstrated in the examples below, for example, gene amplification may be performed on the mammalian artificial chromosome (MMAC) vector in the CHO cell line, such as the CHO DG44 cell line, and, when the MMAC subjected to gene amplification is transferred to the CHO K1 cell line, the CHO cell line exhibiting a very high expression level can be obtained.

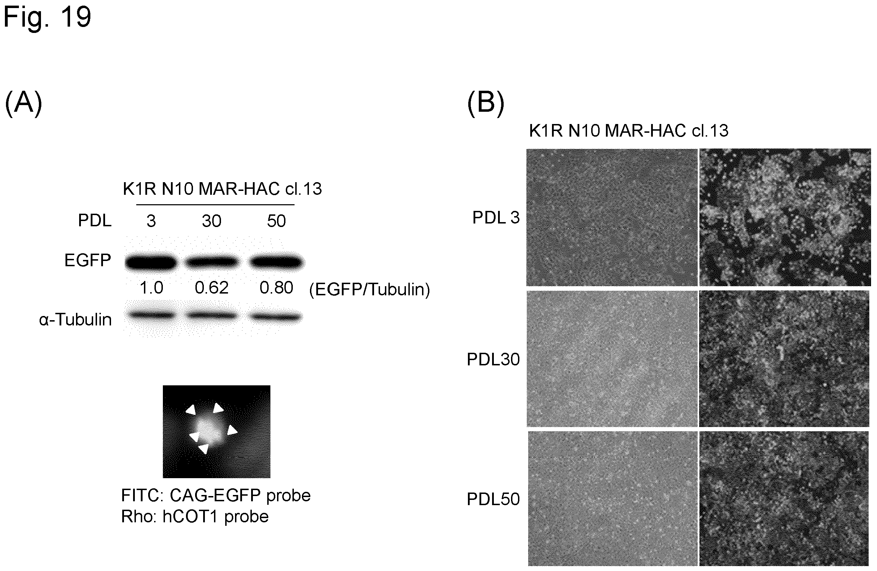

As described in the examples below, in the present invention, the CHO K1 cell line is preferable as the mammalian cell line into which the IR/MAR-MMAC nucleic acid construct is introduced. As shown in, for example, Fig. 17, the expression level of a protein of interest (e.g., EGFP) in the CHO K1 cell line is 2-3 times higher than that of the CHO DG44 cell line. In addition, the expression level of a protein of interest (e.g., EGFP in Fig. 17) in the CHO K1 cell line even at the population doubling level (PDL) 50 can be kept at 80% of the expression level at PDL3, as determined by the stability test during a period of long-term culture (Fig. 19). The protein expression level may be determined by, for example, Western blotting, ELISA, or quantitative RT-PCR.

### 3. Methods for high expression and high production of protein

### (A) Method for high expression of protein

The present invention further provides a method for high expression of a protein encoded by DNA of interest comprising a step of culturing a mammalian cell comprising the IR/MAR-MMAC nucleic acid construct in a medium.

### (A.1) High expression for protein production

For mammalian cell culture, a medium suitable for the type of cells to be cultured is selected. Examples of basal medium that can be generally used include MEM medium, DMEM medium, RPMI medium, F12 medium, Macy's 5A medium, and a mixture thereof (e.g., DMEM/F12). The basal medium may further be supplemented with at least one component, such as serum, serum replacement, antibiotics, glutamic acid, or pyruvic acid. The medium has a pH of, for example, approximately 7.0 to 7.4.

Culture is carried out in the air containing CO₂ at approximately 2% to 10% (approximately 5%, in general), and temperature of about 35°C to 40°C (about 37°C, in general) is suitable.

A culture period is generally 1 day to 3 months, preferably 1 day to 2 months.

Any culture technique may be selected from among, for example, batch culture, flask culture, fed-batch culture, continuous culture, suspension culture, shake culture, agitation culture, and circulation culture. In order to suppress cell aggregation, a culture solution may be allowed to, for example, pass through a hollow-fiber filter to disintegrate the aggregate during culture.

A culture apparatus used for animal cell culture may be used. The apparatus is preferably equipped with aeration, temperature control, agitation, pH adjustment, and DO adjustment functions. Culture may be carried out using, for example, a fermenter tank culture apparatus, an airlift culture apparatus, a fluidized bed culture apparatus, a hollow-fiber culture apparatus, a roller bottle culture apparatus, a packed bed culture apparatus, or a bag culture apparatus.

### (A.2) High expression for monitoring biological function or behavior in cell

Another example of high expression of a protein includes high expression of a reporter gene used for monitoring the behavior or biological functions of a protein of interest in mammalian cells. For example, a reporter gene (e.g., DNA encoding a fluorescent protein or a luminescent protein) comprising a gene control sequence (e.g., a promoter or enhancer) ligated thereto may be operably inserted into DNA of interest. As a result of high expression of the reporter gene, detection sensitivity of reporter assays can be enhanced. This enables observation of the behavior of DNA of interest expressed by certain stimulation in cells using a florescence or luminescence as the indicator. Such observation may be generally carried out in laboratories, and cell culture may be thus performed using a solid medium in accordance with a cell type. In general, the observation may also be carried out using, for example, a fluorescent microscope with a camera.

### (B) Method for high production of protein

The present invention further provides a method for producing a protein of interest comprising the following steps of: culturing mammalian cells comprising the IR/MAR-MMAC nucleic acid construct in a medium; and recovering (or collecting) the produced protein.

The step of culture is as described in (A.1) above.

In the step of cell recovery, for example, the supernatant or the cells after centrifugation, filtration, or other means may be subjected to column chromatography, such as affinity column chromatography, gel filtration chromatography, or ion exchange chromatography, filtration, ultrafiltration, salting out, dialysis, or a combination thereof, in order to obtain the protein produced by the culture. It is intended that the method of recovery is not limited to the methods described above and comprises any methods that can generally be employed for purification of proteins.

When the protein is particularly a glycoprotein such as an antibody, the glycoprotein may be carried out using a protein A column, a protein G column, a protein L column, or an IgG-binding peptide column.

### 4. Non-human animal carrying the IR/MAR-MMAC nucleic acid construct

The present invention further provides a non-human animal that carries the IR/MAR-MMAC nucleic acid construct.

A non-human animal may be prepared by, for example, introducing the nucleic acid construct into an ES cell or iPS cell, introducing the resulting cell into the blastocyst, transplanting the blastocyst nto the uterus of a surrogate mother, and allowing the surrogate mother to give birth to animals. The method of preparation is described below.

An ES cell may be established and maintained by removing an inner cell mass from the blastocyst of the fertilized egg of an animal of interest and by using the mitomycin C-treated mouse fetal fibroblast cell as a feeder (M. J. Evans and M. H. Kaufman, Nature, 292: 154-156, 1981).

The iPS cells generate a colony within approximately 3 to 5 weeks by introducing a certain reprogramming factor (DNA or a protein) into somatic cells (including somatic stem cells) and conducting culture and passage culture in an adequate medium. As reprogramming factors, for example, a combination of Oct3/4, Sox2, Klf4, and c-Myc, a combination of Oct3/4, Sox2, and Klf4, a combination of Oct4, Sox2, Nanog, and Lin28, and a combination of Oct3/4, Sox2, Klf4, c-Myc, Nanog, and Lin28 are known (K. Takahashi and S. Yamanaka, Cell 126: 663-676, 2006; WO 2007/069666; M. Nakagawa et al., Nat. Biotechnol., 26: 101-106, 2008; K. Takahashi et al., Cell 131: 861-872, 2007; J. Yu et al., Science 318: 1917-1920, 2007; and J. Liao et al., Cell Res., 18, 600-603 2008). For example, the mitomycin C-treated mouse fetal fibroblast cell line (e.g., STO) is used as the feeder cell, and vector-introduced somatic cells (approximately 10⁴ to 10⁵ cells/cm²) are cultured on the feeder cell layer using an ES cell culture medium at approximately 37°C. The feeder cell is not essential (Takahashi, K. et al., Cell 131: 861-872, 2007). Examples of basal media include Dulbecco's modified Eagle medium (DMEM), Ham's F-12 medium, and a mixture thereof. As an ES cell culture medium, for example, a mouse ES cell culture medium or a primate ES cell culture medium (ReproCELL Incorporated, Japan) may be used.

ES cells and iPS cells are known to contribute to the germ lines. Thus, such cells into which the IR/MAR-MMAC nucleic acid construct comprising the DNA sequence of interest has been introduced may be injected into the embryonic blastocyst of a mammal of the same species as with the origin of the cells, and the embryos are transplanted into the uterus of the surrogate mother to give birth to animals. Thus, non-human animals (or transgenic non-human animals) may be prepared. Further, the resulting female and male transgenic animals may be subjected to crossing to prepare homozygous animals and offspring animals thereof.

When a heterologous (e.g., human) protein encoded by DNA of interest is to be prepared using the non-human animal, for example, an animal in which the endogenous gene equivalent to the DNA of interest has been deleted or completely inactivated may be used.

Examples of non-human animals include rodents, such as mice, rats, and hamsters.

### EXAMPLES

The present invention will be described in more detail with reference to the following examples, although the technical scope of the present invention is not limited to these examples.

### [Example 1] Establishment of CHO DG44 cell line carrying HAC vector derived from human chromosome 21

### [A] Introduction of HAC from CHO K1 cell comprising human chromosome 21-derived HAC vector into CHO DG44 cell

In order to insert and amplify a gene of interest into/on the HAC in the CHO DG44 cell that exhibits high gene amplification efficiency achieved by the IR/MAR sequence (Y. Araki et al., Plos. One, 7.7. e41787, 2012), the HAC comprising the DNA sequence insertion site (loxP) is introduced into the CHO DG44 cell (Fig. 1, Step 1).

### [A.1] Microcell fusion and isolation of drug resistant clone

The used as the chromosomal donor cell was the CHO kkpqN cl.10 cell line derived from the CHO K1 cell that carries the HAC vector derived from human chromosome 21 (hereafter, referred to as "CHO kkpqN10," JP 2007-295860 A). As the recipient cell, the CHO DG44 cell (provided by Dr. L.A. Chasin, Columbia University) was used. The CHO kkpqN10 cells were inoculated into twelve 25-cm² centrifuge flasks (Nunc) and cultured in a medium (20% FBS, F12) containing colcemid (0.1 µg/ml, Gibco) for 72 hours to induce microcell formation. The centrifuge flasks were filled with a cytochalasin B (10 µg/ml in F12, Sigma) solution kept warm at 37°C in advance, the centrifuge flasks were inserted into an acrylic centrifuge, and centrifugation was then carried out at 37°C and 8,000 rpm (11,899 x g) for 1 hour (JLA-10.5 rotor, Beckman). The microcells were suspended in the serum-free DMEM medium, recovered, and filtered and purified using SWINNEX-25 (Millipore) equipped with 8-µm, 5-µm, and 3-µm filters (Whatman), successively. The purified microcells were resuspended in 2 ml of F12 containing Phytohemagglutinin-P (Difco) at 50 µg/ml. The resuspended microcells were added in an amount of 1 ml each in two 6-cm dishes in which the CHO DG44 cells had been cultured to reach 90% saturation and then allowed to stand at 37°C for 15 minutes. PEG1000 (final concentration: 50% (w/v), Sigma) and DMSO (final concentration: 7% (w/v), Sigma) were dissolved in DMEM, the solution filtered through a 0.22-µm filter (Corning) was applied to the cells over a period of 1 minute, and the cells were washed with serum-free DMEM. The cells were cultured in an F12 medium containing 10% FBS for 24 hours, dispersed by trypsin treatment, and then inoculated into six 10-cm dishes. The cells were cultured in a selection medium (10% FBS, F12) containing blasticidin (4 µg/ml) for 2 weeks, and drug-resistant colonies were then obtained. Microcell fusion was carried out twice to isolate 12 resistant colonies, which colonies were amplified and then subjected to the subsequent analysis (clone name: CHO DG44 N10).

### [A.2] Selection of resistant clone

### [A.2.1] Mono-color FISH analysis

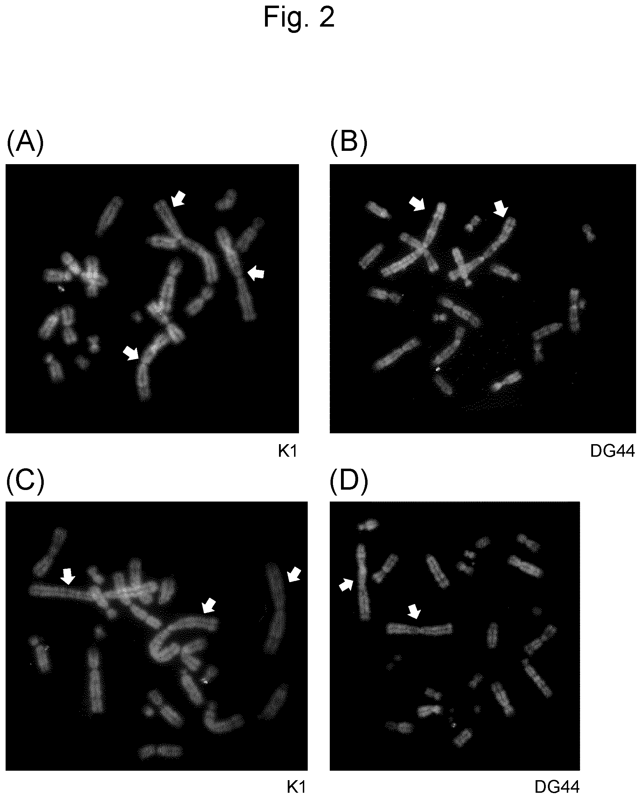

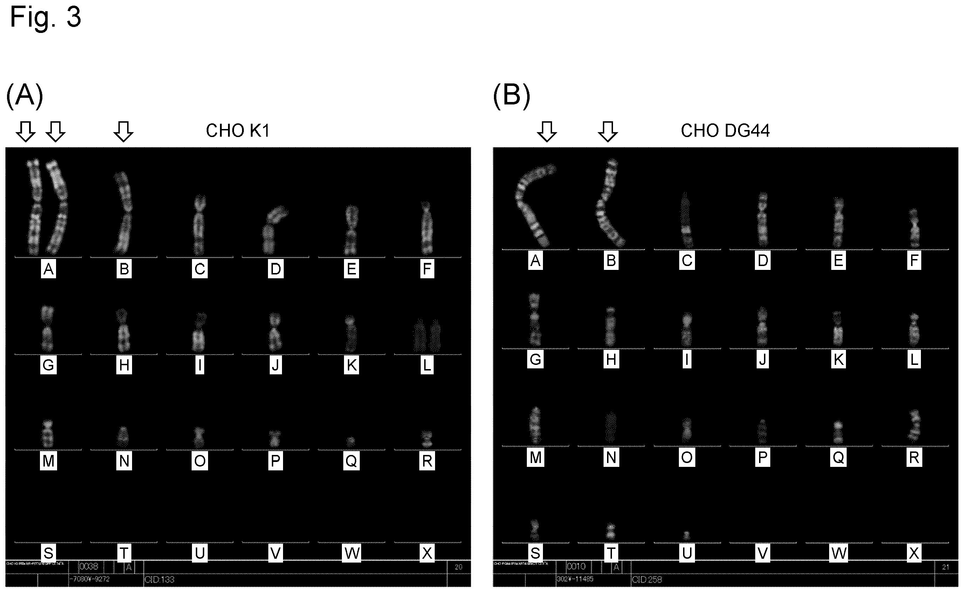

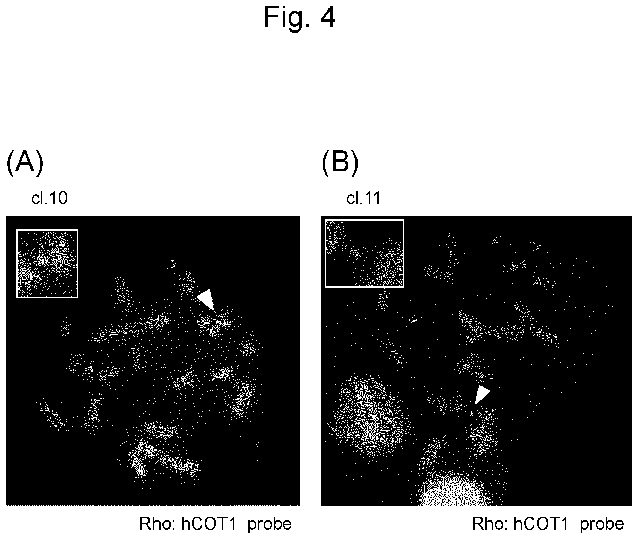

From among the 12 CHO DG44 N10 cell clones obtained above, 4 clones were selected and subjected to FISH analysis using human Cot-1 DNA as a probe in accordance with the method described in the report by Shinohara et al. (Human Molecular Genetics, 10: 1163-1175, 2001). It is possible to distinguish the CHO K1 cell-derived CHO kkpqN10 cell line from the CHO DG44 cell line on the basis of the karyotypic features, i.e. that, while the CHO K1 cell line comprises 3 large chromosomes, the CHO DG44 cell line comprises 2 large chromosomes (Fig. 2 and Fig. 3). As a result of signal detection using probes in addition to the karyotyping, introduction of normal HAC into two CHO DG44 N10 cell clones (cl. 10 and cl. 11) with a retention rate of 90% or higher was confirmed (Fig. 4).

From the results above, it was concluded that the human artificial chromosome (HAC) vector comprising the DNA sequence insertion site had been introduced into the CHO DG44 cell line.

### [Example 2] Introduction of the IR/MAR sequence and the EGFP gene into HAC

[A] A method for inserting the IR/MAR sequence and the EGFP gene into the HAC vector derived from human chromosome 21 is described. As described in Example 1, the CHO DG44 cell that carries the HAC vector derived from human chromosome 21 comprising a loxP site introduced thereinto was prepared. Separately, a plasmid comprising the loxP sequence, the IR/MAR sequence, and the EGFP gene expression unit was prepared, Cre recombinase was allowed to transiently express therein to cause site-directed recombination with the loxP sequence, and the plasmid vector was thus inserted into the human artificial chromosome. The recombinants were selected using acquisition of G418 resistance (reconstruction of the promoter split neo gene expression unit) as an indicator.

### [A.1] Construction of a plasmid vector that carries the loxP sequence and the EGFP gene

### [A.1.1] Insertion of the loxP sequence into the EGFP-expressing vector

The loxP sequence to be integrated into HAC was amplified using the pPAC4 vector (obtained from BACPAC Resource Center, Children's Hospital Oakland Research Institute) as a template.

The primer oligonucleotide sequences prepared based on the nucleotide sequence obtained from the GenBank database (Accession Number: U75992.1) are shown below:
CMV loxP stuI F: 5'-GAAGGCCTGGAGTTCCGCGTTACATAACTTACGGT-3' (SEQ ID NO: 1)
CMV loxP stuI R: 5'-GAAGGCCTGGTGACCTAAGCTTGCATGCAACTTCGT-3' (SEQ ID NO: 2)

PCR was carried out using the ProFlex PCR System (Applied Biosystems) as a thermal cycler and KOD-plus- (Toyobo Co., Ltd.) as Taq polymerase. Buffers and dNTPs (dATP, dCTP, dGTP, and dTTP) included in the kit were used under the recommended conditions. Temperature and cycle conditions were as follows: after thermal denaturation at 94°C for 2 minutes, a cycle of 98°C for 10 seconds and 68°C for 50 seconds was conducted for 16 cycles. The PCR product was purified using the Wizard SV Gel and PCR Clean-Up System kit (Promega Corporation). Thereafter, the resultant was cleaved with the restriction enzyme StuI (NEB), electrophoresed on 1% agarose gel, cleaved from the gel, and then purified using the Wizard SV Gel and PCR Clean-Up System kit (Promega Corporation).

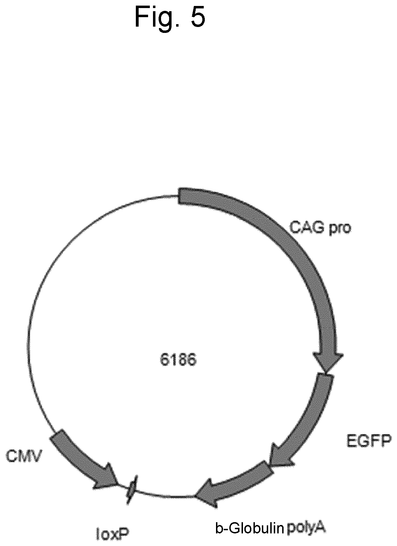

The DNA fragment of the purified loxP sequence digested with the restriction enzyme StuI (NEB) and blunt-ended at both ends was cloned into the StuI site of the pCX-EGFP plasmid vector having the EGFP expression unit using Ligation high v2 (Toyobo Co., Ltd.) in accordance with the recommended protocol. A vector comprising the loxP sequence cloned thereinto in the inverse direction from the EGFP sequence was designated as the pCX-EGFP CMV loxP vector (Fig. 5).

### [A.1.2] Conversion of drug selection marker in pCX-EGFP CMV loxP vector

Because both the pCX-EGFP CMV loxP vector and the pΔBN AR1-Dhfr vector comprising the IR/MAR sequence integrated therein (provided by Dr. Noriaki Shimizu, Hiroshima University) comprise the ampicillin resistant gene (Amp), when the pCX-EGFP CMV loxP vector is inserted into the pΔBN AR1-Dhfr vector, then many self-ligating plasmid vectors are generated, but a plasmid comprising the pCX-EGFP CMV loxP vector sequence cloned into the pΔBN AR1-Dhfr vector might not be obtained. In order to improve efficiency when inserting the pCX-EGFP CMV loxP vector into pΔBN AR1-Dhfr comprising the IR/MAR sequence integrated therein, an experiment of converting the ampicillin resistant (Amp) gene of the pCX-EGFP CMV loxP vector into the kanamycin resistant (Km) gene was carried out. Here, details of the pΔBN AR1-Dhfr plasmid are described in C. Noguchi et al. Plos One. 7. 12. e52990. 2012.

The Km gene was amplified using the pPAC4 vector (described above) as a template. The primer oligonucleotide sequences prepared based on the nucleotide sequence obtained from the GenBank database (Accession Number: U75992.1) are shown below:
Km SpeI PAC4 F: 5'-AAAAGTACTCCAATTAACCAATTCTGATTAGAAAAACT-3' (SEQ ID NO: 3)
Km SpeI PAC4 R: 5'-GGACTAGTAAAGCCACGTTGTGTCTCAAAATCTCTGAT-3' (SEQ ID NO: 4)

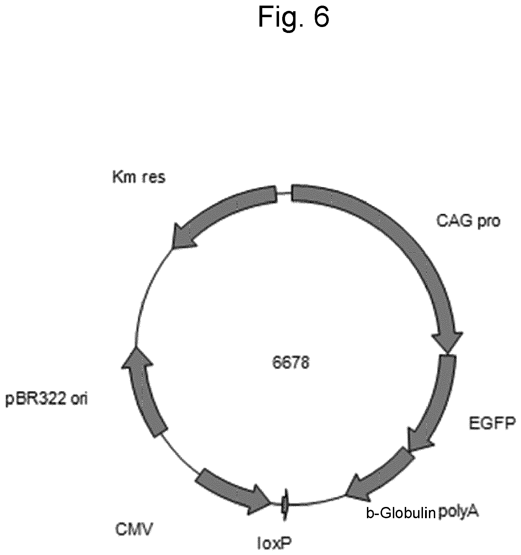

PCR was carried out using the ProFlex PCR System (Applied Biosystems) as a thermal cycler and KOD-plus- (Toyobo Co., Ltd.) as Taq polymerase. Buffers and dNTPs (dATP, dCTP, dGTP, and dTTP) included in the kit were used under the recommended conditions. Temperature and cycle conditions were as follows: after thermal denaturation at 94°C for 2 minutes, a cycle of 98°C for 30 seconds, 60°C for 30 seconds, and 72°C for 1 minute and 10 seconds was conducted for 16 cycles. The PCR product was purified using the Wizard SV Gel and PCR Clean-Up System kit (Promega Corporation). Thereafter, the resultant was cleaved with the restriction enzyme ScaI/SpeI (NEB), electrophoresed on 1% agarose gel, cut out from the gel, and then purified using the Wizard SV Gel and PCR Clean-Up System kit (Promega Corporation). The DNA fragment of the purified Km gene digested with the restriction enzyme StuI (NEB) and blunt-ended at both ends was cloned into the ScaI/SpeI site of pCX-EGFP CMV loxP having the EGFP expression unit using Ligation high v2 (Toyobo Co., Ltd.) in accordance with the recommended protocol. A vector comprising the Km gene cloned thereinto was designated as the pCX-EGFP CMV loxP Km2 vector (Fig. 6).

### [A.2] Insertion of the EGFP loxP sequence into the IR/MAR vector

The pCX-EGFP CMV loxP Km2 vector was cleaved with the restriction enzyme PstI (NEB), electrophoresed on 1% agarose gel, cut out from the gel, and then purified using the Wizard SV Gel and PCR Clean-Up System kit (Promega Corporation). The end of the DNA fragment of the linearized pCX-EGFP CMV loxP Km2 vector was dephosphorylated using the rAPId Alkaline Phosphatase kit (Roche) in accordance with the recommended protocol in order to suppress self-ligation of the vector DNA fragment. The pΔBN AR1-Dhfr vector was also cleaved with the restriction enzyme PstI (NEB), electrophoresed on 1% agarose gel, cut out from the gel, and then purified using the Wizard SV Gel and PCR Clean-Up System kit (Promega Corporation). Subsequently, the dephosphorylated DNA fragment of the pCX-EGFP CMV loxP Km2 vector was ligated to the DNA fragment of the pΔBN AR1-Dhfr vector using Ligation high v2 (Toyobo Co., Ltd.) in accordance with the recommended protocol.

The plasmid vector to be integrated into HAC, which compriises the EGFP gene, the loxP sequence, and the IR/MAR sequence and was prepared by the steps described above, was designated as the pCX-EGFP loxP dhfr vector (Fig. 7).

### [A.3] Integration of the pCX-EGFP loxP dhfr vector into HAC using the Cre/loxP system

### [A.3.1] Gene transfer and isolation of drug resistant clone

Gene trasfer was carried out using lipofection. In accordance with the protocol of Lipofectamine LTX (Invitrogen), 0.5 µg of the Cre-expressing pBS185 CMV-Cre vector (available from Addgene; Plasmid No. #11916) and 1 µg of the pCX-EGFP loxP dhfr vector were introduced into the CHO DG44 N10 cl.11 cells carrying the HAC vector for gene integration in 6 wells that had reached 90% confluency. Twenty four hours after transfection, the cells were inoculated into six 10-cm dishes each containing a selection medium consisting of F12 medium supplemented with 10% FBS and G418 300 µg/ml, and cultured in the medium for 2 weeks, thereby leading to appearance of resistant colonies, which contained many clones obtained by the single introduction. From among the obtained colonies, 24 clones were isolated, amplified, and then subjected to the subsequent analysis (clone name: CHO DG44 N10 MAR-HAC).

In order to prepare a control HAC that does not comprise the IR/MAR sequence, the gene transfer into the CHO DG44 N10 cl.11 cells was carried out in the same manner as that described above, except for the use of the pCX-EGFR loxP Km2 vector instead of the pCX-EGFP loxP dhfr vector. As a result, 3 clones were isolated, amplified, and then subjected to the experiment described below (clone name: CHO DG44 N10 EGFP-HAC).

### [A.4] Selection of drug resistant clone

### [A.4.1] PCR analysis

In order to extract the genomic DNA of the G418-resistant cell line and select a recombinant as a template, PCR was carried out using the primers described below to comfirm whether or not the EGFP and the IR/MAR sequence have been inserted into the human artificial chromosome (HAC) vector in a site-directed manner, in two junction regions appearing by recombination at the loxP region. The primer sequences are shown below.

### Neo junction region

CMV586: 5'-CGTAACAACTCCGCCCCATT-3' (SEQ ID NO: 5)
Neo817: 5'-GCAGCCGATTGTCTGTTGTG-3' (SEQ ID NO: 6)

### Another junction region

CMV188: 5'-GGGCGTACTTGGCATATGAT-3' (SEQ ID NO: 7)
CX-EGFP HAC June R1: 5'-ATCCGCTCACAATTCCACACA-3' (SEQ ID NO: 8)

PCR was carried out using the ProFlex PCR System (Applied Biosystems) as a thermal cycler and KOD-FX- (Toyobo Co., Ltd.) as Taq polymerase. Buffers and dNTPs (dATP, dCTP, dGTP, and dTTP) included in the kit were used under the recommended conditions. Temperature and cycle conditions were as follows: after thermal denaturation at 95°C for 10 minutes, a cycle of 94°C for 30 seconds, 55°C for 30 seconds, and 72°C for 1 minute and 30 seconds was conducted for 35 cycles. Out of 24 isolated CHO DG44 N10 MAR-HAC clones, 12 clones were analyzed and found to be positive. That is, the 12 clones were determined to comprise the EGFP and the IR/MAR sequence inserted into HAC and were subjected to the subsequent analysis (Fig. 1, Step 2).

### [B] Amplification of the EGFP gene by the IR/MAR sequence on the HAC vector

### [B.1.1] Confirmation of gene amplification via genome PCR analysis

In order to confirm amplification of EGFP gene based on integration of the IR/MAR sequence into HAC, the genome was extracted from the CHO DG44 N10 MAR-HAC clone, and comparative analysis was performed by real-time PCR using the CHO DG44 N10 EGFP-HAC clone comprising a single copy of the EGFP gene integrated therein (DG44 EGFP-MAC cl.12) as a control. (Note: the purpose of the PCR analysis was not to measure the absolute copy number of EGFP but was intended to conduct the experiments to simply determine whether or not the EGFP was amplified in comparison with the control.)

PCR was carried out using the StepOne real-time PCR System (Applied Biosystems) as a thermal cycler and, as Taq polymerase and a buffer, Power SYBR Green PCR master Mix (ThermoFisher) under the recommended conditions.

The primer sequences used are shown below. The EGFP primers and the NV1 primers as the genomic internal standards were prepared, respectively (Nissom PM et al., Biologicals, 35, 211-5, 2007).
EGFP gqPCR F: 5'-CTTCTTCAAGTCCGCCATGC-3' (SEQ ID NO: 9)
EGFP gqPCR R: 5'-GGTCTTGTAGTTGCCGTCGT-3' (SEQ ID NO: 10)
NV1 CHO gPCR ref F: 5'-ACAGGTTTCTGCTTCTGGCA-3' (SEQ ID NO: 11)
NV1 CHO gPCR ref R: 5'-CATCAGCTGACTGGTTCACA-3' (SEQ ID NO: 12)

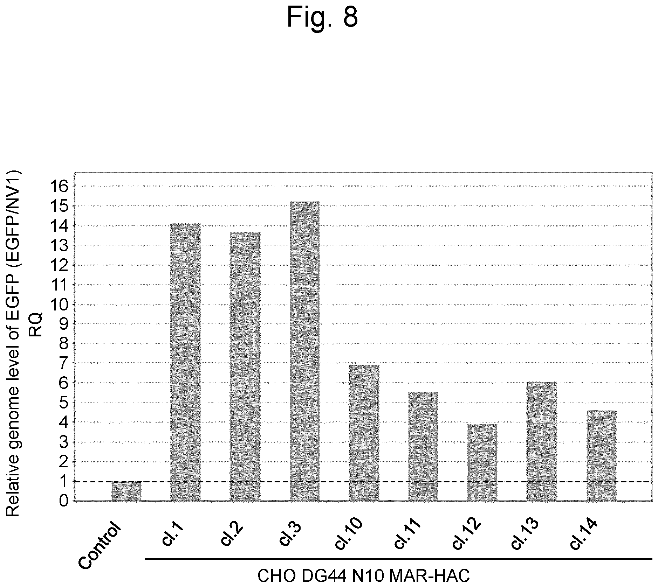

From among the 24 CHO DG44 N10 MAR-HAC clones, 8 clones were analyzed by real-time PCR. As a result, all the analyzed clones were found to comprise a greater number of the EGFP sequences compared with the control CHO DG44 N10 EGFP-HAC. Among them, 3 clones comprising a particularly great number of EGFP sequences (cl.1, cl.2, and cl.3) were subjected to the subsequent analysis (Fig. 8).

### [B.1.2] Two-color FISH analysis

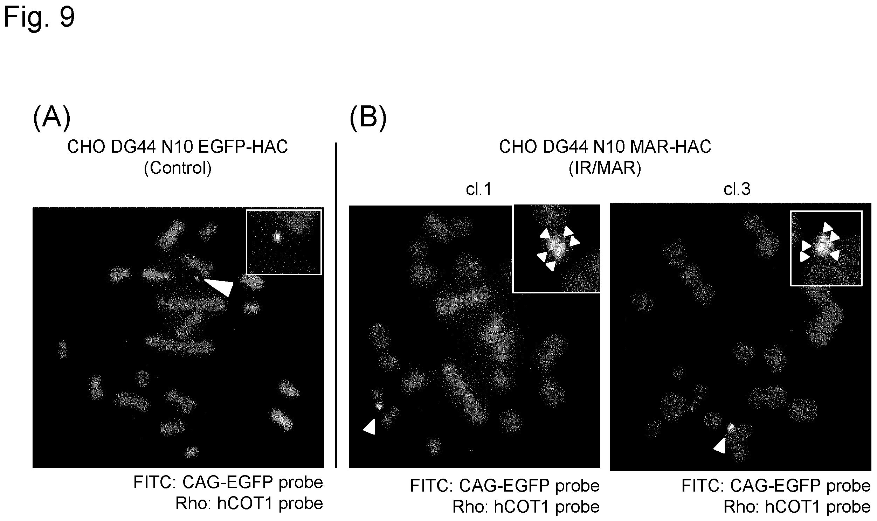

On the basis of the above results, the 3 clones were selected and subjected to two-color FISH analysis according to Matsubara et al. (FISH experimental protocol, Shujunsha Co., Ltd., Japan, 1994). FISH analysis was carried out using human cot-1 DNA and the pCX-EGFP plasmid as probes. As a result, all the clones were found to have larger chromosomes and a larger number of EGFP signals on human cot-1 signals, compared with the control (Fig. 9). From these results, it was determined that the EGFP gene was amplified on HAC, and the clones were subjected to the subsequent expression analysis.

### [B.2] Analysis of EGFP expression level by Western blotting

In order to determine whether or not the EGFP expression of the CHO DG44 N10 MAR-HAC clone was increased accompanied with amplification of the gene sequence, the protein expression levels were analyzed by Western blotting.

Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with a pore size of 0.45 µm (GE Healthcare) using the XCell SureLock Mini-Cell electrophoresis system. Subsequently, the blocking reaction was carried out using a Western blocking reagent (Roche) in accordance with the recommended protocol at 4°C overnight, then the reaction product was washed 4 times with PBS-T for 10 minutes and subjected to the subsequent antibody reaction. In the EGFP detection, the reaction was conducted using, as the first antibody 1, the 5,000-fold diluted (0.2 ng/µl) anti-EGFP mouse antibody (abcam: ab184601) for 1 hour at room temperature. After washed 3 times with PBS-T for 10 minutes, the subsequent reaction wasconducted using, as the second antibody, the 5,000-fold diluted (0.2 ng/µl) anti-mouse IgG goat antibody (abcam: ab6789) for 45 minutes. After washed 3 times with PBS-T for 10 minutes, the further reaction was conducted using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan).

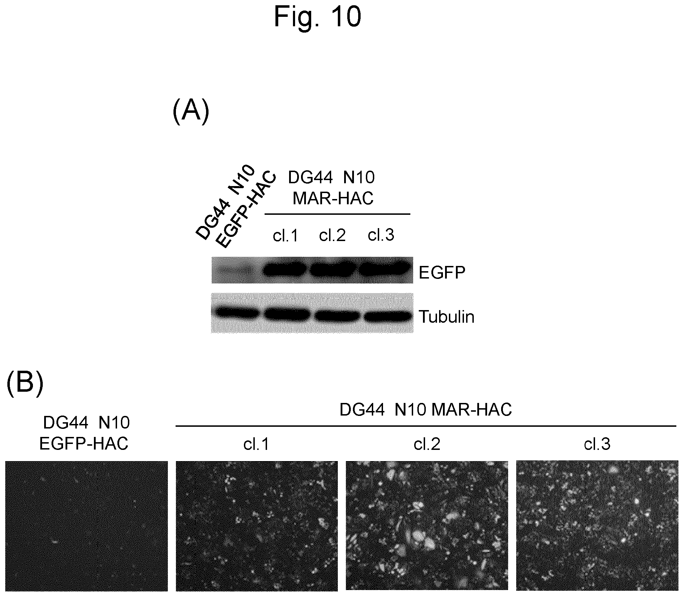

As the results of the Western blot analysis, higher expression levels were observed in the CHO DG44 N10 MAR-HAC clone, compared with the control (Fig. 10A). In addition, as the results of the fluorescent microscopic observation, higher fluorescence brightness was observed in the CHO DG44 N10 MAR-HAC clone compared with the control, as with the results of Western blotting (Fig. 10B). From these results, it was determined that the gene amplification could be achieved by integrating the IR/MAR sequence and the EGFP gene into HAC and that the EGFP protein expression level could increase due to gene amplification (Fig. 1, Step 3). This experimental system was designated as "the MAR-HAC gene amplification method."

### [Example 3] Comparison of gene expression levels between the CHO K1 cell and the CHO DG44 cell

HAC comprising a single copy of EGFP was introduced into the CHO K1 cells and the CHO DG44 cells, and then the expression levels of EGFP from the same genome information were compared, whereby the protein production efficiency was compared between the cells.

### [A] Integration of the EGFP gene into HAC in the CHO kkpqN10 cell derived from the CHO K1 cell

pCX-EGFP CMV loxP Km2 used in Example 1 is introduced into the CHO kkpqN10 cell, which is a CHO K1 cell carrying HAC for gene integration, so that a single copy of the EGFP gene is integrated into HAC via recombination using the Cre/loxP system.

### [A.1.1] Gene transfer and isolation of drug resistant clone

Gene transfer was carried out via lipofection. In accordance with the protocol of Lipofectamine 2000 (Invitrogen), 5 µg of the Cre-expression vector pBS185 CMV-Cre (available from Addgene; Plasmid No. #11916) and 10 µg of the pCX-EGFP CMV loxP Km2 vector were introduced into the CHO kkpqN10 cells carrying the HAC vector for gene integration that had reached 90% confluency in 10-cm dishes (clone name: CHO K1 N10 EGFP-HAC). Twenty four hours after transfection, the cells were inoculated into twenty 10-cm dishes each containing a selection medium comprising F12 medium supplemented with 10% FBS, Blasticidin S (Bsd) 8 µg/ml, and G418 800 µg/ml, followed by two-weeks culture, and, as a result, resistant colonies appeared, which contained many clones obtained by the single introduction, 42 clones of which were isolated, amplified, and then subjected to the subsequent analysis.

### [A.2] Selection of drug resistant clone

### [A.2.1] PCR analysis

In order to select recombinants as templates following extraction of the genomic DNAs of Bsd- and G418-resistant cell lines, PCR was carried out using the primers indicated below, and whether or not the EGFP gene have been inserted into the human artificial chromosome (HAC) vector in a site-directed manner was confirmed at 2 junction regions that appeared by recombination in the loxP region. The primer sequences are as follows.

### Neo junction region

CMV586: 5'-CGTAACAACTCCGCCCCATT-3' (SEQ ID NO: 5)
Neo817: 5'-GCAGCCGATTGTCTGTTGTG-3' (SEQ ID NO: 6)

### Another junction region

CMV188: 5'-GGGCGTACTTGGCATATGAT-3' (SEQ ID NO: 7)
CX-EGFP HAC June R1: 5'-ATCCGCTCACAATTCCACACA-3' (SEQ ID NO: 8)

PCR was carried out using the ProFlex PCR System (Applied Biosystems) as a thermal cycler and KOD-FX- (Toyobo Co., Ltd.) as Taq polymerase. Buffers and dNTPs (dATP, dCTP, dGTP, and dTTP) included in the kit were used under the recommended conditions. Temperature and cycle conditions were as follows: after thermal denaturation at 95°C for 10 minutes, a cycle of 94°C for 30 seconds, 55°C for 30 seconds, and 72°C for 1 minute and 30 seconds was conducted for 35 cycles. Among the 42 isolated CHO K1 EGFP-HAC clones, 8 clones were found to be positive by analysis. The 8 clones were subjected to the subsequent analysis.

### [A.2.2] Two-color FISH analysis

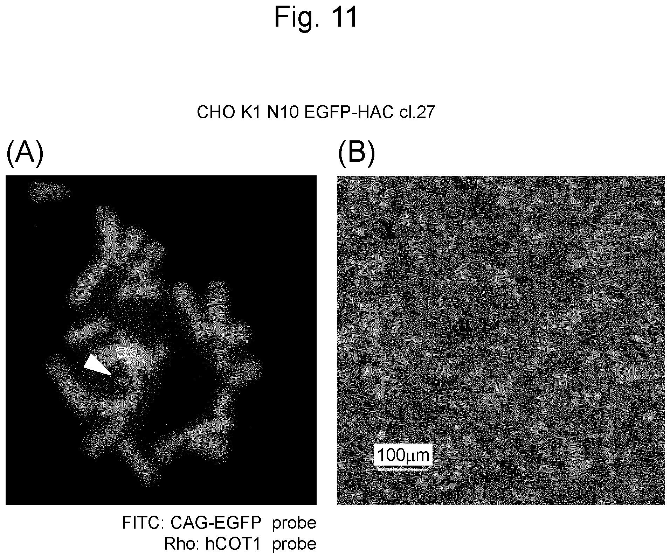

Among the 8 clones selected from the above results, 5 clones were subjected to two-color FISH analysis according to Matsubara et al. (FISH experimental protocols, Shujunsha Co., Ltd., 1994). FISH analysis was carried out using human cot-1 DNA and the pCX-EGFP plasmid as probes. As a result, a single clone (i.e., CHO K1 EGFP-HAC cl.27) was found to comprise the human artificial chromosome (HAC) at a retention rate of 100% and indicated EGFP-derived signals detected on HAC, thereby determing that the EGFP gene has been inserted into HAC in a site-directed manner (Fig. 11A). In addition, the CHO kkpqN10 pCX-EGFP HAC cl.27 cell emitted EGFP fluorescence at a high rate under the fluorescent microscope, correlating with the HAC retention rate (Fig. 11B). From the results, CHO K1 EGFP-HAC cl.27 was used in the subsequent experiments.

### [B] Transfer of EGFP-HAC from the CHO K1 N10 EGFP-HAC cl.27 cell to the CHO DG44 cell

### [B.1] Establishment of CHO DG44 EGFP-HAC

As the chromosomal donor cell, the CHO K1 N10 EGFP-HAC cl.27 cell was used. As the recipient cell, the CHO DG44 cell (described above) was used. The CHO K1 N10 EGFP-HAC cl.27 cells were inoculated into twelve 25-cm² centrifuge flasks (Nunc) and cultured in a medium (20% FBS, F12) containing colcemid (0.1 µg/ml, Gibco) for 72 hours to induce microcell formation. The centrifuge flasks were filled with a cytochalasin B (10 µg/ml in F12, Sigma) solution kept warm at 37°C in advance, the centrifuge flasks were inserted into an acrylic centrifuge, and centrifugation was then carried out at 37°C and 8,000 rpm (11,899 x g) for 1 hour (JLA-10.5 rotor, Beckman). The microcells were suspended in the serum-free DMEM medium, recovered, and filtered and purified using SWINNEX-25 (Millipore) equipped with 8-µm, 5-µm, and 3-µm filters (Whatman), successively. The purified microcells were resuspended in 2 ml of F12 containing 50 µg/ml Phytohemagglutinin-P (Difco). The resuspended microcells were added in an amount of 1 ml each in two 6-cm dishes in which the CHO DG44 cells have been cultured to reach 90% saturation, and then allowed to stand at 37°C for 15 minutes. PEG1000 (final concentration: 50% (w/v), Sigma) and DMSO (final concentration: 7% (w/v), Sigma) were dissolved in DMEM, the solution filtered through a 0.22-µm filter (Corning) was applied to the cells over a period of 1 minute, and the cells were washed with serum-free DMEM. The cells were cultured in an F12 medium containing 10% FBS for 24 hours, dispersed by trypsin treatment, and then inoculated into six 10-cm dishes. The cells were cultured in a selection medium (10% FBS, F12) containing blasticidin (4 µg/ml) for 2 weeks, and drug-resistant colonies were then obtained. Microcell fusion was carried out twice to isolate 14 resistant colonies, which were amplified and then subjected to the subsequent analysis (clone name: CHO DG44 EGFP-HAC).

### [B.2] Two-color FISH analysis

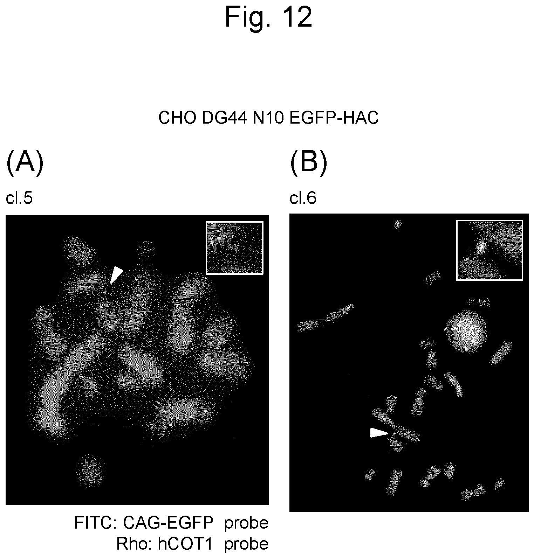

Among the 14 clones selected from the above results, 3 clones were subjected to two-color FISH analysis according to Matsubara et al. (FISH experimental protocols, Shujunsha Co., Ltd., Japan, 1994). FISH analysis was carried out using human cot-1 DNA and the pCX-EGFP plasmid as probes. As a result, 2 clones (i.e., CHO DG44 EGFP-HAC cl.5 and cl.6) were found to have karyotypic features of the CHO DG44 cell, retain the human artificial chromosome (HAC) at a retention rate of 90% or higher, and show EGFP-derived signals detected therein. It was thus confirmed that EGFP-HAC had been transferred from the CHO K1 N10 EGFP-HAC cl.27 cell to the CHO DG44 cell (Fig. 12).

### [C] Comparative analysis of the HAC-derived EGFP gene expression level between the CHO K1 cell and the CHO DG44 cell

As comparative cells, the CHO K1 N10 EGFP-HAC cl.27 cell derived from the CHO K1 cell line and the CHO DG44 EGFP-HAC cl.6 cell derived from the CHO DG44 cell were used. Since the HACs retained in the cells are of the same origin, it was considered possible to compare the EGFP gene expression levels in the intracellular environment.

### [C.1] Analysis of EGFP expression level by Western blotting

Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with 0.45-µm pore size (GE Healthcare) using the XCell SureLock Mini-Cell electrophoresis system. Subsequently, a blocking reaction was carried out using a Western blocking reagent (Roche) in accordance with the recommended protocol at 4°C overnight, the reaction product was washed 4 times with PBS-T for 10 minutes, and the resultant was subjected to the subsequent antibody reaction. EGFP detection was carried out by conducting a reaction using, as Antibody 1, the 5,000-fold diluted (0.2 ng/µl) anti-EGFP mouse antibody (abcam: ab184601) for 1 hour at room temperature. After the reaction product was washed 3 times with PBS-T for 10 minutes, the subsequent reaction was carried out using, as Antibody 2, the 5,000-fold diluted (0.2 ng/µl) anti-mouse IgG goat antibody (abcam: ab6789) for 45 minutes. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). EGFP protein quantification was carried out by measuring the band area using the Image J software and using the tubulin-corrected value.

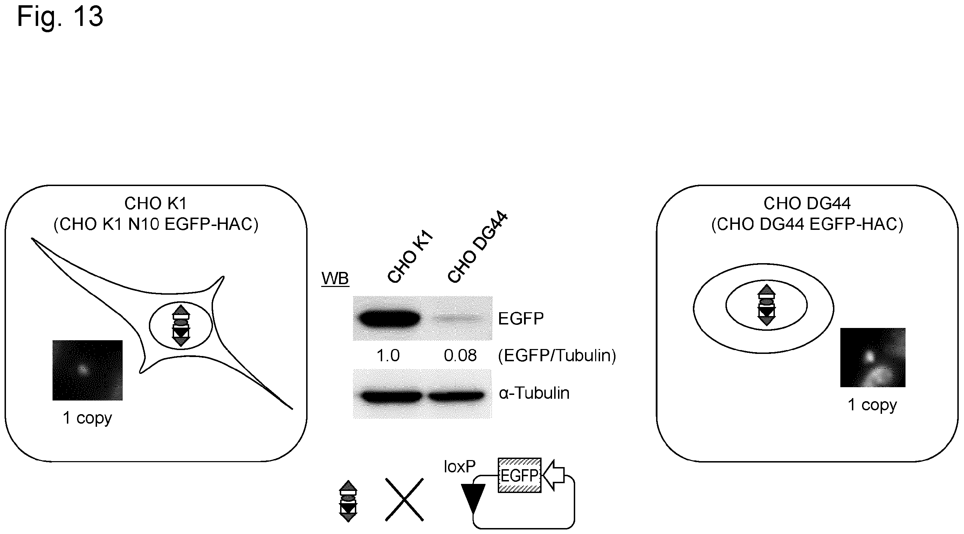

As a result of the Western blot analysis, the protein expression level of the CHO K1 N10 EGFP-HAC clone was found to be 12.5 times higher than that of the CHO DG44 EGFP-HAC clone (Fig. 13). From the results, it was determined that the CHO K1 cell was able to express the gene integrated into HAC more efficiently than the CHO DG44 cell.

### [Example 4] Transfer of the gene amplifying HAC prepared using the MAR-HAC system into the heterologous cell

The EGFP gene amplified on HAC using the MAR-HAC in Example 2 was transferred into the CHO K1 cells on the basis of the finding of Example 3. Whether or not such transfer would further enhance the EGFP gene expression level was examined (Fig. 14).

### [A] Establishment of the microcell-mediated chromosome transfer method using the CHO DG44 cell

Since there was no achievement in the microcell-mediated chromosome transfer technique using the CHO DG44 cell in the past, the ability of the CHO DG44 cell for microcell formation was examined herein.

### [A.1] Confirmation of the ability of the CHO DG44 cell for microcell formation

In order to confirm whether or not the CHO DG44 cell has the ability for microcell formation, colcemid treatment was carried out using the CHO DG44 N10 MAR-HAC cl.1 cell (described above). The CHO DG44 N10 MAR-HAC cl.1 cells were inoculated into 25-cm² centrifuge flasks (Nunc) and cultured in a medium (20% FBS, F12) containing colcemid (0.1 µg/ml, Gibco) for 72 hours to induce microcell formation. As a result, formation of many microcells was confirmed (Fig. 15).

From the results, the CHO DG44 cell was determined to have the ability for microcell formation, and the subsequent experiment for transfer of MAR-HAC into the CHO K1 cell via microcell-mediated chromosome transfer was carried out.

### [B] Transfer of MAR-HAC into the CHO K1 cell via microcell-mediated chromosome transfer

### [B.1] Transfer of MAR-HAC into the CHO K1 cell

As the chromosomal donor cell, the CHO DG44 N10 MAR-HAC cl.1 cell that carries HAC comprising the amplified EGFP gene integrated therein (described above) was used. As the recipient cell, the CHO kkpqN10 cell derived from the CHO K1 cell (described above) was used. The CHO DG44 N10 MAR-HAC cl.1 cells were inoculated into twelve 25-cm² centrifuge flasks (Nunc) and cultured in a medium (20% FBS, F12) containing colcemid (0.1 µg/ml, Gibco) for 72 hours to induce microcell formation. The centrifuge flasks were filled with a cytochalasin B (10 µg/ml in F12, Sigma) solution kept warm at 37°C in advance, the centrifuge flasks were inserted into an acrylic centrifuge, and centrifugation was then carried out at 37°C and 8,000 rpm (11,899 x g) for 1 hour (JLA-10.5 rotor, Beckman). The microcells were suspended in the serum-free DMEM medium, recovered, and filtered and purified using SWINNEX-25 (Millipore) equipped with 8-µm, 5-µm, and 3-µm filters (Whatman), successively. The purified microcells were resuspended in 2 ml of F12 containing 50 µg/ml Phytohemagglutinin-P (Difco). The resuspended microcells were added in an amount of 1 ml each in two 6-cm dishes in which the CHO kkpqN10 cells have been cultured to reach 90% saturation and then allowed to stand at 37°C for 15 minutes. PEG1000 (final concentration: 50% (w/v), Sigma) and DMSO (final concentration: 7% (w/v), Sigma) were dissolved in DMEM, the solution filtered through a 0.22-µm filter (Corning) was applied to the cells over a period of 1 minute, and the cells were washed with serum-free DMEM. The cells were cultured in an F12 medium containing 10% FBS for 24 hours, dispersed by trypsin treatment, and then inoculated into six 10-cm dishes. The cells were cultured in a selection medium (10% FBS, F12) containing G418 (800 µg/ml) for 1 week, and drug-resistant colonies were then obtained. Microcell fusion was carried out 2 times to isolate 24 resistant colonies, and the isolated colonies were amplified and then subjected to the subsequent analysis (clone name: CHO K1R N10 MAR-HAC).

### [B.2] Two-color FISH analysis

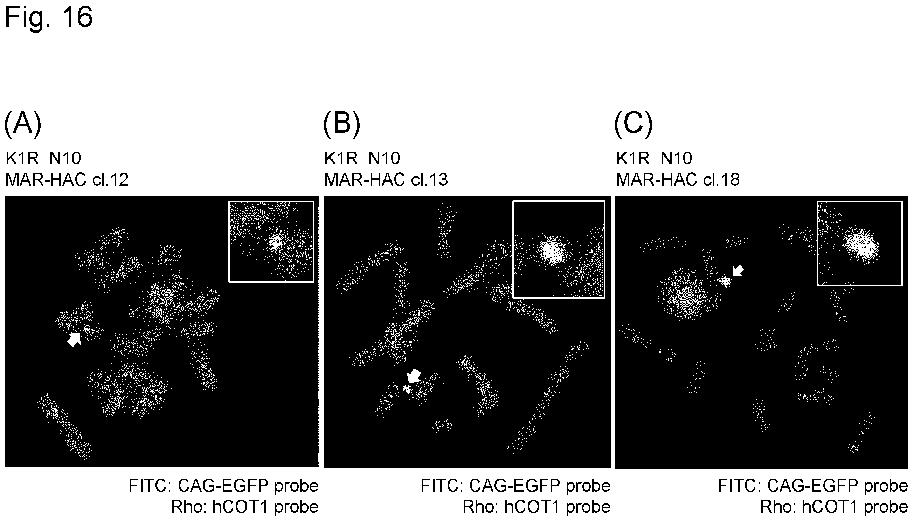

Among the 24 clones selected from the above results, 5 clones were subjected to two-color FISH analysis according to Matsubara et al. (FISH experimental protocols, Shujunsha Co., Ltd., Japan, 1994). FISH analysis was carried out using human cot-1 DNA and the pCX-EGFP plasmid as probes. As a result, all the clones were found to have karyotypic features of the CHO K1 cell and larger chromosomes stained with the human cot-1 probe, compared with common HAC. In addition, a plurality of EGFP-derived signals were detected thereon (Fig. 16).

From the results above, transfer of MAR-HAC from the CHO DG44 MAR-HAC cl.1 cell to the CHO kkpqN10 cell was confirmed.

### [C] Comparative analysis of EGFP protein expression level

In order to confirm whether or not the EGFP expression levels would be enhanced via transfer of MAR-HAC into the CHO K1 cell, the EGFP expression levels were compared between the CHO DG44 MAR-HAC cl.1 cell and the CHO K1R N10 MAR-HAC cell clone.

### [C.1] Analysis of EGFP expression level by Western blotting

Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with 0.45-µm pore size (GE Healthcare) using the XCell SureLock Mini-Cell electrophoresis system. Subsequently, a blocking reaction was carried out using a Western blocking reagent (Roche) in accordance with the recommended protocol at 4°C overnight, the reaction product was washed 4 times with PBS-T for 10 minutes, and the resultant was subjected to the subsequent antibody reaction. EGFP detection was carried out by conducting a reaction using, as Antibody 1, the 5,000-fold diluted (0.2 ng/µl) anti-EGFP mouse antibody (abcam: ab184601) for 1 hour at room temperature. After the reaction product was washed 3 times with PBS-T for 10 minutes, the subsequent reaction was carried out using, as Antibody 2, the 5,000-fold diluted (0.2 ng/µl) anti-mouse IgG goat antibody (abcam: ab6789) for 45 minutes. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). EGFP protein quantification was carried out by measuring the band area using the Image J software and using the tubulin-corrected value.

As a result of the Western blot analysis, the protein expression level of the CHO K1R N10 MAR-HAC cell clone was found to be 2.01 to 2.85 times higher than that of the donor CHO DG44 MAR-HAC cl.1 cell (Fig. 17). From the results, it was detrmined that the transfer of the HAC, which has comprised the EGFP gene amplified in the CHO DG44 cell using the MAR-HAC system and has been integrated therein, into the CHO K1 cell was able to further enhance the EGFP gene expression level.

### [C.2] Comparison of EGFP expression level between HAC comprising a single copy of the EGFP gene and MAR-HAC

In order to accurately examine the effects of gene amplification using the MAR-HAC system, EGFP expression levels were compared between HAC comprising a single copy of the EGFP gene and HAC comprising EGFP amplified using the MAR-HAC system by conducting experiments using,as a host, the CHO K1 cell with high protein expression efficiency.

### [C.2.1] Analysis of EGFP expression level by Western blotting

As the cell comprising the HAC in which a single copy of the EGFP gene was integrated, the CHO K1 N10 EGFP-HAC cl.27 cell (described above) was used. As the cell comprising HAC comprising EGFP amplified using the MAR-HAC system, the CHO K1R N10 MAR-HAC cell clone was used.

Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with a pore size of 0.45 µm (GE Healthcare) using the XCell SureLock Mini-Cell electrophoresis system. Subsequently, a blocking reaction was carried out using a Western blocking reagent (Roche) in accordance with the recommended protocol at 4°C overnight, the reaction product was washed 4 times with PBS-T for 10 minutes, and the resultant was subjected to the subsequent antibody reaction. EGFP detection was carried out by conducting a reaction using, as Antibody 1, the 5,000-fold diluted (0.2 ng/µl) anti-EGFP mouse antibody (abcam: ab184601) for 1 hour at room temperature. After the reaction product was washed 3 times with PBS-T for 10 minutes, the subsequent reaction was carried out using, as Antibody 2, the 5,000-fold diluted (0.2 ng/µl) anti-mouse IgG goat antibody (abcam: ab6789) for 45 minutes. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). EGFP protein quantification was carried out by measuring the band area using the Image J software and using the tubulin-corrected value.

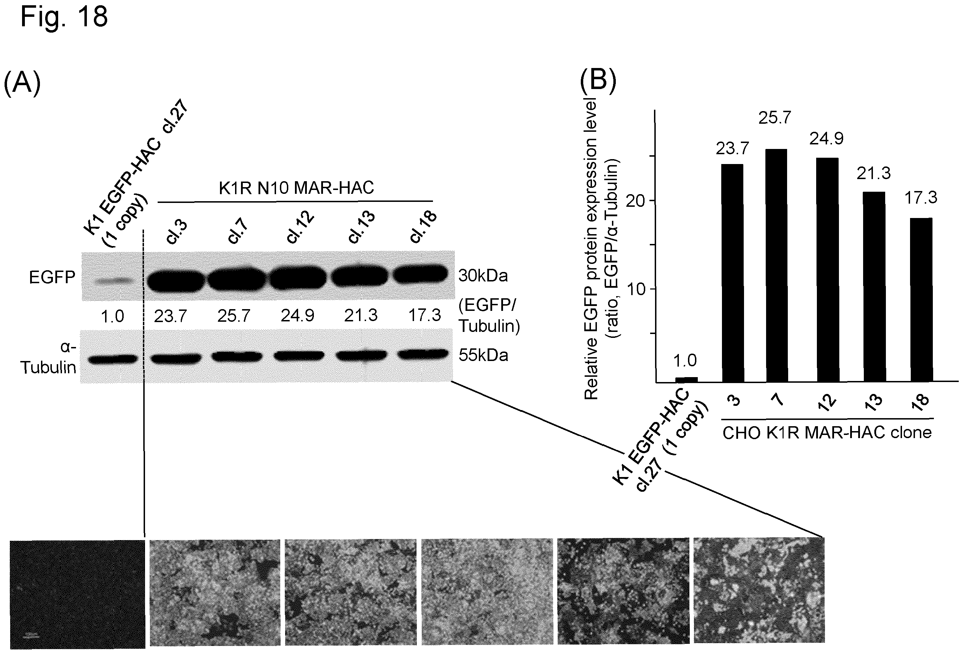

As a result of the Western blot analysis, the protein expression level of the CHO K1R N10 MAR-HAC cell clone was found to be 17.3 to 25.7 times higher than that of the CHO K1 N10 EGFP-HAC cl.27 cell (Fig. 18). From the results, it was determined that the expression level was able to increase by at most 25.7 times via amplification of the gene integrated into HAC using the MAR-HAC system.

### [Example 5] Evaluation of protein expression stability via long-term culture

In order to examine whether or not the amplified gene on HAC constructed using the MAR-HAC system can be stably expressed for a long period of time, long-term passage cultures were carried out using the CHO K1R N10 MAR-HAC cl.13 cell (described above), and the EGFP expression level of the primary culture and those of the passage cultures were compared. The culture periods were determined by measuring the population doubling levels (PDL), and the EGFP expression levels were compared and analyzed by Western blotting at the stages of PDL3, PDL30, and PDL50.

### [A] Analysis of EGFP expression level by Western blotting

Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with a pore size of 0.45 µm (GE Healthcare) using the XCell SureLock Mini-Cell electrophoresis system. Subsequently, a blocking reaction was carried out using a Western blocking reagent (Roche) in accordance with the recommended protocol at 4°C overnight, the reaction product was washed 4 times with PBS-T for 10 minutes, and the resultant was subjected to the subsequent antibody reaction. EGFP detection was carried out by conducting a reaction using, as Antibody 1, the 5,000-fold diluted (0.2 ng/µl) anti-EGFP mouse antibody (abcam: ab184601) for 1 hour at room temperature. After the reaction product was washed 3 times with PBS-T for 10 minutes, the subsequent reaction was carried out using, as Antibody 2, the 5,000-fold diluted (0.2 ng/µl) anti-mouse IgG goat antibody (abcam: ab6789) for 45 minutes. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). EGFP protein quantification was carried out by measuring the band area using the Image J software and using the tubulin-corrected value.

As a result of the Western blot analysis, the expression level at PDL30 was found to be as lower as 62% when compared with the expression level at PDL3, while the expression level was maintain at 80% in case of PDL50 (Fig. 19). From the results, it was determined that the gene amplified on HAC using the MAR-HAC system was able to maintain the expression level of 80% up to PDL50.

### [Example 6] Amplification and production of the anti-VEGF antibody gene using the MAR-HAC system

[A] The method for inserting an IR/MAR sequence and an anti-VEGF antibody gene into a HAC vector derived from human chromosome 21 was described. As described in Example 1, the CHO DG44 cell carrying the human chromosome 21-derived HAC vector into which the loxP site has been introduced was prepared (Fig. 20, Step 1). Separately, a plasmid comprising the loxP sequence, the IR/MAR sequence, and the anti-VEGF antibody gene expression unit was prepared, Cre recombinase was allowed to transiently express therein to cause site-directed recombination with the loxP sequence, and the plasmid was thus inserted into the human artificial chromosome (Fig. 20, Step 2). The inserted recombinant was selected using acquisition of G418 resistance as an indicatior (i.e., reconstitutionof a promoter-split type of neo gene expression unit) .

### [A.1] Construction of a plasmid vector that carries the anti-VEGF antibody gene

The enhancer sequence A7 having a function of enhancing gene expression was cloned, the heavy chain gene and the light chain gene of the anti-VEGF antibody were prepared as artificially synthesized genes, and the anti-VEGF antibody-producing vector comprising the A7 element was prepared. The method of preparation will be described below in detail.

### [A.1.1] Cloning of A7 element

In order to prepare for a subcloning vector, the inspB4ins2 vector (provided by Dr. Tetsuya Ohbayashi, Research Initiative Center, Organization for Research Initiation and Promotion, Tottori University, Japan) was digested with AvrII (NEB) and SpeI-HF (NEB), and the resultant was electrophoresed on 0.8% agarose gel, followed by purification with the MonoFas DNA purification kit (GL Sciences). The purified fragments were subjected to self-ligation using Ligation High ver.2 (Toyobo Co., Ltd.) to prepare the pB4ins1 subcloning vector. The inspB4ins2 vector is described in detail in Y. Yoshimura et al., Transgenic Research, 24. 4. pp. 717-727, 2015.

### [A.1.2] Cloning of A7 element via PCR

The A7 region is a 8,420-bp polynucleotide located in the position from88992123 to 89000542 of human chromosome 11 (Accession Number: AP003400.2). On the basis of the sequence information, the following primers were designed.
A7-PacI-41F: 5'-ATTAATTAATCTTAGTATGGTAAACCTTTTGAAGTAGATTC-3' (SEQ ID NO: 13)
A7-MluI-45R: 5'-GTAACGCGTCAAGTTTTTATTTTGTTCTCACAATTAAGTCTATAC-3' (SEQ ID NO: 14)

Using the primers prepared, PCR was carried out under the conditions described below using the genomic DNA of human IMR-90 cell (obtained from ATCC) as a template and KOD Fx DNA polymerase (Toyobo Co., Ltd.); i.e., after pre-denaturation at 95°C for 2 minutes, a cycle of 98°C for 10 seconds and 68°C for 10 minutes was conducted for 35 cycles. The PCR product was purified using the MonoFas DNA purification kit. The purified product was digested with MluI (NEB) and then inserted into a vector prepared by digesting pB4ins1 with NruI (NEB) and MluI using Ligation High ver.2 (Toyobo Co., Ltd.), resulting in production of pB4-A7.

### [A.1.3] Synthesis of the genes that produce the VEGF antibody

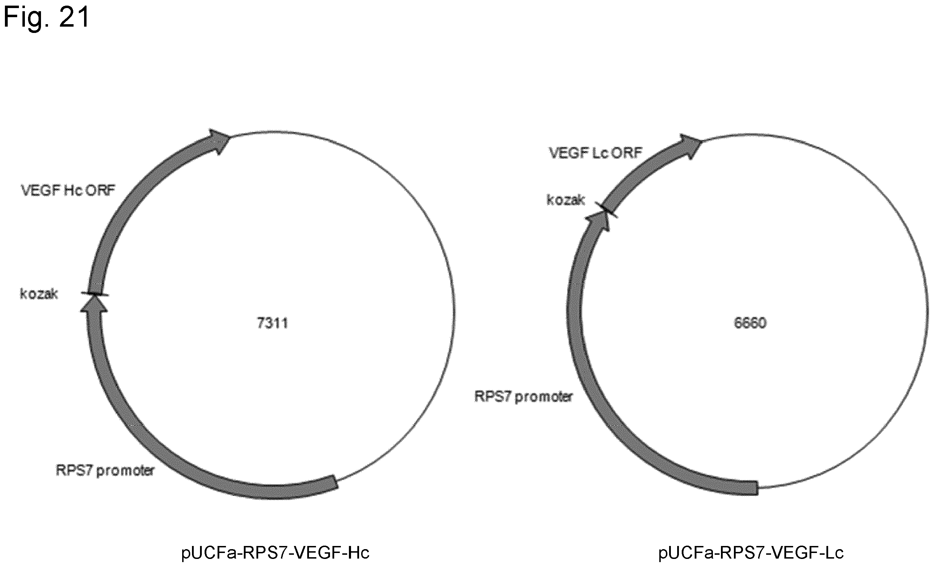

Concerning the anti-VEGF antibody gene, the amino acid sequences of the heavy chain gene and the light chain gene were obtained from the GenBank database (Accession Numbers: KX119517 and KX119516). In addition, as a promoter for gene expression, the promoter sequence of the human RPS7 gene was obtained based on the nucleotide sequence obtained from the GenBank database (Accession Number: NG_011744.1). On the basis of these sequence information, the preparation of synthetic genes was consigned to FASMAC, and the following plasmid vectors comprising the anti-VEGF antibody gene were prepared (Fig. 21).

Anti-VEGF heavy chain gene vector: pUCFa-RPS7-VEGF-Hc (SEQ ID NO: 15)

ATCAGATCGGAAGAGCACACGTCTGAACTCCAGTCTTCAATGGTATCGCCCAGAT
AGCTTGGCGTAATCATGGTCATGGTCATAGCTGTTTCCTGTGTGAAATTGTTATCC
GCTCACAATTCCACACAACATACGAGCCGGAAGCATAAAGTGTAAAGCCTGGGG
TGCCTAATGAGTGAGCTAACTCACATTAATTGCGTTGCGCTCACTGCCCGCTTTCC
AGTCGGGAAACCTGTCGTGCCAGCTGCATTAATGAATCGGCCAACGCGCGGGGA
GAGGCGGTTTGCGTATTGGGCGCTCTTCCGCTTCCTCGCTCACTGACTCGCTGCGC
TCGGTCGTTCGGCTGCGGCGAGCGGTATCAGCTCACTCAAAGGCGGTAATACGGT
TATCCACAGAATCAGGGGATAACGCAGGAAAGAACATGTGAGCAAAAGGCCAGC
AAAAGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAACCC
GACAGGACTATAAAGATACCAGGCGTTTCCCCCTGGAAGCTCCCTCGTGCGCTCT
CCTGTTCCGACCCTGCCGCTTACCGGATACCTGTCCGCCTTTCTCCCTTCGGGAAG
CGTGGCGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGCGCCTT
ATCCGGTAACTATCGTCTTGAGTCCAACCCGGTAAGACACGACTTATCGCCACTG
GCAGCAGCCACTGGTAACAGGATTAGCAGAGCGAGGTATGTAGGCGGTGCTACA
GAGTTCTTGAAGTGGTGGCCTAACTACGGCTACACTAGAAGAACAGTATTTGGTA
TCTGCGCTCTGCTGAAGCCAGTTACCTTCGGAAAAAGAGTTGGTAGCTCTTGATC
CGGCAAACAAACCACCGCTGGTAGCGGTGGTTTTTTTGTTTGCAAGCAGCAGATT
ACGCGCAGAAAAAAAGGATCTCAAGAAGATCCTTTGATCTTTTCTACGGGGTCTG
ACGCTCAGTGGAACGAAAACTCACGTTAAGGGATTTTGGTCATGAGATTATCAAA
AAGGATCTTCACCTAGATCCTTTTAAATTAAAAATGAAGTTTTAAATCAATCTAA
AGTATATATGAGTAAACTTGGTCTGACAGTTACCAATGCTTAATCAGTGAGGCAC
CTATCTCAGCGATCTGTCTATTTCGTTCATCCATAGTTGCCTGACTCCCCGTCGTG
TAGATAACTACGATACGGGAGGGCTTACCATCTGGCCCCAGTGCTGCAATGATAC
CGCGAGACCCACGCTCACCGGCTCCAGATTTATCAGCAATAAACCAGCCAGCCGG
AAGGGCCGAGCGCAGAAGTGGTCCTGCAACTTTATCCGCCTCCATCCAGTCTATT
AATTGTTGCCGGGAAGCTAGAGTAAGTAGTTCGCCAGTTAATAGTTTGCGCAACG

TTGTTGCCATTGCTACAGGCATCGTGGTGTCACGCTCGTCGTTTGGTATGGCTTCA
TTCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCGC
AGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATA
GTGTATGCGGCGACCGAGTTGCTCTTGCCCGGCGTCAATACGGGATAATACCGCG
CCACATAGCAGAACTTTAAAAGTGCTCATCATTGGAAAACGTTCTTCGGGGCGAA
AACTCTCAAGGATCTTACCGCTGTTGAGATCCAGTTCGATGTAACCCACTCGTGC
ACCCAACTGATCTTCAGCATCTTTTACTTTCACCAGCGTTTCTGGGTGAGCAAAAA
CAGGAAGGCAAAATGCCGCAAAAAAGGGAATAAGGGCGACACGGAAATGTTGA
ATACTCATACTCTTCCTTTTTCAATATTATTGAAGCATTTATCAGGGTTATTGTCTC
ATGAGCGGATACATATTTGAATGTATTTAGAAAAATAAACAAATAGGGGTTCCGC
GCACATTTCCCCGAAAAGTGCCACCTGACGTCTAAGAAACCATTATTATCATGAC
ATTAACCTATAAAAATAGGCGTATCACGAGGCCCTTTCGTCTCGCGCGTTTCGGT
GATGACGGTGAAAACCTCTGACACATGCAGCTCCCGGAGACGGTCACAGCTTGTC
TGTAAGCGGATGCCGGGAGCAGACAAGCCCGTCAGGGCGCGTCAGCGGGTGTTG
GCGGGTGTCGGGGCTGGCTTAATTAAATGCCCCAAAACATCAGGTTAATGGCGTT
CTTGATGTCATTCTCGCGATGCGACAGATCCGCAACCTCTTCGCCAATTACCGAC
ACTGGTACTGACGCCATATCCGTGGTCATCATACGCCAGCTTTCATCCCCAATGTG
GACAACCGGATAGAGTTCACGGAAACTTTGTCGCTCAGTAACCGTGCAGAAGC
CAACGGAATCACCGTGCGACGACCAGGAGTATCGATGATGTCGCTTTGCACATCC
ACAAACAGGCGATAGCGACTTTCGCGTTTGTAGGTGTAGACTTTGAACTGCGGTA
CGTTACAGAGAGCGCAACTGCTGTGACGGACGATCTGTACGCGCTTCTTCGCTAT
TCCGCCAACTCGCAAACGGAGGATGTGCTGCAAGGCGATTAAGTTGGGTAACGC
CTGGGTTTTCCCAGTCACGACGTTGTAAAACGACGGCCAGTGAATTGTAATAGCT
CTCTGAGTACGGAACACTCTTTCCCTTAACGACGCTCTTCCGATCTGATgaacgcgttcc
ttaaggacggatcgggagatctcccgatcccctatgtctcagtacaatctgctctgatgccgcatagttaagccagtatctgctccctgctt
gtgtgttggaggtcgctgagtagtgcgcgagcaaaatttaagctacaacaaggcaaggcttgaccgacaattgcatgaagaatctgctt
agggttaggcgttttgcgctgcttcgcgatgtacgggccagatattgacattgattattgactaggcttttgcaaaaagcttgtttaaacctt

gtatattaacagcacattaacagctaaaaagaaaaactcacataatcatattagttcatagaaacaatgcatttgacatactccaacatccat
tcatgatttatttatttttatttatatattttttgagatggagtctcgctgtcacccaggctggagtgcaatggctcgatctcggctcactgc
aggctccgcccccggcggttcacgccattctcctgcctcagcctcccgagtagctgggactacaggcgcccgccacctcgcccagct
aatttttgtatttttagtagagacggggtttcaccgtgttagccaggatggtctcgatctcctgacctcgtgatccacccgcctcggcctcc
caaagtgctgggattacaggcgtgagccaccgtgcccggcccatgattttaaaaaacctctcagaaatagaaacagagggaacttcc
tgaatttgattaaaaatacctgcaaaatcctagagctaatattatacttaatggtgaaagactgaatggttttcccctaagatggagaacaag
gcacggatgtccttgctcaccactcctattcaacaaaggactggaaaccagaaataactttaattttttttgttctcaagtgataggttcaca
gagaaaaagctttactggatgaacttttagattactacttttatagagcagcagagataaaagccaggtcgaaaagtgcatgtggagtaa
ggaaatggacctagttcgacaaaagggctcagaacgactgcccagatgagattgtagacgcagctgtagtttactttctatctggaaga
aacttcaagttatccttaattttccaaggagacagtcacttaccttttaaaaacattattagagaagcaccgggcgggagcatatctagca
ttaaaaatgtgggatgaataccatctctgcttggtaaaggtggttgggaatcctgagagagggcacctagtgtggcttctgcatttttcaca
gtgcctggaccacggctgaaagtaactcttgcatgacatttgacagaagaggaaaccgaagctcagattaaattccctgtccacagcgg
gatcattcggcacgagttcctccctgtctggaatgctcttccccagcaatagatcctgcggctcttccgtgtctcagtctaatgtcattccgt
tccaggattcccgacttcttaaagcataaataatccctccccaccctctcattgtactgttatgtaagttattacaatatgtcattatatatttagt
catactgctttaggtaatgtcttctccactgaactgtaagctccatgagggcaagagttcagtcggttttacttaataattagcacctagtaca
gtactagcatagaatgaaggcctcgcaatttttttaaatttatttttagacagggtcttgcgctgtcgcccaggctggagtgcagtggtgca
acctcggctcacggcagcctcgacctttcggctccagcgatcctcccgcgtcggcctccggggtagctgggactgcaggcgcgcac
caccatgactggctaattttttttttttttttttgtagacatggggtctcgccatgttgcccaggctggttcctgagctcaagtgatcctcctgc
ctcggcctcccaaagtgctgggattacaggcgtgagcctcagcgcccagccaagttagcctttttttaaacgtcctgtctccggaggttgc
cgaagttggttttcttcggcctccttctctctcccaggcccagggctgggacgaggccggttcccgcctgcaacctgcactgaagacgg
gaaccttgggagccggtaccggaacgctcggaaacggcaccaaagtacgaatcctagggcggaaaagcgttaccaagacactcgtc
cccagagccgcttcctgggactctctagcctcctaccgcttctcagtgatgttccggtttccgccctcctcctcgcgctgtttccgcctcttg
ccttcggacgccggattttgacgtgctctcgcgagatttgggtctcttcctaagccggcgctcggcaaggtaggttggcggcctgctctc
cgacagaacttttcttcttgggttgaggaaaacgccttttggagtcaggccctggaggggcgagccttgctcacagggtggggatacag
ccgattacccgccctgtgctttccgatggcttctgcggggcgagcggggcctggccggggggtgcgggcgggagggcgagccagc
ggcgcctgcagcccgggccgcgtaacgctgaccgctgtgccttcagttctcccaggagaaagccgctagccgccaccATGGA
GTTCGGGTTGTCATGGCTGTTCCTGGTCGCCATTCTGAAAGGAGTGCAGTGCGAG
GTTCAATTGGTGGAGTCTGGCGGAGGTCTCGTGCAGCCCGGAGGGAGCCTCCGCC
TGAGCTGCGCCGCTTCAGGGTACACATTCACAAACTACGGCATGAACTGGGTTCG

ACAGGCACCCGGCAAGGGACTTGAATGGGTGGGTTGGATCAACACATACACTGG
AGAGCCCACTTATGCTGCCGATTTTAAAAGACGGTTCACTTTTAGTCTCGACACAT
CCAAGTCCACTGCCTATCTTCAGATGAATAGCTTGCGCGCAGAGGACACAGCCGT
ATACTATTGCGCCAAGTATCCACATTATTACGGGAGTTCTCATTGGTATTTCGACG
TCTGGGGTCAGGGTACACTGGTAACTGTGAGTTCCGCCTCAACCAAGGGACCTTC
CGTATTTCCCCTCGCTCCAAGCTCTAAGTCCACTTCTGGAGGGACTGCTGCTCTCG
GATGTCTGGTAAAGGACTATTTCCTGAGCCAGTCACCGTCTCTTGGAACTCTGGC
GCCTTGACTTCTGGCGTTCATACATTTCCCGCCGTGCTGCAGAGTTCCGGATTGTA
TTCATTGTCTTCAGTCGTAACAGTGCCATCCTCATCATTGGGAACACAGACATATA
TCTGTAATGTCAACCATAAGCCTTCTAACACCAAGGTCGATAAGAAGGTTGAACC
TAAATCATGCGACAAGACACACACTTGTCCACCATGTCCTGCACCTGAACTTTTG
GGCGGACCTAGCGTATTTCTTTTCCCACCTAAGCCCAAAGATACCCTTATGATCTC
CCGTACACCCGAAGTTACCTGTGTGGTTGTGGATGTCAGCCATGAAGATCCTGAA
GTTAAGTTCAACTGGTATGTGGATGGTGTTGAAGTACACAACGCCAAAACCAAGC
CCAGGGAGGAGCAGTACAATTCAACTTACAGGGTTGTATCTGTCCTCACCGTACT
GCATCAGGATTGGCTCAACGGCAAGGAGTACAAGTGTAAAGTGAGCAACAAAGC
TCTTCCTGCTCCCATTGAAAAGACTATTAGTAAAGCTAAGGGTCAGCCTAGAGAA
CCCCAAGTGTACACTTTGCCTCCCAGTCGGGAAGAGATGACTAAGAACCAAGTAT
CCCTGACATGCCTTGTGAAGGATTTTATCCATCAGATATCGCAGTGGAATGGGA
GAGCAATGGCCAGCCCGAGAACAACTATAAAACTACTCCTCCTGTCCTGGATTCT
GATGGGTCCTTCTTCCTGTATTCCAAGTTGACAGTCGATAAAAGCCGTTGGCAGC
AGGGAAACGTGTTCAGTTGCAGTGTGATGCACGAGGCCTTGCACAATCACTACAC
ACAGAAAAGCCTGTCCCTTTCACCAGGGAAGTAGgcggccgctgcagggagatttcgattccaccgc
cgccttctatgaaaggttgggcttcggaatcgttttccgggacgccggctggatgatcctccagcgcggggatctcatgctggagttctt
cgcccaccccaacttgtttattgcagcttataatggttacaaataaagcaatagcatcacaaatttcacaaataaagcatttttttcactgcatt
ctagttgtggtttgtccaaactcatcaatgtatcttagtcggcgcgccgacggactcgagtcgtcgac

Anti-VEGF light chain gene vector: pUCFa-RPS7-VEGF-Lc (SEQ ID NO: 16)

ATCAGATCGGAAGAGCACACGTCTGAACTCCAGTCTTCAATGGTATCGCCCAGAT
AGCTTGGCGTAATCATGGTCATGGTCATAGCTGTTTCCTGTGTGAAATTGTTATCC
GCTCACAATTCCACACAACATACGAGCCGGAAGCATAAAGTGTAAAGCCTGGGG
TGCCTAATGAGTGAGCTAACTCACATTAATTGCGTTGCGCTCACTGCCCGCTTTCC
AGTCGGGAAACCTGTCGTGCCAGCTGCATTAATGAATCGGCCAACGCGCGGGGA
GAGGCGGTTTGCGTATTGGGCGCTCTTCCGCTTCCTCGCTCACTGACTCGCTGCGC
TCGGTCGTTCGGCTGCGGCGAGCGGTATCAGCTCACTCAAAGGCGGTAATACGGT
TATCCACAGAATCAGGGGATAACGCAGGAAAGAACATGTGAGCAAAAGGCCAGC
AAAAGGCCAGGAACCGTAAAAAGGCCGCGTTGCTGGCGTTTTTCCATAGGCTCCG
CCCCCCTGACGAGCATCACAAAAATCGACGCTCAAGTCAGAGGTGGCGAAACCC
GACAGGACTATAAAGATACCAGGCGTTTCCCCCTGGAAGCTCCCTCGTGCGCTCT
CCTGTTCCGACCCTGCCGCTTACCGGATACCTGTCCGCCTTTCTCCCTTCGGGAAG
CGTGGCGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGCGCCTT
ATCCGGTAACTATCGTCTTGAGTCCAACCCGGTAAGACACGACTTATCGCCACTG
GCAGCAGCCACTGGTAACAGGATTAGCAGAGCGAGGTATGTAGGCGGTGCTACA
GAGTTCTTGAAGTGGTGGCCTAACTACGGCTACACTAGAAGAACAGTATTTGGTA
TCTGCGCTCTGCTGAAGCCAGTTACCTTCGGAAAAAGAGTTGGTAGCTCTTGATC
CGGCAAACAAACCACCGCTGGTAGCGGTGGTTTTTTTGTTTGCAAGCAGCAGATT
ACGCGCAGAAAAAAAGGATCTCAAGAAGATCCTTTGATCTTTTCTACGGGGTCTG
ACGCTCAGTGGAACGAAAACTCACGTTAAGGGATTTTGGTCATGAGATTATCAAA
AAGGATCTTCACCTAGATCCTTTTAAATTAAAAATGAAGTTTTAAATCAATCTAA
AGTATATATGAGTAAACTTGGTCTGACAGTTACCAATGCTTAATCAGTGAGGCAC
CTATCTCAGCGATCTGTCTATTTCGTTCATCCATAGTTGCCTGACTCCCCGTCGTG
TAGATAACTACGATACGGGAGGGCTTACCATCTGGCCCCAGTGCTGCAATGATAC
CGCGAGACCCACGCTCACCGGCTCCAGATTTATCAGCAATAAACCAGCCAGCCGG
AAGGGCCGAGCGCAGAAGTGGTCCTGCAACTTTATCCGCCTCCATCCAGTCTATT
AATTGTTGCCGGGAAGCTAGAGTAAGTAGTTCGCCAGTTAATAGTTTGCGCAACG
TTGTTGCCATTGCTACAGGCATCGTGGTGTCACGCTCGTCGTTTGGTATGGCTTCA

TTCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCGC
AGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATA
GTGTATGCGGCGACCGAGTTGCTCTTGCCCGGCGTCAATACGGGATAATACCGCG
CCACATAGCAGAACTTTAAAAGTGCTCATCATTGGAAAACGTTCTTCGGGGCGAA
AACTCTCAAGGATCTTACCGCTGTTGAGATCCAGTTCGATGTAACCCACTCGTGC
ACCCAACTGATCTTCAGCATCTTTTACTTTCACCAGCGTTTCTGGGTGAGCAAAAA
CAGGAAGGCAAAATGCCGCAAAAAAGGGAATAAGGGCGACACGGAAATGTTGA
ATACTCATACTCTTCCTTTTTCAATATTATTGAAGCATTTATCAGGGTTATTGTCTC
ATGAGCGGATACATATTTGAATGTATTTAGAAAAATAAACAAATAGGGGTTCCGC
GCACATTTCCCCGAAAAGTGCCACCTGACGTCTAAGAAACCATTATTATCATGAC
ATTAACCTATAAAAATAGGCGTATCACGAGGCCCTTTCGTCTCGCGCGTTTCGGT
GATGACGGTGAAAACCTCTGACACATGCAGCTCCCGGAGACGGTCACAGCTTGTC
TGTAAGCGGATGCCGGGAGCAGACAAGCCCGTCAGGGCGCGTCAGCGGGTGTTG
GCGGGTGTCGGGGCTGGCTTAATTAAATGCCCCAAAACATCAGGTTAATGGCGTT
CTTGATGTCATTCTCGCGATGCGACAGATCCGCAACCTCTTCGCCAATTACCGAC
ACTGGTACTGACGCCATATCCGTGGTCATCATACGCCAGCTTTCATCCCCAATGTG
GACAACCGGATAGAGTTCACGGAAACTTTGTCGCTCAGTAACCGTGCAGAAGC
CAACGGAATCACCGTGCGACGACCAGGAGTATCGATGATGTCGCTTTGCACATCC
ACAAACAGGCGATAGCGACTTTCGCGTTTGTAGGTGTAGACTTTGAACTGCGGTA
CGTTACAGAGAGCGCAACTGCTGTGACGGACGATCTGTACGCGCTTCTTCGCTAT
TCCGCCAACTCGCAAACGGAGGATGTGCTGCAAGGCGATTAAGTTGGGTAACGC
CTGGGTTTTCCCAGTCACGACGTTGTAAAACGACGGCCAGTGAATTGTAATAGCT
CTCTGAGTACGGAACACTCTTTCCCTTAACGACGCTCTTCCGATCTGATggttaattaacc
gcgatcgcgcgcctgcaggactacgtagtcacgcgtgatccttaaggacggatcgggagatctcccgatcccctatgtctcagtacaat
ctgctctgatgccgcatagttaagccagtatctgctccctgcttgtgtgttggaggtcgctgagtagtgcgcgagcaaaatttaagctaca
acaaggcaaggcttgaccgacaattgcatgaagaatctgcttagggttaggcgttttgcgctgcttcgcgatgtacgggccagatattga
cattgattattgactaggcttttgcaaaaagcttgtttaaaccttgtatattaacagcacattaacagctaaaaagaaaaactcacataatcat

attagttcatagaaacaatgcatttgacatactccaacatccattcatgatttatttatttttatttatatatttttttgagatggagtctcgctgt
cacccaggctggagtgcaatggctcgatctcggctcactgcaggctccgcccccggcggttcacgccattctcctgcctcagcctccc
gagtagctgggactacaggcgcccgccacctcgcccagctaatttttgtatttttagtagagacggggtttcaccgtgttagccaggatg
gtctcgatctcctgacctcgtgatccacccgcctcggcctcccaaagtgctgggattacaggcgtgagccaccgtgcccggcccatga
ttttaaaaaaacctctcagaaatagaaacagagggaacttcctgaatttgattaaaaatacctgcaaaatcctagagctaatattatacttaa
tggtgaaagactgaatggttttcccctaagatggagaacaaggcacggatgtccttgctcaccactcctattcaacaaaggactggaaa
ccagaaataactttaatttttttgttctcaagtgataggttcacagagaaaaagctttactggatgaacttttagattactacttttatagagca
gcagagataaaagccaggtcgaaaagtgcatgtggagtaaggaaatggacctagttcgacaaaagggctcagaacgactgcccaga
tgagattgtagacgcagctgtagtttactttctatctggaagaaacttcaagttatccttaattttccaaggagacagtcacttaccttttaaaa
aacattattagagaagcaccgggcgggagcatatctagcattaaaaatgtgggatgaataccatctctgcttggtaaaggtggttgggaa
tcctgagagagggcacctagtgtggcttctgcatttttcacagtgcctggaccacggctgaaagtaactcttgcatgacatttgacagaag
aggaaaccgaagctcagattaaattccctgtccacagcgggatcattcggcacgagttcctccctgtctggaatgctcttccccagcaat
agatcctgcggctcttccgtgtctcagtctaatgtcattccgttccaggattcccgacttcttaaagcataaataatccctccccaccctctc
attgtactgttatgtaagttattacaatatgtcattatatatttagtcatactgctttaggtaatgtcttctccactgaactgtaagctccatgagg
gcaagagttcagtcggttttacttaataattagcacctagtacagtactagcatagaatgaaggcctcgcaatttttttaaatttatttttagac
agggtcttgcgctgtcgcccaggctggagtgcagtggtgcaacctcggctcacggcagcctcgacctttcggctccagcgatcctccc
gcgtcggcctccggggtagctgggactgcaggcgcgcaccaccatgactggctaattttttttttttttttttgtagacatggggtctcgcc
atgttgcccaggctggttcctgagctcaagtgatcctcctgcctcggcctcccaaagtgctgggattacaggcgtgagcctcagcgccc
agccaagttagcctttttaaacgtcctgtctccggaggttgccgaagttggttttcttcggcctccttctctctcccaggcccagggctgg
gacgaggccggttcccgcctgcaacctgcactgaagacgggaaccttgggagccggtaccggaacgctcggaaacggcaccaaa
gtacgaatcctagggcggaaaagcgttaccaagacactcgtccccagagccgcttcctgggactctctagcctcctaccgcttctcagt
gatgttccggtttccgccctcctcctcgcgctgtttccgcctcttgccttcggacgccggattttgacgtgctctcgcgagatttgggtctctt
cctaagccggcgctcggcaaggtaggttggcggcctgctctccgacagaacttttcttcttgggttgaggaaaacgccttttggagtcag
gccctggaggggcgagccttgctcacagggtggggatacagccgattacccgccctgtgctttccgatggcttctgcggggcgagcg
gggcctggccgggggggtgcgggcgggagggcgagccagcggcgcctgcagcccgggccgcgtaacgctgaccgctgtgccttc
agttctcccaggagaaagccgctagccgccaccATGAAGTACCTCCTTCCTACCGCTGCCGCTGGCC
TGCTGTTGCTTGCCGCTCAGCCTGCAATGGCCGACATTCAGATGACCCAGTCTCCC
AGCTCTCTCTCCGCTTCTGTTGGTGACAGAGTTACTATTACATGTAGCGCCTCTCA
AGACATCTCTAATTACCTGAATTGGTATCAACAAAAGCCTGGAAAGGCACCCAAG

GTGCTGATCTATTTCACAAGCTCACTGCATTCCGGCGTGCCCTCCAGATTTAGCGG
GTCAGGATCTGGAACCGACTTTACATTGACCATTTCCAGTCTGCAACCCGAAGAT
TTCGCTACCTACTACTGTCAGCAGTACAGTACCGTGCCCTGGACCTTCGGGCAGG
GCACCAAAGTCGAAATCAAGCGGACAGTCGCAGCCCCATCTGTATTCATATTTCC
ACCTTCAGACGAGCAGCTCAAGAGCGGCACAGCAAGCGTAGTTTGCCTGCTCAAT
AACTTTTATCCTCGCGAGGCCAAGGTCCAGTGGAAAGTGGATAACGCTCTCCAGT
CAGGAAACTCCCAGGAGAGCGTCACCGAGCAGGACTCAAAAGATAGCACATACT
CTTTGTCTAGTACCCTTACTCTTTCTAAAGCCGACTATGAGAAGCACAAGGTATAT
GCCTGCGAAGTGACTCACCAGGGGCTGTCCAGTCCCGTTACTAAGTCCTTTAACC
GAGGTGAATGCTAGgcggccgctgcagggagatttcgattccaccgccgccttctatgaaaggttgggcttcggaatcgt
tttccgggacgccggctggatgatcctccagcgcggggatctcatgctggagttcttcgcccaccccaacttgtttattgcagcttataat
ggttacaaataaagcaatagcatcacaaatttcacaaataaagcatttttttcactgcattctagttgtggtttgtccaaactcatcaatgtatc
ttatcatgtctgtataccgtcggcgcgccgacggactcgagtcgtcgac

### [A.1.4] Construction of the anti-VEGF antibody-producing vector comprising the A7 element

The synthesized light chain vector, pUCFa-RPS7-VEGF-Lc, was digested with the restriction enzymes AscI (NEB) and XhoI (NEB), and the synthesized heavy chain vector, pUCFa-RPS7-VEGF-Hc, was digested with the restriction enzymes MluI-HF (NEB) and XhoI, followed by agarose gel electrophoresis. The fragments of the intended sizes were purified using the MonoFas DNA purification kit, and the VEGF light chain and the VEGF heavy chain were ligated using Ligation High ver.2 (Toyobo Co., Ltd.) to prepare the pUC-Fa-RPS7-VEGF-LcHc vector.

pB4-A7 was digested with NaeI (NEB) and MluI, and pUC-Fa-RPS7-VEGF-LcHc was digested with MluI and SnaBI (NEB), followed by agarose gel electrophoresis. The fragments of the intended sizes were purified using the MonoFas DNA purification kit, and the fragments were ligated using Ligation High ver.2 (Toyobo Co., Ltd.) to prepare the pUC-Fa-A7-RPS7-VEGF-LcHc vector comprising the A7 element.

### [A.1.5] Introduction of multiple cloning sites into IR/MAR vector

In order to insert the antibody gene into the IR/MAR vector, additional restriction enzyme sites; i.e., PacI, AscI, FseI, and SacII, and a loxG sequence for recombination were inserted into the Pst1 site of pΔBN AR1-Dhfr to construct a vector. The method for the preparation will be described below.

The pCX-EGFP CMV loxG Km vector used in Example 2 was used as a template to clone a sequence comprising the loxP sequence and then to introduce a plurality of restriction enzyme sites via PCR. The primer sequences are shown below.
Pst1 loxG Km2 F: 5'-AACTGCAGGCCGGCCGCGGAGGCCTGGTGACCTAAGCTTGCATGC-3' (SEQ ID NO: 17)
Pst1 loxG Km2 R: 5'-ATTGGTTCTGCAGAGCTTGGCGCGCCAAGCTTAATTAAATGAGCCATATTCAACG GG-3' (SEQ ID NO: 18)

PCR was carried out using the ProFlex PCR System (Applied Biosystems) as a thermal cycler and KOD-FX- (Toyobo Co., Ltd.) as Taq polymerase. Buffers and dNTPs (dATP, dCTP, dGTP, and dTTP) included in the kit were used under the recommended conditions. Temperature and cycle conditions were as follows; i.e., after thermal denaturation at 94°C for 2 minutes, a cycle of 98°C for 10 seconds and 68°C for 3 minutes was conducted for 20 cycles. The PCR product was purified using the Wizard SV Gel and PCR Clean-Up System kit (Promega Corporation). Thereafter, the purified product was cleaved with the restriction enzyme PstI (NEB), electrophoresed on 1% agarose gel, cut out from the gel, and then purified using the Wizard SV Gel and PCR Clean-Up System kit (Promega Corporation). Subsequently, this PCR product was introduced at the Pst1 (NEB) site of the pΔBN AR1-Dhfr vector in the manner described below. The pΔBN AR1-Dhfr vector was cleaved with the restriction enzyme PstI (NEB), electrophoresed on 1% agarose gel, cut out from the gel, and then purified using the Wizard SV Gel and PCR Clean-Up System kit (Promega Corporation). In order to prevent self-ligation of the DNA fragment of the linearized pΔBN AR1-Dhfr vector, the ends of the DNA fragment were dephosphorylated using the rAPId Alkaline Phosphatase kit (Roche) in accordance with the recommended protocol. Subsequently, the DNA fragment comprising loxP derived from the pCX-EGFP CMV loxG Km vector was ligated to the dephosphorylated DNA fragment of the pΔBN AR1-Dhfr vector using Ligation high v2 (Toyobo Co., Ltd.) in accordance with the recommended protocol. The final form of the modified IR/MAR vector prepared by inserting additional restriction enzyme sites (PacI, AscI, FseI, and SacII) and the loxG sequence into pΔBN AR1-Dhfr was designated as the "pΔBN AR1-Dhfr loxG Km2 MSC vector" (Fig. 22).

### [A.1.6] Introduction of the anti-VEGF antibody gene into the IR/MAR vector

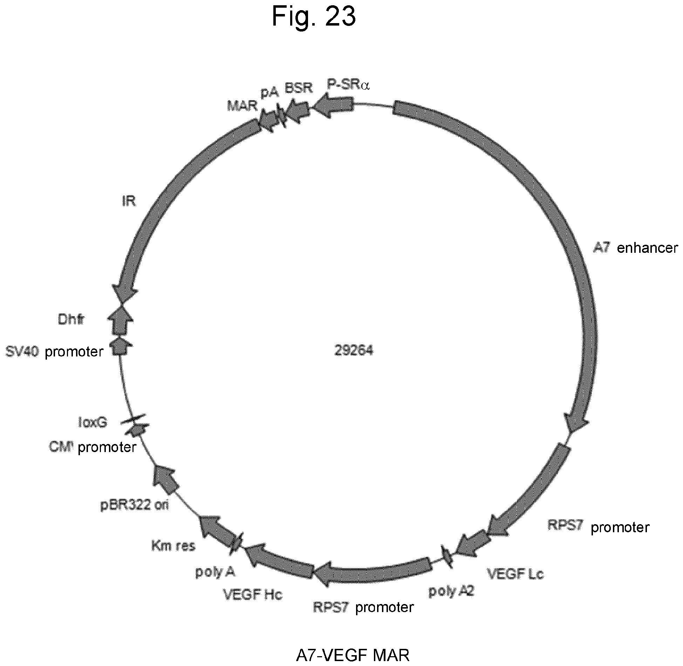

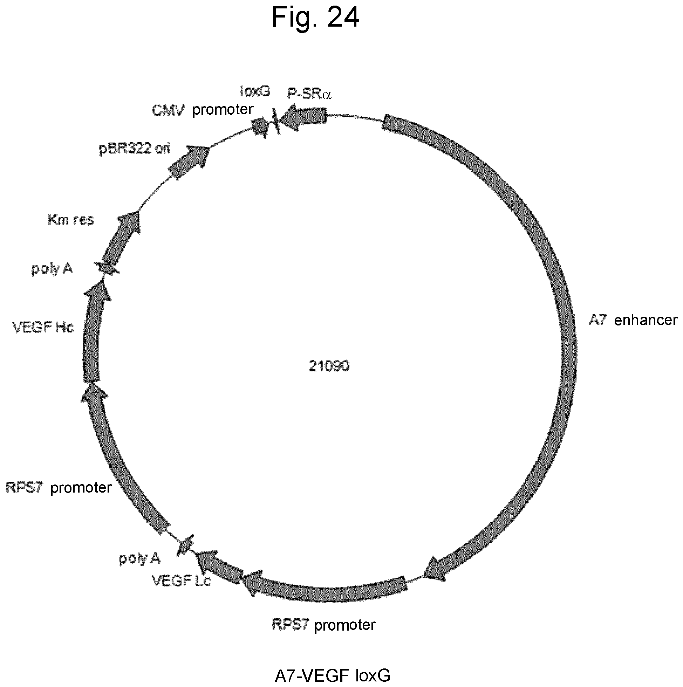

The IR/MAR vector (pΔBN AR1-Dhfr loxG Km2 MSC) was digested with AscI and dephosphorylated using Antarctic Phosphatase (NEB). The pUC-Fa-A7-RPS7-VEGF-LcHc was digested with AscI and separated via agarose gel electrophoresis. These DNA fragments were purified using the MonoFas DNA purification kit and ligated using Ligation High ver.2. The thus obtained anti-VEGF antibody-producing IR/MAR vector for artificial chromosome integration was designated as "A7-VEGF IR MAR" (Fig. 23). In addition, a control vector that lacks the IR/MAR region was prepared. A7-VEGF IR MAR was digested with HpaI (NEB) and FseI (NEB) and blunt-ended in accordance with the method of Blunting high (Toyobo Co., Ltd.). The blunt-ended plasmid DNAs were electrophoresed on agarose gel electrophoresis, the fragments of interest were purified using the MonoFas DNA purification kit, and the fragments were ligated using Ligation High ver.2. The control vector thus obtained was designated as "A7-VEGF loxG" (Fig. 24).

### [A.2] Integration of the anti-VEGF antibody gene vector into HAC using the Cre/loxP system

### [A.2.1] Gene transfer and isolation of drug resistant clone

Gene transfer was carried out via lipofection. In accordance with the protocol of Lipofectamine LTX (Invitrogen), 0.5 µg of the Cre-expression vector pBS185 CMV-Cre (available from Addgene; Plasmid No. #11916) and 1 µg of the A7-VEGF IR MAR vector were introduced into the CHO DG44 N10 cl.11 cells carrying the HAC vector for gene transfer in 6 wells, which cells have reached 90% confluency. Twenty four hours after transfection, the cells were inoculated into six 10-cm dishes each containing a selection medium comprising F12 medium supplemented with 10% FBS and G418 300 µg/ml, followed by 2-weeks culture, and, as a result, resistant colonies appeared, 18 clones of which were isolated, amplified, and then subjected to the subsequent analysis (clone name: CHO DG44 VEGF MAR-HAC).

In order to prepare a control HAC that does not comprise the IR/MAR sequence, the gene transfer into the CHO DG44 N10 cl.11 cells was carried out in the manner described above, using the A7-VEGF loxG vector. As a result, 22 clones were isolated, amplified, and then subjected to the experiment described below (clone name: CHO DG44 VEGF-HAC).

### [A.3] Selection of drug resistant clone

### [A.3.1] PCR analysis

In order to extract the genomic DNA of a G418-resistant cell line and to select recombinants as templates, PCR was carried out using the primers described below to comfirm whether or not the EGFP and the IR/MAR sequence have been inserted into the human artificial chromosome (HAC) vector in a site-directed manner, in two junction regions appearing by recombination at the loxP region. The primer sequences are shown below.,
Neo junction region
CMV586: 5'-CGTAACAACTCCGCCCCATT-3' (SEQ ID NO: 5)
Neo817: 5'-GCAGCCGATTGTCTGTTGTG-3' (SEQ ID NO: 6)
Another junction region
CMV188: 5'-GGGCGTACTTGGCATATGAT-3' (SEQ ID NO: 7)
MAR junction 5'-CAGTGCTGCAATGATACCGC-3' (SEQ ID NO: 19)

PCR was carried out using the ProFlex PCR System (Applied Biosystems) as a thermal cycler and KOD-FX- (Toyobo Co., Ltd.) as Taq polymerase. Buffers and dNTPs (dATP, dCTP, dGTP, and dTTP) included in the kit were used under the recommended conditions. Temperature and cycle conditions were as follows; i.e., after thermal denaturation at 95°C for 10 minutes, a cycle of 94°C for 30 seconds, 55°C for 30 seconds, and 72°C for 1 minute and 30 seconds was conducted for 35 cycles. Among the 18 isolated CHO DG44 VEGF MAR-HAC clones, 9 clones were analyzed and found to be positive. That is, the 9 clones were evaluated to comprise the anti-VEGF antibody gene and the IR/MAR sequence inserted into HAC, and they were then subjected to the subsequent analysis (Fig. 20, Step 2).

### [B4] Amplification of the anti-VEGF antibody gene caused by the IR/MAR sequence on the HAC vector

### [B.4.1] Confirmation of gene amplification via genome PCR analysis

In order to confirm that the anti-VEGF antibody gene was amplified due to the integration of the IR/MAR sequence into HAC, the genome was extracted from the CHO DG44 VEGF MAR-HAC clone, and comparative analysis was performed via real-time PCR using, as a control, the CHO DG44 VEGF-HAC clone comprising a single copy of the anti-VEGF antibody gene integrated therein. (Note: the PCR analysis was not intended to measure the absolute copy number of the anti-VEGF antibody genes but was intended to conduct the experiment to simply measure the gene amplification as compared with the control.)

PCR was carried out using the StepOne real-time PCR System (Applied Biosystems) as a thermal cycler, and Power SYBR Green PCR master Mix (ThermoFisher) as Taq polymerase and a buffer, under the recommended conditions.

The primer sequences used are shown below. The primers for the anti-VEGF antibody gene heavy chain (Hc) and light chain (Lc) and the NV1 primers for the internal genome standard were used (Nissom PM et al., Biologicals, 35, 211-5, 2007).
VEGF Hc qRT F: 5'-CTGCACCTGAACTTTTGGGC-3' (SEQ ID NO: 20)
VEGF Hc qRT R: 5'-TCGGGTGTACGGGAGATCAT-3' (SEQ ID NO: 21)
VEGF Lc qRT F: 5'-CAGTACAGTACCGTGCCCTG-3' (SEQ ID NO: 22)
VEGF Lc qRT R: 5'-AATACAGATGGGGCTGCGAC-3' (SEQ ID NO: 23)
NV1 CHO gPCR ref F: 5'-ACAGGTTTCTGCTTCTGGCA-3' (SEQ ID NO: 11)
NV1 CHO gPCR ref R: 5'-CATCAGCTGACTGGTTCACA-3' (SEQ ID NO: 12)

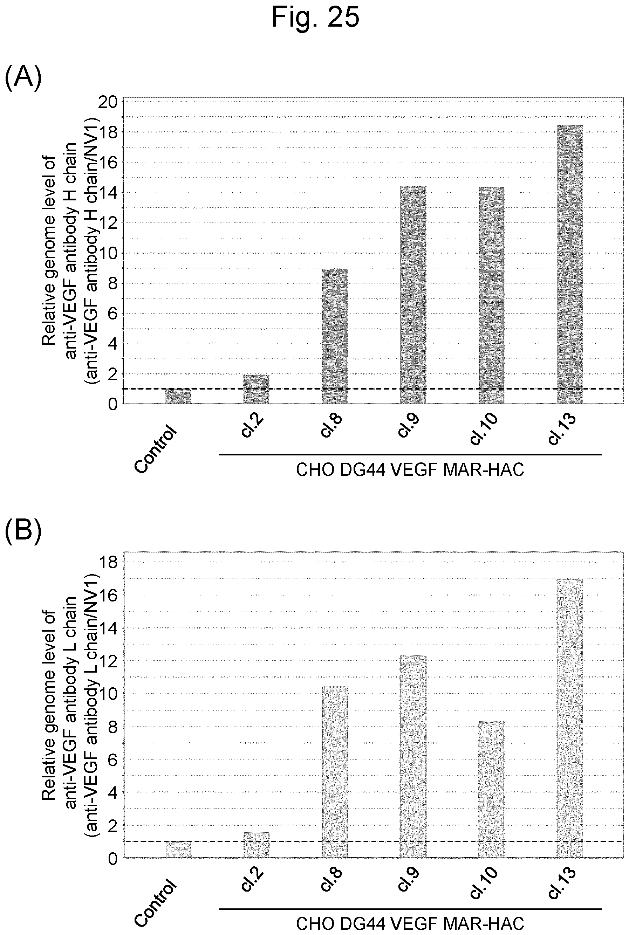

Among the 9 CHO DG44 VEGF MAR-HAC clones, 5 clones were analyzed by real-time PCR. As a result, 4 clones were found to comprise anti-VEGF antibody gene sequences more than the control cell comprising a single copy of the anti-VEGF antibody gene (CHO DG44 VEGF-HAC cl.7). In particular, the 4 clones comprising many anti-VEGF antibody gene sequences (i.e., cl.8, cl.9, cl.10, and cl.13) were subjected to the subsequent analysis (Fig. 25).

### [B.4.2] Two-color FISH analysis

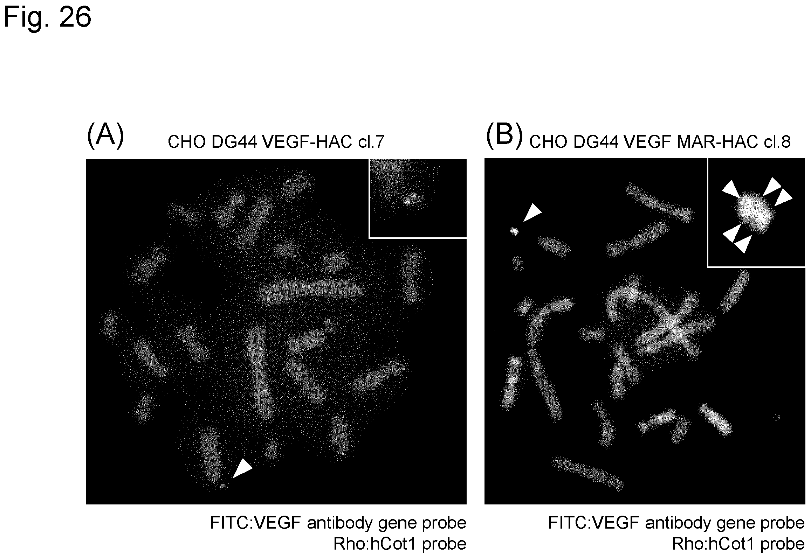

For the 4 clones selected from the above-mentioned results, two-color FISH analysis was conducted according to Matsubara et al. (FISH experimental protocols, Shujunsha Co., Ltd., Japan, 1994). The FISH analysis was carried out using human cot-1 DNA and the A7-VEGF-loxG plasmid vector as probes. As a result, a single clone (i.e., CHO DG44 VEGF MAR-HAC cl.8) was found to maintaine independently from the host chromosome in the cell, and the clone had larger chromosomes and a larger number of anti-VEGF antibody gene signals on human cot-1 signals, when compared with the control (CHO DG44 VEGF-HAC cl.7) (Fig. 26). On the basis of these results, it was determined that the anti-VEGF antibody gene was amplified on HAC, and the clone was used for the subsequent expression analysis. In addition, the HAC retention rate was found to be 90% or higher in both the clones indicated above.

### [B.5] Analysis of anti-VEGF antibody production level via antibody purification from culture supernatant

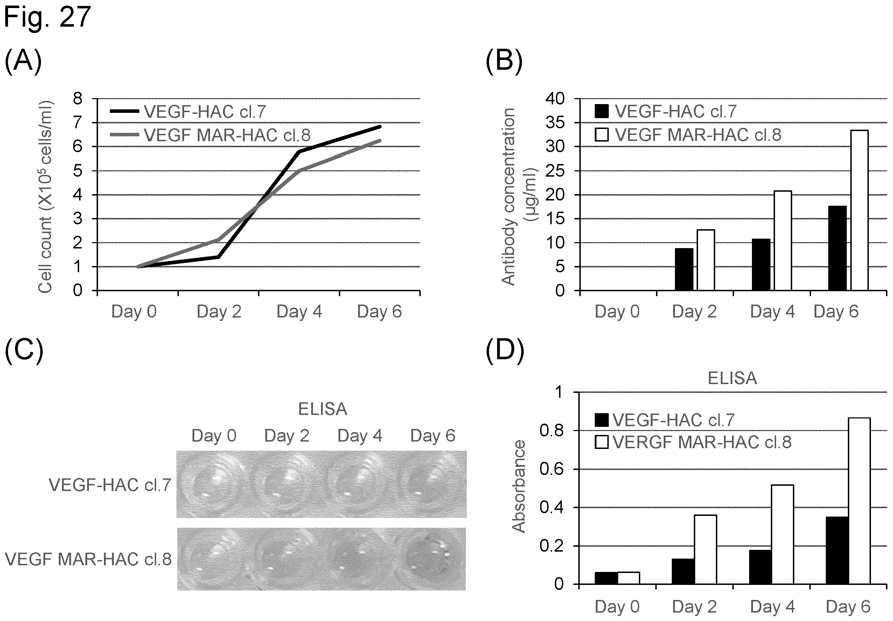

In order to examine whether or not the expression level of the anti-VEGF antibody of the CHO DG44 VEGF MAR-HAC clone was increased accompanied with gene sequence amplification, the antibodies produced from the culture supernatants were purified, and the concentrations thereof were measured and analyzed. Antibody purification was carried out using the spin-column-based IgG antibody purification kit (APK-10G, Cosmo Bio Co., Ltd., Japan) in accordance with the instructions included in the kit. Antibody concentration was measured using an absorption spectrometer (Scrum Inc.; Denovix). As a control, CHO DG44 VEGF-HAC cl.7 was used, and CHO DG44 VEGF MAR-HAC cl.8 was used as a cell that carries IR/MAR. The DG44 culture supernatant was obtained by seeding each of the cells in a 6-cm culture dish at cell density of 1 × 10⁵ cells/ml, 3 times every 2 days over a period of 6 days, followed by purifying the antibody from the culture supernatant and then measuring the concentration of the antibody. As a result, although the growth rate was found to be substantially the same between the cells (Fig. 27A), the level of antibody production of the MAR-HAC clone was found to be higher by approximately 2 times on the day 6, compared with the control (Fig. 27B).

### [B.6] Analysis of anti-VEGF antibody specificity via ELISA

In order to confirm whether or not the antibody produced from each of the DG44 cells could recognize the VEGF protein being a specific antigen, enzyme linked immunosorbent assay (ELISA) was carried out. ELISA was carried out by adding 100 µl each of the VEGF antigen protein (Sino Biological: 11086-HNAH) diluted at 50 ng/well with PBS to the 96-well plate onto which the protein can be immobilized (442404, Thermo Fisher), and allowing the plate to stand at room temperature for 1 hour to immobilize the antigen. Subsequently, the plate was washed 3 times with TBS-T (Wako), 350 µl of 5% skim milk/T-BST (Wako) was added as a blocking buffer, and the plate was then allowed to stand at room temperature for 1 hour. The plate was washed 3 times with TBS-T (Wako), 100 µl each of the DG44 cell culture supernatant was used as a primary antigen, and the plate was then allowed to stand at room temperature for 1 hour. Thereafter, the plate was washed 3 times with TBS-T (Wako), 100 µl each of the antibody reacting with the human antibody (goat anti-human IgG HRP, BETHYL, A80-119P) diluted to 1/50000-fold was added as a secondary antibody, and the plate was then allowed to stand at room temperature for 30 minutes. The plate was washed 3 times with TBS-T (Wako), OPD, an enzyme substrate, was added at 0.5 mg/ml to a substrate buffer (Thermo Fisher), a solution containing H₂O₂ (Wako) at an amount of 1/1000 was added in an amount of 1,000 µl each, and the reaction was allowed to proceed for 30 minutes. Thereafter, 1 M H₂SO₄ (Wako) was added to terminate the reaction, the wavelength at A492 nm was measured using an absorption plate reader (EPOCH2, Baio Tek), and the antibody reaction was then quantified.

As a result, it was confirmed that antibodies specifically reacting with the VEGF antigen were produced from the cells in correlation with the results of measurement of antibody concentration (Fig. 27B). In addition, the antigen-specific antibody binding reaction level of VEGF MAR-HAC was found to be 2 times or higher than that of the control VEGF-HAC (Figs. 27C and 27D). From these results, it was determined that the gene amplification was able to be achieved by integrating the IR/MAR sequence and the anti-VEGF antibody gene into HAC and, as a consequence, the anti-VEGF antibody production level could be enhanced due to the gene amplification (Fig. 20, Step 3).

### [Example 7] Establishment of CHO DG44 cell line carrying MAC vector derived from mouse chromosome 11

### [A] Introduction of HAC from CHO K1 cell comprising mouse chromosome 11-derived MAC vector into CHO DG44 cell

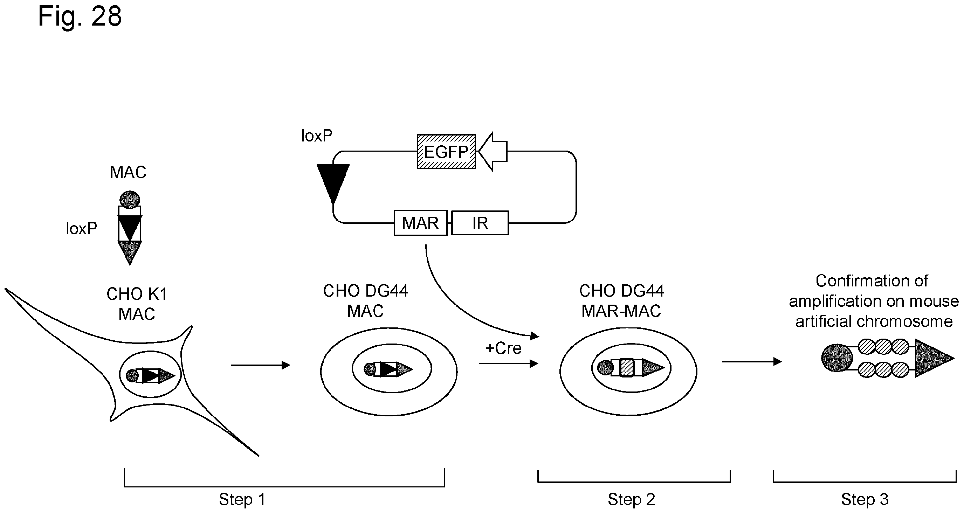

Application of the gene amplification system to the mouse artificial chromosome (MAC) in addition to the human artificial chromosome was examined. The MAC vector is described in detail in JP 2011-549044 A. In order to insert and amplify a gene of interest into/on the MAC in the CHO DG44 cell that exhibits high gene amplification efficiency achieved by the IR/MAR sequence, MAC comprising the DNA sequence insertion site (loxP) is introduced into the CHO DG44 cell (Fig. 28, Step 1).

### [A.1] Microcell fusion and isolation of drug resistant clone

As the chromosomal donor cell, the CHO K1 MAC cell line derived from the CHO K1 cell that carries the MAC vector derived from mouse chromosome 11 (hereafter, referred to as "CHO K1 MAC," JP 2011-549044 A) was used. As the recipient cell, the CHO DG44 cell (provided by Dr. L.A. Chasin, Columbia University) was used. The CHO K1 MAC cells were inoculated into twelve 25-cm² centrifuge flasks (Nunc) and cultured in a medium (20% FBS, F12) containing colcemid (0.1 µg/ml, Gibco) for 72 hours to induce microcell formation. The centrifuge flasks were filled with a cytochalasin B (10 µg/ml in F12, Sigma) solution kept warm at 37°C in advance, the centrifuge flasks were inserted into an acrylic centrifuge, and centrifugation was then carried out at 37°C and 8,000 rpm (11,899 x g) for 1 hour (JLA-10.5 rotor, Beckman). The microcells were suspended in the serum-free DMEM medium, recovered, and filtered and purified using SWINNEX-25 (Millipore) equipped with 8-µm, 5-µm, and 3-µm filters (Whatman), successively. The purified microcells were resuspended in 2 ml of F12 containing Phytohemagglutinin-P (Difco) at 50 µg/ml. The resuspended microcells were added in an amount of 1 ml each in two 6-cm dishes in which the CHO DG44 cells have been cultured to reach 90% saturation, and then were allowed to stand at 37°C for 15 minutes. PEG1000 (final concentration: 50% (w/v), Sigma) and DMSO (final concentration: 7% (w/v), Sigma) were dissolved in DMEM, the solution filtered through a 0.22-µm filter (Corning) was applied to the cells over a period of 1 minute, and the cells were washed with serum-free DMEM. The cells were cultured in an F12 medium containing 10% FBS for 24 hours, dispersed by trypsin treatment, and then inoculated into six 10-cm dishes. The cells were cultured in a selection medium (10% FBS, F12) containing blasticidin (4 µg/ml) for 2 weeks, and drug-resistant colonies were then obtained. Microcell fusion was carried out to isolate 4 resistant colonies, which were then amplified for the subsequent analysis (clone name: CHO DG44 MAC).

### [A.2] Selection of resistant clone

### [A.2.1] Mono-color FISH analysis

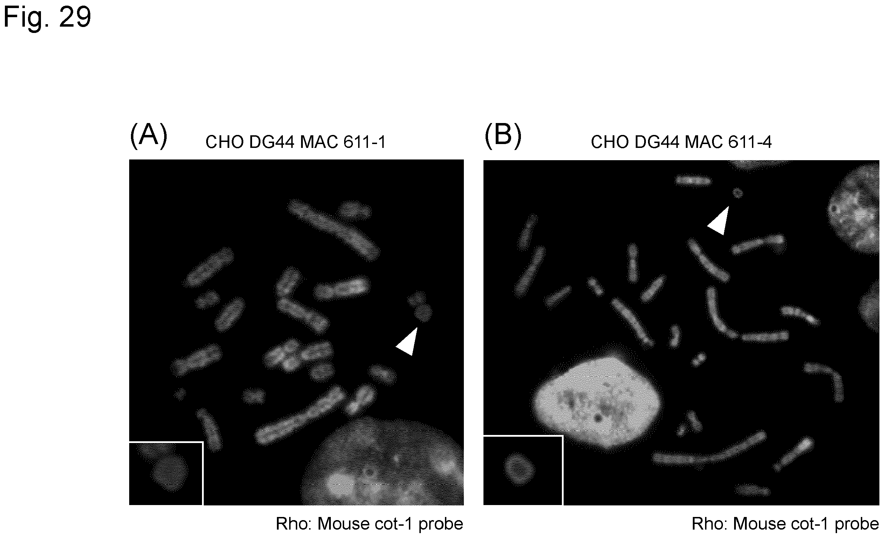

In accordance with the method described in the report by Shinohara et al. (Human Molecular Genetics, 10: 1163-1175, 2001), the 4 CHO DG44 MAC cell clones obtained above were subjected to FISH analysis using mouse Cot-1 DNA as a probe. It is possible to distinguish the CHO K1 cell-derived CHO K1 MAC from the CHO DG44 cell line based on the karyotypic features. While the CHO K1 cell line comprises 3 large chromosomes, the CHO DG44 cell line comprises 2 large chromosomes (Fig. 2 and Fig. 3). From the results of signal detection using a probe in addition to the karyotyping, it was confirmed that normal MAC has benn introduced into 2 clones of the CHO DG44 MAC cell (i.e., cl.611-1 and 611-4) with a retention rate of 30% or higher, which is slightly lower (Fig. 29).

From the above-mentioned results, it was concluded that the mouse artificial chromosome (MAC) vector comprising the DNA sequence insertion site was introduced into the CHO DG44 cell line.

### [Example 8] Introduction of the IR/MAR sequence and the EGFP gene into MAC

[A] The method for inserting the IR/MAR sequence and the EGFP gene into the MAC vector derived from mouse chromosome 11 was described. As described in Example 7, the CHO DG44 MAC cell that carries the mouse chromosome 11-derived MAC vector with introduced loxP site was prepared. Separately, the pCX-EGFP loxP dhfr plasmid vector, which comprises the loxP sequence, the IR/MAR sequence, and the EGFP gene expression unit, used in Example 2 was prepared, Cre recombinase was allowed to transiently express therein to cause site-directed recombination with the loxP sequence, and the plasmid vector was thus inserted into the mouse artificial chromosome. The recombinants were selected using, as an indicator, acquisition of G418 resistance (i.e., reconstitution of the promoter-split type of neo gene expression unit).

### [A.1] Integration of the pCX-EGFP loxP dhfr vector into MAC using the Cre/loxP system

### [A.1.1] Gene transfer and isolation of drug resistant clone

Gene transfer was carried out via lipofection. In accordance with the protocol of Lipofectamine LTX (Invitrogen), 0.5 µg of the Cre-expression vector pBS185 CMV-Cre (available from Addgene; Plasmid No. #11916) and 1 µg of the pCX-EGFP loxP dhfr vector were introduced into the CHO DG44 MAC cl.611-1 and cl.611-4 cells, which have reached 90% confluency, carrying the MAC vector for gene integration in 6 wells. Twenty four hours after transfection, the cells were inoculated into six 10-cm dishes each containing a selection medium that comprises a F12 medium supplemented with 10% FBS and 300 µg/ml G418, and then culture was conducted therein for 2 weeks, whereby resistant colonies appeared, which contained many clones obtained by the single introduction. Among them, 6 clones were isolated, amplified, and then subjected to the subsequent analysis (clone name: CHO DG44 MAR-MAC).

In order to prepare a control MAC that does not comprise the IR/MAR sequence, the gene transfer into the cl.611-1 and cl.611-4 cells was carried out in the same manner as described above, using the pCX-EGFR loxP Km2 vector instead of the pCX-EGFP loxP dhfr vector. As a result, 6 clones were isolated, amplified, and then subjected to the experiments described below (clone name: CHO DG44 EGFP-MAC).

### [A.2] Selection of drug resistant clone

### [A.2.1] PCR analysis

In order to select recombinants, the genomic DNA of the G418-resistant cell line was extracted, then PCR was carried out using the extracted genomic DNA, as templates, and the primers indicated below, and whether or not the EGFP and the IR/MAR sequence have been inserted into the mouse artificial chromosome (MAC) vector in a site-directed manner was confirmed at an junction region that appeared by recombination in the loxP region The primer sequences are as follows.

### Neo junction region

CMV586: 5'-CGTAACAACTCCGCCCCATT-3' (SEQ ID NO: 5)
Neo817: 5'-GCAGCCGATTGTCTGTTGTG-3' (SEQ ID NO: 6)

PCR was carried out using the ProFlex PCR System (Applied Biosystems) as a thermal cycler and KOD-FX- (Toyobo Co., Ltd.) as Taq polymerase. Buffers and dNTPs (dATP, dCTP, dGTP, and dTTP) included in the kit were used under the recommended conditions. Temperature and cycle conditions were as follows; i.e., after thermal denaturation at 95°C for 10 minutes, a cycle of 94°C for 30 seconds, 55°C for 30 seconds, and 72°C for 1 minute and 30 seconds was conducted for 35 cycles. The 6 isolated CHO DG44 MAR-MAC clones were analyzed and found to be positive. That is, the 6 clones were evaluated to comprise the EGFP and the IR/MAR sequence inserted into MAC, and were then subjected to the subsequent analysis (Fig. 28, Step 2).

### [B] Amplification of the EGFP gene by the IR/MAR sequence on the MAC vector

### [B.1.1] Confirmation of gene amplification via genome PCR analysis

In order to confirm that the EGFP gene was amplificed due to the integration of the IR/MAR sequence into MAC, the genome was extracted from the CHO DG44 MAR-MAC clone, and comparative analysis was performed by real-time PCR using, as a control, the CHO DG44 EGFP-MAC clone with integrated single copy of the EGFP gene. (Note: the PCR analysis was not intended to measure the absolute copy number of EGFP but was intended to conduct the experiments to simply determine whether or not the EGFP was amplified in comparison with the control.

PCR was carried out using the StepOne real-time PCR System (Applied Biosystems) as a thermal cycler and Power SYBR Green PCR master Mix (ThermoFisher) as Taq polymerase and a buffer, under the recommended conditions.

The primer sequences used are shown below. The EGFP primers and the NV1 primers as the genomic internal standards were prepared, respectively (Nissom PM et al., Biologicals, 35, 211-5, 2007).
EGFP gqPCR F: 5'-CTTCTTCAAGTCCGCCATGC-3' (SEQ ID NO: 9)
EGFP gqPCR R: 5'-GGTCTTGTAGTTGCCGTCGT-3' (SEQ ID NO: 10)
NV1 CHO gPCR ref F: 5'-ACAGGTTTCTGCTTCTGGCA-3' (SEQ ID NO: 11)
NV1 CHO gPCR ref R: 5'-CATCAGCTGACTGGTTCACA-3' (SEQ ID NO: 12)

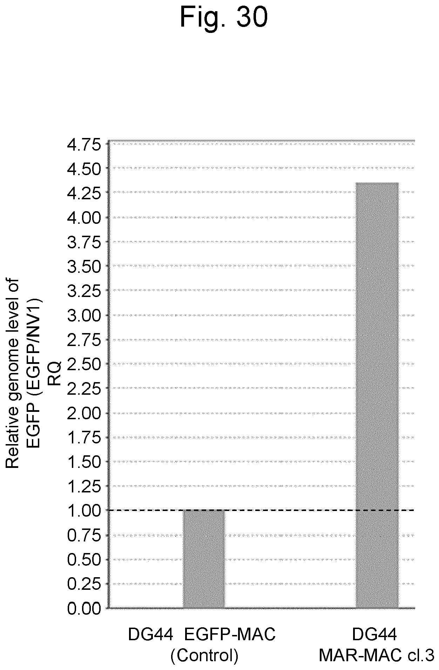

The 6 CHO DG44 MAR-MAC clones obtained were analyzed by real-time PCR. As a result, a single clone was found to comprise EGFP sequences more than the control CHO DG44 EGFP-MAC cl.12. This clone was subjected to the subsequent analysis (Fig. 30).

### [B.1.2] Two-color FISH analysis

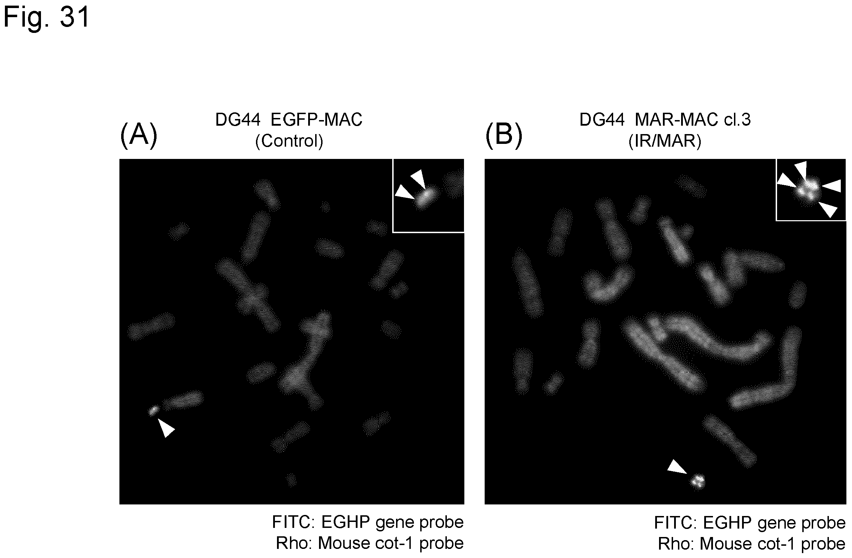

For 3 clones selected from the above-mentioned results, two-color FISH analysis was conducted according to Matsubara et al. (FISH experimental protocols, Shujunsha Co., Ltd., Japan, 1994). The FISH analysis was carried out using mouse cot-1 DNA and pCX-EGFP plasmid as probes. As a result, a single clone was found to maintaine the EGFP gene independently from the host chromosome ,and the clone had larger chromosomes and many EGFP signals on mouse cot-1 signals, when compared with the control (DG44 EGFP-MAC cl.12) (Fig. 31). From these results, it was determined that the EGFP gene was amplified on MAC, and the said clone was subjected to the subsequent expression analysis.

### [B.2] Analysis of EGFP expression level by Western blotting

In order to confirm whether or not the EGFP expression level of the CHO DG44 MAR-MAC clone was increasd accompanied with gene sequence amplification, the protein expression levels were analyzed by Western blotting.

The Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with a pore size of 0.45 µm (GE Healthcare) using the XCell SureLock Mini-Cell electrophoresis system. Subsequently, a blocking reaction was carried out using a Western blocking reagent (Roche) in accordance with the recommended protocol at 4°C overnight, the reaction product was washed 4 times with PBS-T for 10 minutes, and the resultant was subjected to the subsequent antibody reaction. EGFP detection was carried out by conducting a reaction using, as Antibody 1, the 5,000-fold diluted (0.2 ng/µl) anti-EGFP mouse antibody (abcam: ab184601) for 1 hour at room temperature. After the reaction product was washed 3 times with PBS-T for 10 minutes, the subsequent reaction was carried out using, as Antibody 2, the 5,000-fold diluted (0.2 ng/µl) anti-mouse IgG goat antibody (abcam: ab6789) for 45 minutes. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan).

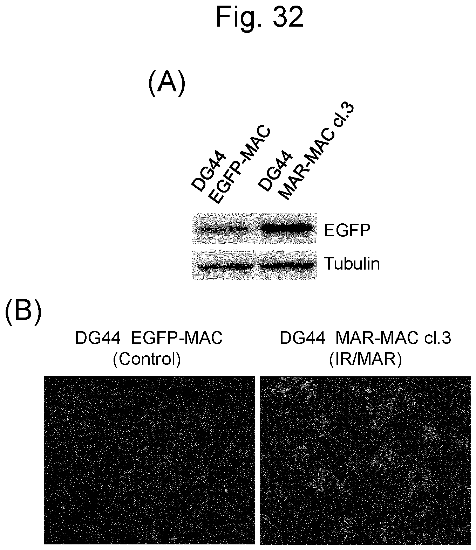

As a result of Western blot analysis, higher protein expression levels were observed in the CHO DG44 MAR-MA clones, when compared with the control (Fig. 32A). As a result of fluorescent microscopic observation, higher fluorescence brightness was observed in the CHO DG44 MAR-MAC clones compared with the control, as with the results of Western blotting (Fig. 32B). On the basis of these results, it was determined that gene amplification could have been achieved by integrating the IR/MAR sequence and the EGFP gene into MAC and, as a consequence, the EGFP protein expression levels could have been enhanced. This gene amplification technique was found to be applicable to, not only human-derived chromosomes but also to mouse-derived and mammalian-derived chromosomes (Fig. 28, Step 3).

### [Example 9] Establishment of the CHO DG44 cell line carrying the bottom-up type of artificial chromosome (tet-O HAC)

### [A] Introduction of HAC from the CHO K1 cell comprising the bottom-up type of artificial chromosome (tet-O HAC) vector into the CHO DG44 cell

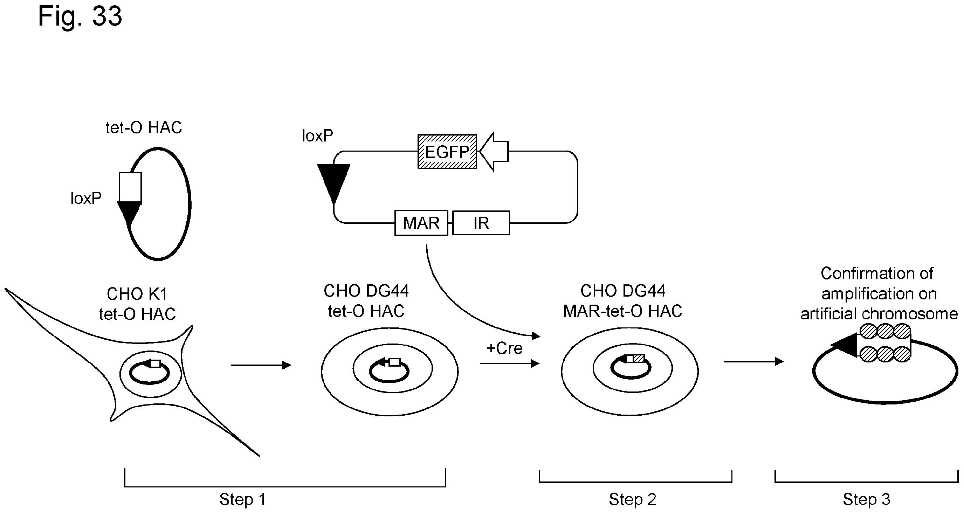

In this Example, whether or not the gene amplification system is applicable to the bottom-up type of an artificial chromosome in addition to the top-down type pf an artificial chromosome was examined. The bottom-up type of artificial chromosome tet-O HAC vector is described in detail in Y. Iida et al., ACS Synth. Biol., 21. 3. pp. 83-90, 2014. In order to insert and amplify a gene of interest into/on the HAC in the CHO DG44 cell that exhibits high gene amplification efficiency achieved by the IR/MAR sequence, the tet-O HAC comprising the DNA sequence insertion site (loxP) is introduced into the CHO DG44 cell (Fig. 33, Step 1).

### [A.1] Microcell fusion and isolation of drug resistant clone

As the chromosomal donor cell, the CHO K1 tet-O HAC cell line derived from the CHO K1 cell that carries the tet-O HAC vector was used. As the recipient cell, the CHO DG44 cell (provided by Dr. L.A. Chasin, Columbia University) was used. The CHO kkpqN10 cells were inoculated into twelve 25-cm² centrifuge flasks (Nunc) and cultured in a medium (20% FBS, F12) containing colcemid (0.1 µg/ml, Gibco) for 72 hours to induce microcell formation. The centrifuge flasks were filled with a cytochalasin B (10 µg/ml in F12, Sigma) solution kept warm at 37°C in advance, the centrifuge flasks were inserted into an acrylic centrifuge, and centrifugation was then carried out at 37°C and 8,000 rpm (11,899 x g) for 1 hour (JLA-10.5 rotor, Beckman). The microcells were suspended in the serum-free DMEM medium, recovered, and filtered and purified using SWINNEX-25 (Millipore) equipped with 8-µm, 5-µm, and 3-µm filters (Whatman), successively. The purified microcells were resuspended in 2 ml of F12 containing Phytohemagglutinin-P (Difco) at 50 µg/ml. The resuspended microcells were added in an amount of 1 ml each in two 6-cm dishes in which the CHO DG44 cells have been cultured to reach 90% saturation, and then were allowed to stand at 37°C for 15 minutes. PEG1000 (final concentration: 50% (w/v), Sigma) and DMSO (final concentration: 7% (w/v), Sigma) were dissolved in DMEM, the solution filtered through a 0.22-µm filter (Corning) was applied to the cells over a period of 1 minute, and the cells were washed with serum-free DMEM. The cells were cultured in an F12 medium containing 10% FBS for 24 hours, dispersed by trypsin treatment, and then inoculated into six 10-cm dishes. The cells were cultured in a selection medium (10% FBS, F12) containing blasticidin (4 µg/ml) for 2 weeks, and drug-resistant colonies were then obtained. Since many resistant colonies were obtained as a result of microcell fusion, 12 clones were isolated, amplified, and then subjected to the subsequent analysis (clone name: CHO DG44 tet-O HAC).

### [A.2] Selection of resistant clone

### [A.2.1] Mono-color FISH analysis

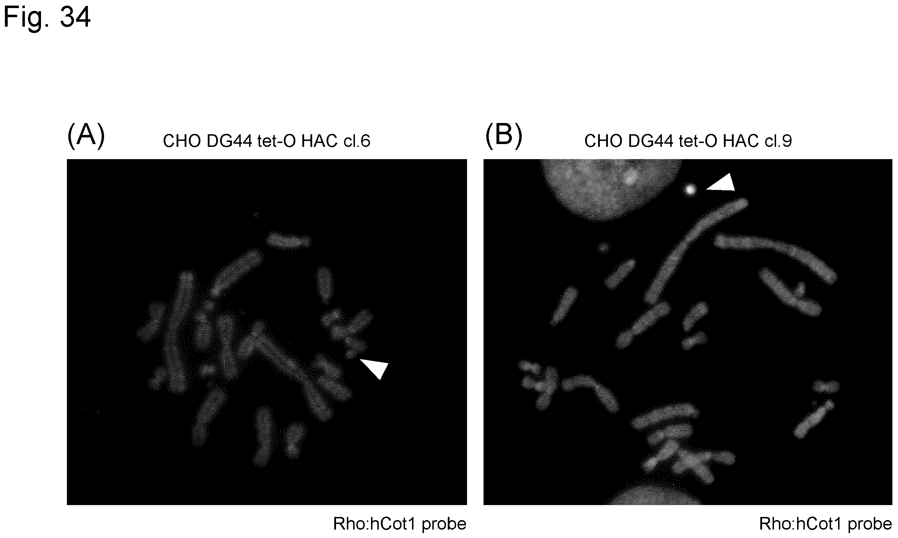

In accordance with the method described in the report by Shinohara et al. (Human Molecular Genetics, 10: 1163-1175, 2001), 9 clones selected from among the 12 CHO DG44 tet-O HAC cell clones obtained above were subjected to FISH analysis using human Cot-1 DNA as a probe. It is possible to distinguish the CHO K1 cell-derived CHO K1 tet-O HAC from the CHO DG44 cell line based on the karyotypic features. While the CHO K1 cell line comprises 3 large chromosomes, the CHO DG44 cell line comprises 2 large chromosomes (Fig. 2 and Fig. 3). As a result of signal detection using a probe in addition to the karyotyping, the introduction of normal tet-O HAC into two CHO DG44 tet-O HAC cell clones (cl.6 and cl.9) with a retention rate of 90% or higher was confirmed (Fig. 34).

From the above-mentioned results, it was concluded that the bottom-up type of artificial chromosome tet-O HAC vector, which comprises the DNA sequence insertion site, have been introduced into the CHO DG44 cell line.

### [Example 10] Introduction of the IR/MAR sequence and the EGFP gene into the tet-O HAC vector

[A] The method for inserting the IR/MAR sequence and the EGFP gene into the tet-O HAC vector was described. As described in Example 9, the CHO DG44 tet-O HAC cells carrying the tet-O HAC vector into which a loxP site was introduced, were prepared (cl.6 and cl.9). Separately, the pCX-EGFP loxP dhfr plasmid vector used in Example 2, which comprises the loxP sequence, the IR/MAR sequence, and the EGFP gene expression unit, was prepared, Cre recombinase was allowed to transiently express therein to cause site-directed recombination with the loxP sequence, and the plasmid vector was thus inserted into the tet-O HAC. The recombinants were selected using, as an indicator, acquisition of G418 resistance (i.e., reconstitution of the promoter-split type of neo gene expression unit).

### [A.1] Integration of the pCX-EGFP loxP dhfr vector into tet-O HAC using the Cre/loxP system

### [A.1.1] Gene transfer and isolation of drug resistant clone

Gene transfer was carried out by lipofection. In accordance with the protocol of Lipofectamine LTX (Invitrogen), 0.5 µg of the Cre-expressing pBS185 CMV-Cre vector (available from Addgene; Plasmid No. #11916) and 1 µg of the pCX-EGFP loxP dhfr vector were introduced into the CHO DG44 tet-O HAC cl.6 and cl.9 cells, in 6 wells, that have reached 90% confluency. Twenty four hours after transfection, the cells were inoculated into six 10-cm dishes each containing a selection medium that comprises an F12 medium supplemented with 10% FBS and 300 µg/ml G418, and then the culture was conducted therein for 2 weeks, whereby resistant colonies appeared, which contained many clones obtained by the single introduction. Among them, 16 clones were isolated, amplified, and then subjected to the subsequent analysis (clone name: CHO DG44 MAR-tet-O HAC).

### [A.2] Selection of drug resistant clone

### [A.2.1] PCR analysis

In order to select recombinants, the genomic DNA of the G418-resistant cell line was extracted, then PCR was carried out using the extracted genomic DNA, as templates, and the primers indicated below, and whether or not the EGFP and the IR/MAR sequence have been inserted into the mouse artificial chromosome (MAC) vector in a site-directed manner was confirmed at an junction region that appeared by recombination in the loxP region The primer sequences are as follows.

### Neo junction region

CMV586: 5'-CGTAACAACTCCGCCCCATT-3' (SEQ ID NO: 5)
Neo817: 5'-GCAGCCGATTGTCTGTTGTG-3' (SEQ ID NO: 6)

PCR was carried out using the ProFlex PCR System (Applied Biosystems) as a thermal cycler and KOD-FX- (Toyobo Co., Ltd.) as Taq polymerase. Buffers and dNTPs (dATP, dCTP, dGTP, and dTTP) included in the kit were used under the recommended conditions. Temperature and cycle conditions were as follows; i.e., after thermal denaturation at 95°C for 10 minutes, a cycle of 94°C for 30 seconds, 55°C for 30 seconds, and 72°C for 1 minute and 30 seconds was conducted for 35 cycles. The 12 isolated CHO DG44 tet-O HAC clones were analyzed and found to be positive. That is, the 12 clones were evaluated to comprise the EGFP and the IR/MAR sequence inserted into tet-O HAC, and were then subjected to the subsequent analysis (Fig. 33, Step 2).

### [B] Amplification of the EGFP gene by the IR/MAR sequence on the tet-O HAC vector

### [B.1.1] Confirmation of gene amplification via genome PCR analysis

In order to confirm that the EGFP gene was amplificed due to the integration of the IR/MAR sequence into MAC, the genome was extracted from the CHO DG44 tet-O HAC clone, and comparative analysis was performed by real-time PCR using, as a control, the CHO DG44 N10 EGFP-HAC clone with integrated single copy of the EGFP gene. (Note: the PCR analysis was not intended to measure the absolute copy number of EGFP but was intended to conduct the experiments to simply determine whether or not the EGFP was amplified in comparison with the control.)

PCR was carried out using the StepOne real-time PCR System (Applied Biosystems) as a thermal cycler and Power SYBR Green PCR master Mix (ThermoFisher) as Taq polymerase and a buffer, under the recommended conditions.

The primer sequences used are indicated below. The EGFP primers and the NV1 primers as the genomic internal standards were prepared, respectively (Nissom PM et al., Biologicals, 35, 211-5, 2007).
EGFP gqPCR F: 5'-CTTCTTCAAGTCCGCCATGC-3' (SEQ ID NO: 9)
EGFP gqPCR R: 5'-GGTCTTGTAGTTGCCGTCGT-3' (SEQ ID NO: 10)
NV1 CHO gPCR ref F: 5'-ACAGGTTTCTGCTTCTGGCA-3' (SEQ ID NO: 11)
NV1 CHO gPCR ref R: 5'-CATCAGCTGACTGGTTCACA-3' (SEQ ID NO: 12)

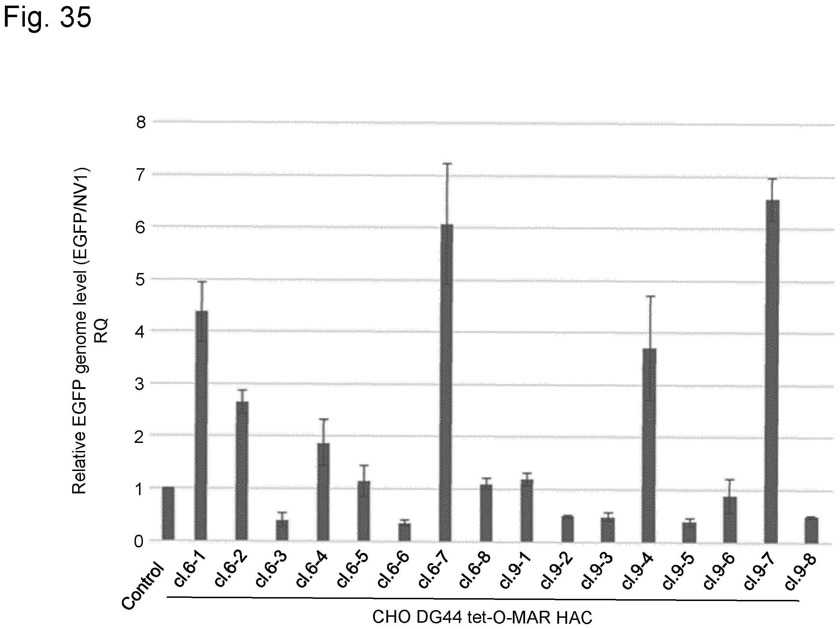

The 16 CHO DG44 tet-O HAC clones obtained were analyzed by real-time PCR. As a result, all the clones were found to comprise the EGFP sequences more than the control CHO DG44 N10 EGFP-HAC. Among them, 2 clones comprising particularly many EGFP sequences (i.e., cl.6-1 and cl.9-7) were subjected to the subsequent analysis (Fig. 35).

### [B.1.2] Two-color FISH analysis

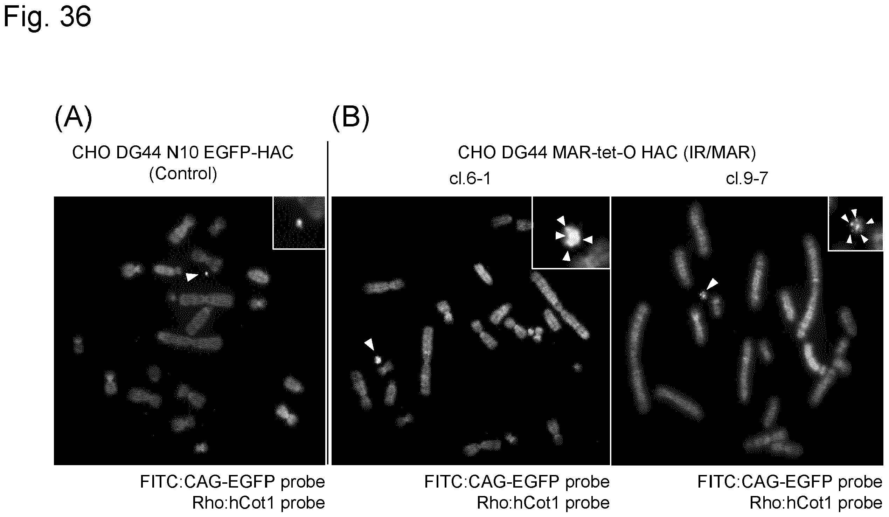

For 2 clones selected from the above-mentioned results, two-color FISH analysis was conducted according to Matsubara et al. (FISH experimental protocols, Shujunsha Co., Ltd., Japan, 1994). The FISH analysis was carried out using mouse cot-1 DNA and pCX-EGFP plasmid as probes. As a result, both the clones were found to have larger chromosomes and many EGFP signals on human cot-1 signals, when compared with the control (CHO DG44 N10 EGFP-HAC) (Fig. 36). From these results, it was determined that the EGFP gene was amplified on tet-O HAC, and the said clones were subjected to the subsequent expression analysis.

### [B.2] Analysis of EGFP expression level by Western blotting

In order to confirm whether or not the EGFP expression level of the CHO DG44 tet-O HAC clones (i.e., cl.6-1 and cl.9-7) was increasd accompanied with gene sequence amplification, the protein expression levels were analyzed by Western blotting.

The Western blotting was carried out using, as a cell lysis solution, RIPA buffer (Nacalai Tesque) containing a proteolytic inhibitor, and protein electrophoresis was carried out on 10% SDS acrylamide gel (Nacalai Tesque) at 2 µg clones/well using a Lapidus Mini Slab electrophoresis tank (ATTO). Proteins were transferred to a PVDF membrane with a pore size of 0.45 µm (GE Healthcare) using the XCell SureLock Mini-Cell electrophoresis system. Subsequently, a blocking reaction was carried out using a Western blocking reagent (Roche) in accordance with the recommended protocol at 4°C overnight, the reaction product was washed 4 times with PBS-T for 10 minutes, and the resultant was subjected to the subsequent antibody reaction. EGFP detection was carried out by conducting a reaction using, as Antibody 1, the 5,000-fold diluted (0.2 ng/µl) anti-EGFP mouse antibody (abcam: ab184601) for 1 hour at room temperature. After the reaction product was washed 3 times with PBS-T for 10 minutes, the subsequent reaction was carried out using, as Antibody 2, the 5,000-fold diluted (0.2 ng/µl) anti-mouse IgG goat antibody (abcam: ab6789) for 45 minutes. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan). Tubulin for the internal standard was detected by conducting a reaction using the 5,000-fold diluted (0.2 ng/µl) anti-α tubulin rabbit HRP-DirecT antibody (MBL: PM054-7) for 1 hour at room temperature. After the reaction product was washed 3 times with PBS-T for 10 minutes, the subsequent reaction was carried out using the Pierce ECL Western blotting substrate (ThermoFisher) luminescence reagent in accordance with the recommended protocol. Protein detection was carried out using ImageQuant LAS4000 (FUJIFILM Corporation, Japan).

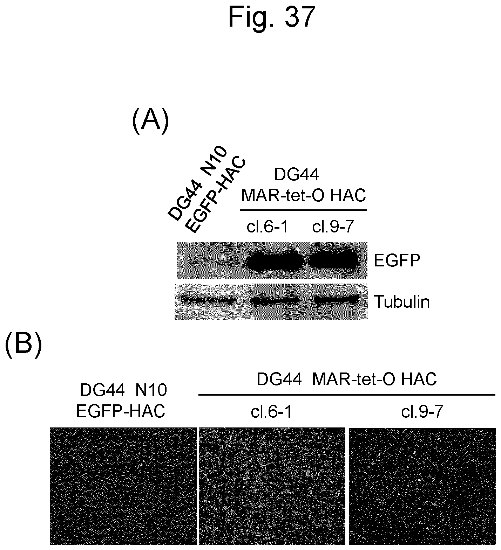

As a result of Western blot analysis, higher protein expression levels were observed in the CHO DG44 tet-O HAC clones, when compared with the control (Fig. 37A). As a result of fluorescent microscopic observation, higher fluorescence brightness was observed in the CHO DG44 tet-O HAC clone compared with the control, as with the results of Western blotting (Fig. 37B). On the basis of the results, it was determined that gene amplification could have been achieved by integrating the IR/MAR sequence and the EGFP gene into tet-O HAC and, as a consequence, the EGFP protein expression levels could have been enhanced. This method of gene amplification was found to be applicable to, not only to the top-down type of an artificial chromosome but also to the bottom-up type of an artificial chromosome (Fig. 33, Step 3).

### INDUSTRIAL APPLICABILITY

According to the present invention, a mammalian cell transformed with the IR/MAR-MMAC nucleic acid construct comprising DNA encoding a gene of interest is cultured, thereby enabling stable and high expression of the gene of interest.

### SEQUENCE LISTING FREE TEXT

SEQ ID NOs: 1 to 4, 9 to 14, 17, 18, and 20 to 23: primers
SEQ ID NOs: 5 to 8 and 19: binding regions
SEQ ID NO: 15: anti-VEGF light chain gene vector
SEQ ID NO: 16: anti-VEGF light chain gene vector

All publications, patents, and patent applications cited herein are incorporated herein by reference in their entirety.

## Claims

1. A nucleic acid construct comprising a DNA sequence of interest, a matrix attachment region, and a replication origin (or a replication initiation region) in a mammalian artificial chromosome vector, and used for high expression of the DNA.

2. The nucleic acid construct according to claim 1, wherein the mammalian artificial chromosome vector is a human artificial chromosome vector or a rodent artificial chromosome vector.

3. The nucleic acid construct according to claim 2, wherein the rodent artificial chromosome vector is a mouse artificial chromosome vector.

4. The nucleic acid construct according to claim 2, wherein the rodent artificial chromosome vector is a hamster-derived artificial chromosome vector.

5. The nucleic acid construct according to claim 4, wherein the hamster-derived artificial chromosome vector is an artificial chromosome vector derived from the Chinese hamster ovary (CHO) cell.

6. The nucleic acid construct according to any one of claims 1 to 5, wherein the matrix attachment region is derived from the matrix attachment region of an Igκ gene locus.

7. The nucleic acid construct according to any one of claims 1 to 6, wherein the replication origin (or, the replication initiation region) is derived from the replication initiation region of a dihydrofolate reductase gene locus.

8. The nucleic acid construct according to any one of claims 1 to 7, wherein the DNA comprises a gene or gene locus encoding a protein, cDNA, recombinant DNA or modified DNA, or DNA comprising a gene control region and a reporter gene.

9. The nucleic acid construct according to any one of claims 1 to 8, wherein the protein encoded by the DNA sequence of interest is derived from a human.

10. A mammalian cell for high expression of a protein encoded by a DNA sequence, which comprises the nucleic acid construct according to any one of claims 1 to 9.

11. The mammalian cell according to claim 10, which is a Chinese hamster ovary (CHO) cell.

12. The mammalian cell according to claim 11, wherein the CHO cell is the CHO K1 cell line.

13. A method for high expression of a protein encoded by a DNA sequence comprising a step of culturing the mammalian cell according to any one of claims 10 to 12 in a medium.

14. A method for production of a protein encoded by a DNA sequence comprising the following steps of: culturing the mammalian cell according to any one of claims 10 to 12 in a medium; and recovering the produced protein.

15. A non-human animal carrying the nucleic acid construct according to any one of claims 1 to 9.

16. The non-human animal according to claim 15, which is a rodent.

17. The non-human animal according to claim 16, wherein the rodent is a mouse.

18. The non-human animal according to claim 16, wherein the rodent is a rat.
